# EUROPEAN PATENT APPLICATION

(11) **EP 4 640 682 A2**
(43) Date of publication of application: **29.10.2025**
(21) Application number: 25172269.0
(22) Date of filing: 24.04.2025
(51) Int. Cl.: C07D 417/04, A61P 35/00, C12N 15/11

(54) **COMPOSITIONS OF AGPAT4 INHIBITORS AND METHODS OF USING THEREOF TO TREAT CANCER**

(30) Priority: 25.04.2024 US 202463638643 P
(71) Applicant: The University of Hong Kong, Pokfulam, Hong Kong (CN); Centre for Oncology and Immunology Limited, New Territories, Hong Kong (HK); Laboratory for Synthetic Chemistry and Chemical Biology Limited, Hong Kong Shatin (HK)
(72) Inventor: MA, Stephanie Kwai Yee, Hong Kong (CN); CHUNG, Yik Sham Clive, Hong Kong (CN); NG, Kai Yu, Hong Kong (CN); KOO, Tin Yan, Hong Kong (CN)
(74) Representative: HGF

(57) **Abstract**

Methods and compositions for treating cancer, e.g., liver cancer in a subject in need thereof are provided. Compositions including an effective amount of an 1-Acylglycerol-3-Phosphate O-Acyltransferase 4 (AGPAT4) inhibitor alone, or in combination with a kinase inhibitor and methods of use thereof for treating cancer are disclosed. The composition includes one or more small molecule inhibitors, inhibitory nucleic acids, or inhibitory proteins in a pharmaceutically acceptable carrier. Administration of the AGPAT4 inhibitor, alone or in combination with a kinase inhibitor is effective to reduce cancer cell proliferation or viability in a subject with cancer. Methods of selecting and treating subjects with cancers, particularly liver cancer, are also provided.

## Description

### FIELD OF THE INVENTION

The disclosed invention is generally directed to AGPAT4 as a diagnostic marker and therapeutic target for cancer detection and treatment.

### BACKGROUND OF THE INVENTION

Hepatocellular Carcinoma (HCC), the most common form of liver cancer, is a deadly disease with limited treatment options and a poor prognosis. Although advances in detection and treatment have increased the likelihood of promising treatments in the early stages of this disease, the overall prognosis of HCC is still unsatisfactory because of late presentation when patients are no longer eligible for curative therapies such as liver resection or transplantation. In such cases, the multi-kinase inhibitor sorafenib has for a very long time been the only drug approved by the US Food and Drug Administration (FDA) as a first-line treatment, expanding patient median survival for approximately 3 months (1). Despite the initial response, sorafenib-treated tumors rarely regress completely, and the therapeutic effects of the drug are often temporary. Unfortunately, most patients develop disease progression, and in the case of HCC, radiological progression under sorafenib occurs after 4-5 months of treatment (2). In recent years, other targeted therapies, including lenvatinib and immune checkpoint inhibitors, such as monoclonal antibodies targeting PD-1 and PD-L1, have been adopted as first-line treatment strategies for HCC patients with unresectable tumors. However, because of tumor heterogeneity and the immune-suppressive microenvironment of the disease, only a small fraction of patients responds well to these therapies (3). Thus, there remains an urgent need to characterize the drivers of resistance to identify markers that can predict treatment outcomes and therapeutic strategies that can improve the efficacy of current FDA-approved drugs.

Tumor lineage plasticity is emerging as an important mechanism for therapeutic resistance (4). As cancer is a dynamic disease, subpopulations of tumor cells or different cell states continue to evolve during malignant progression and therapeutic treatment. This ongoing evolution generates a highly heterogeneous tumor, which includes a diverse collection of cancer cells harbouring distinct molecular signatures and cellular identities (5). Cancer stemness is a property that is now widely accepted to be associated with drug resistance, tumor relapse and the general unfavourable outcome of HCC (6). Indeed, there is now abundant evidence to show that HCC tumor growth is also fuelled by stem-like cells within the tumor called tumor-initiating cells (TICs) that can undergo phenotypic switching to a drug-tolerant, slow-cycling state or differentiate into multiple cellular lineages to avoid drug toxicity-mediated cell death (7). While a critical state of disease, the molecular mechanisms of HCC lineage plasticity are poorly understood, and druggable therapeutic targets are lacking, which impedes effective treatment of this deadly disease.

There remains an urgent need to develop compounds that bind AGPAT4 with high efficacy and high specificity.

Therefore, it is an object of the present invention to provide compounds that bind AGPAT4 with high specificity and high efficacy.

It is also an object of the invention to provide compositions for treating cancers marked by overexpression of AGPAT4.

It is another object of the invention to provide compositions for detecting cancers marked by AGPAT4 overexpression.

It is a further object of the invention to provide methods of using the compounds and compositions for cancer therapy.

### BRIEF SUMMARY OF THE INVENTION

Compositions and methods for treating cancer are provided. The compositions include compounds and pharmaceutical compositions for inhibiting or reducing the expression of 1-Acylglycerol-3-Phosphate O-Acyltransferase 4 (AGPAT4) are provided. The pharmaceutical compositions typically contain one or more compounds or molecules for inhibiting or reducing the expression of AGPAT4 (herein, "AGPAT4 inhibitors") and optionally one or more pharmaceutically acceptable excipients in a pharmaceutically acceptable carrier. The pharmaceutical compositions can be formulated for administration via a variety of routes, such as oral administration, intramuscular administration, intravenous administration, intraperitoneal administration, or subcutaneous administration, or a combination thereof. Optionally, the pharmaceutical composition further contains one or more additional active agent(s), such as one or more additional anticancer agent(s).

The AGPAT4 inhibitors are selected from small molecule inhibitors, inhibitory nucleic acids, inhibitory peptides, inhibitory proteins, and derivatives and/or variants thereof.

Typically, the small molecule inhibitor covalently binds to the cysteine residue Cys228 on AGPAT4. Upon binding to Cys228 on AGPAT4, the small molecule inhibitor engages in an π-π interaction with the residue W106 and inhibits or reduces AGPAT4 activity. In some forms, the small molecule inhibitor has the structure of Formula I presented below, a pharmaceutically acceptable salt, a prodrug, analog, or derivative thereof; or a pharmaceutically acceptable salt of a prodrug, analog, or derivative of the inhibitor represented by Formula I. 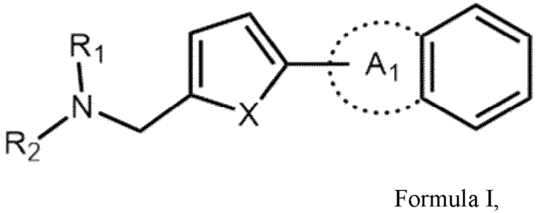 wherein:
(i) A₁ is a C₅-C₆ aromatic ring or a C₄-C₅ heteroaromatic ring;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4;
(iv) X is O, S, NR₃, PR₃, CR₃R₄ or SiR₃R₄ where R₃ and R₄ are independently a hydrogen, a deuterium, a substituted or unsubstituted alkyl, or a substituted or unsubstituted aryl.

In some forms, the inhibitory nucleic acid is an antisense oligonucleotide (ASO), a siRNA, a miRNA, a shRNA, or an external guide sequence. Exemplary shRNA that can be used in the composition contains the sequences:
Mouse Agpat4 clone ID NM_026644.2 (clone 41): 
5’ CGCACCAAAGGCTTT
GCTATTACTTCAAGAGAGTAATAGCAAAGCCTTTGGTGTTTTTTG -3’
(SEQ ID NO:2)
Human AGPAT4 clone ID NM_020133.3 (clone 82): 
5’-
CCGGATGATTGGTGTGACGGAAATTCTCGAGAATTTCCGTCACACCAAT
CATTTTTTG-3’ (SEQ ID NO:17)
Human AGPAT4 clone ID NM_020133.3 (clone 93): CCGGGTGATCATAGAAAGGGTATTTCTCGAGAAATACCCTTTCTATGA TCACTTTTTG-3' (SEQ ID NO: 18) or variants thereof.

Methods of using the AGPAT4 inhibitors or pharmaceutical compositions containing AGPAT4 inhibitors for treating cancer are also disclosed. Generally, the method for treating cancer includes a step of administering the AGPAT4 inhibitors or pharmaceutical composition thereof to a subject in need thereof. Typically, following the administration step, an effective amount of the AGPAT4 inhibitors is administered to the subject, such that cancer cells are killed and/or growth or proliferation of the cancer cells are reduced or prevented, and thereby ameliorate one or more symptoms associated with the cancer in the subject, such as reduce the tumor volume and/or tumor weight. For example, following the administration step or all of the administration steps (if the administration occurs more than one time), an effective amount of the AGPAT4 inhibitors is administered to the subject, such that the tumor volume and/or tumor weight in the subject is at least 10%, at least 20%, at least 30%, at least 40%, or at least 50% smaller/less than a control that is administered with the pharmaceutically acceptable excipient(s) only. Optionally, the anticancer effect is achieved without observable toxicity to the subject, as indicated by body vital measurements (e.g., body weight), standard hematology markers, and/or blood biochemical parameters compared to the control. In some forms, the effective amount of AGPAT4 inhibitors administered to the subject is from about 0.1 µg to about 1000 µg, from about 0.1 µg to about 500 µg, from about 0.1 µg to about 200 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject.

In some forms, the cancer is selected from liver cancer, breast cancer, colorectal cancer, HNSC (head and neck squamous cell carcinoma), LIHC (liver hepatocellular carcinoma) and PAAD (pancreatic adenocarcinoma), glioma, testicular cancer, or endometrial cancer . Preferably, the cancer to be treated is the cancer characterized by increased expression and/or activity of AGPAT4.

Combination therapies for treating cancer are also provided. Generally, the method for the combination therapy includes a step of administering an effective amount of a pharmaceutical composition containing an AGPAT4 inhibitor in combination with an effective amount of a kinase inhibitor. The AGPAT4 inhibitor and the kinase inhibitor can be formulated separately or together in the same admixture. The administration of the combination of the composition and the kinase inhibitor reduces cancer cell proliferation, reduces cancer cell viability, or reduces both cancer cell viability and proliferation in a subject with cancer to a greater degree than administering to the subject the same amount of the AGPAT4 inhibitor alone or the same amount of the kinase inhibitor alone. In some forms, the kinase inhibitor is a receptor tyrosine kinase inhibitor.

Additional advantages of the disclosed method and compositions will be set forth in part in the description which follows, and in part will be understood from the description, or can be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other forms the values may range in value either above or below the stated value in a range of approx. +/- 5%; in other forms the values may range in value either above or below the stated value in a range of approx. +/- 2%; in other forms the values may range in value either above or below the stated value in a range of approx. +/- 1%. The preceding ranges are intended to be made clear by context, and no further limitation is implied.

### BRIEF DESCRIPTION OF THE DRAWINGS

The accompanying drawings illustrate several embodiments of the disclosed method and compositions and together with the description, serve to explain the principles of the disclosed method and compositions.

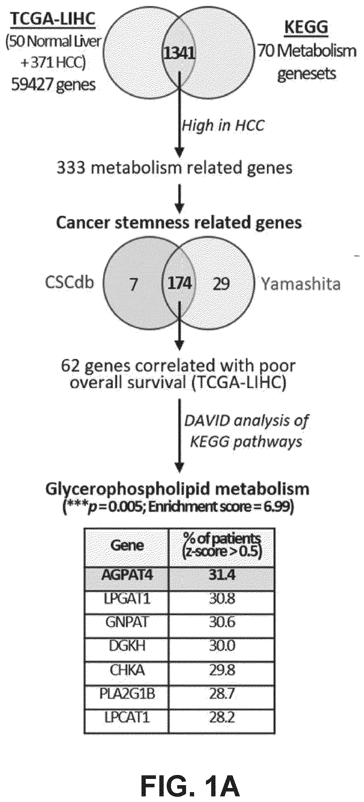

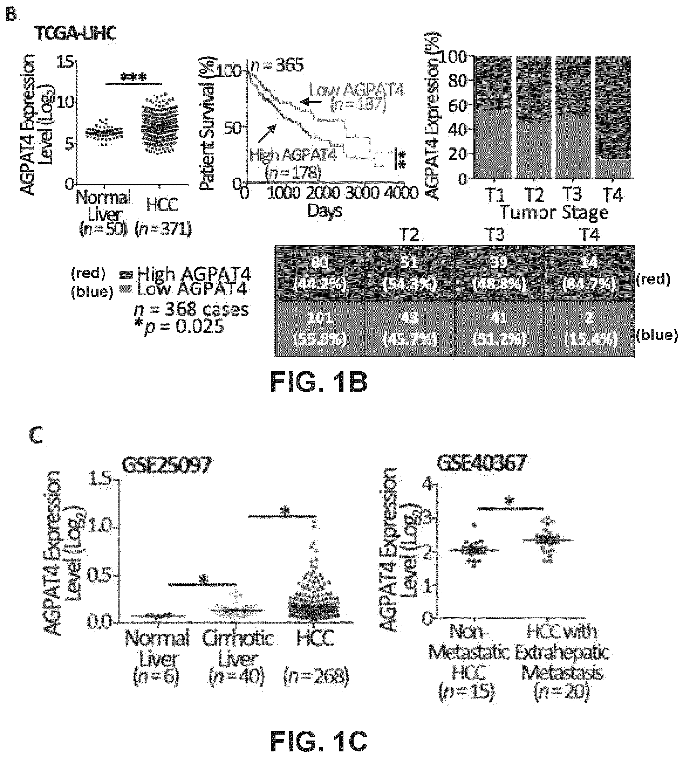

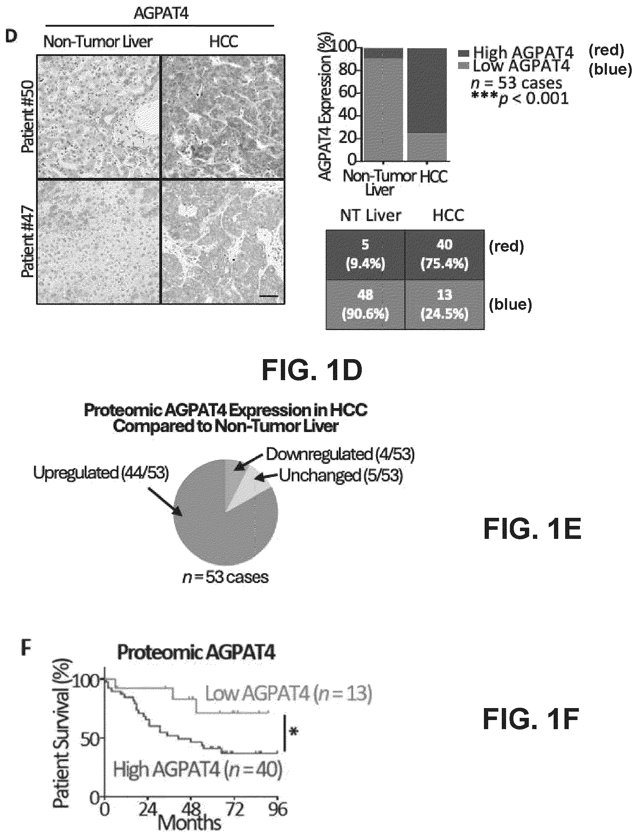

**FIG. 1A** is a schematic representation of the approach for identifying potential metabolic drivers of stemness in HCC. **FIG. 1B** are results from analysis of AGPAT4 expression and its correlation with survival and tumor stage in The Cancer Genome Atlas (TCGA) Liver Hepatocellular Carcinoma (LIHC) dataset. **FIG. 1C** are results from analysis of AGPAT4 expression in GSE25097 (comparing normal liver, cirrhotic liver, and HCC) and GSE40367 (comparing non-metastatic HCC and HCC with extrahepatic metastasis) datasets. **FIGs. 1D** **and1E** show AGPAT4 protein expression in paired non-tumor (NT) liver and HCC clinical biopsies as examined by immunohistochemistry, with expression intensities scored. Representative AGPAT4 staining images of paired non-tumor and tumor samples from two HCC patients. Scale bar =100µm. **FIG. 1F** are results from Kaplan-Meier analysis of the overall survival in patients with high and low AGPAT4 scoring in their tumors. **p*<0.05, ****p*<0.001 (unless otherwise specified).

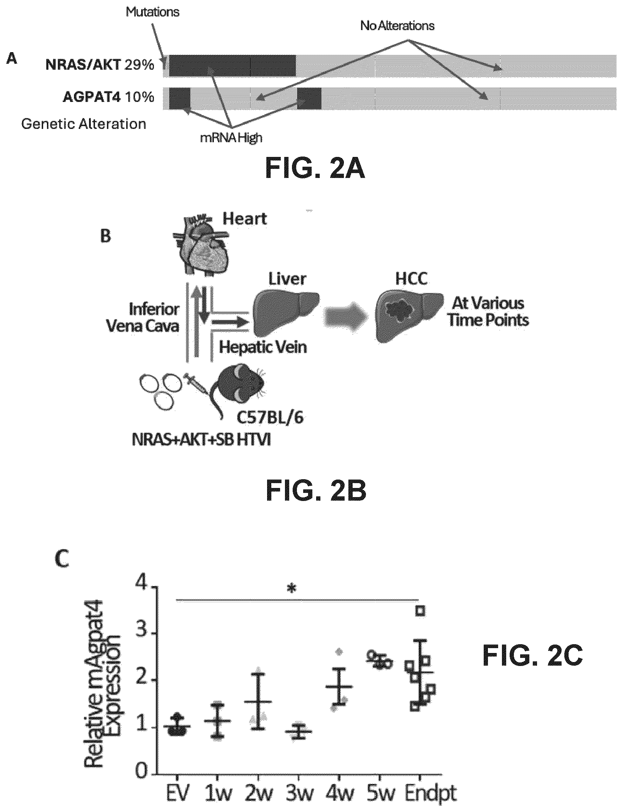

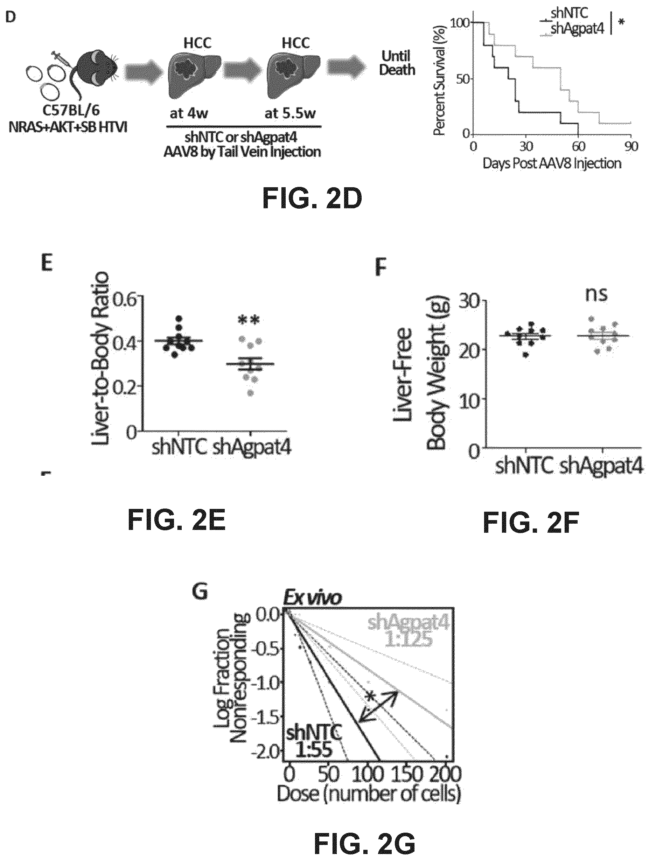

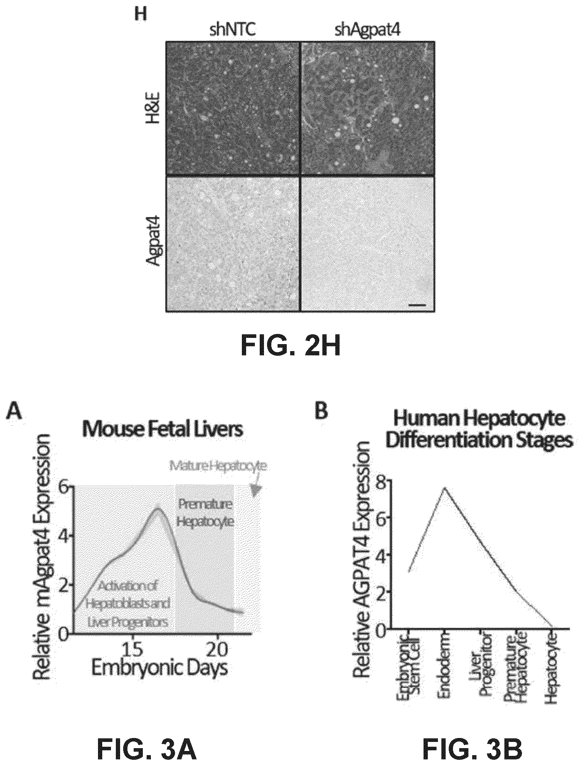

**FIG. 2A** shows oncoprint of NRAS+AKT alterations and/or overexpression or AGPAT4 overexpression in 372 HCC patients (TCGA-LIHC via cBioPortal). **FIG. 2B** is a schematic representation of the hydrodynamic tail vein injection (HTVI) HCC model in C57BL/6 mice. **FIG. 2C** are results from qPCR analysis of Agpat4 expression in mice that received HTVI of either the empty vector (EV) control or NRAS+AKT and sleeping beauty (SB) transposase for HCC induction with samples collected at various time points. *n* = 22 in total (EV, *n =* 3; 1 week, *n =* 3; 2 weeks, *n* =3; 3 weeks, *n* = 3; 4 weeks, *n =* 3; 5 weeks, *n =* 3; endpoint, *n =* 7). **FIG. 2D****, left** illustrates a strategy for testing the functional significance of Agpat4 in hepatocarcinogenesis. The NRAS, AKT, and SB transposases were delivered by HTVI to induce HCC. AAV8 virus particles with shRNA scrambled non-targeting control (shNTC) or shAgpat4 were administered twice, at 4 and 5.5 weeks. Mice were sacrificed at the humane endpoint. **FIG. 2D****, right** is Kaplan-Meier survival estimate curves for the overall survival of each treatment group. **FIG. 2E** shows liver-to-body weight ratio in each treatment group. **FIG. 2F** shows liver-free body weight in each treatment group. **FIG. 2G** are results from an *ex vivo* limiting dilution assay of HCC tumor cells harvested from each treatment group to evaluate the frequency of tumor-initiating cells. **FIG. 2H** are H&E and immunohistochemical images of Agpat4 staining in HCC tumors harvested from each treatment group. Scale bar = 100µm. **p*<0.05, ***p*<0.01. ns, not significant.

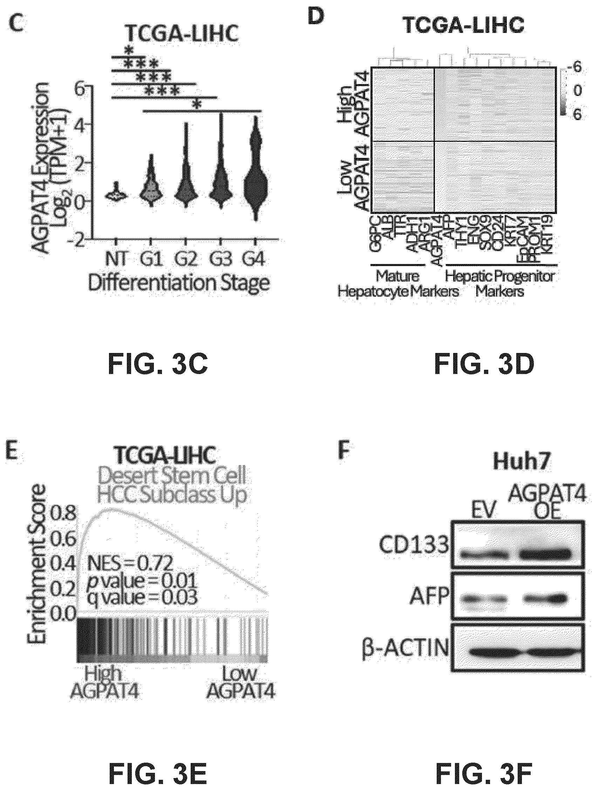

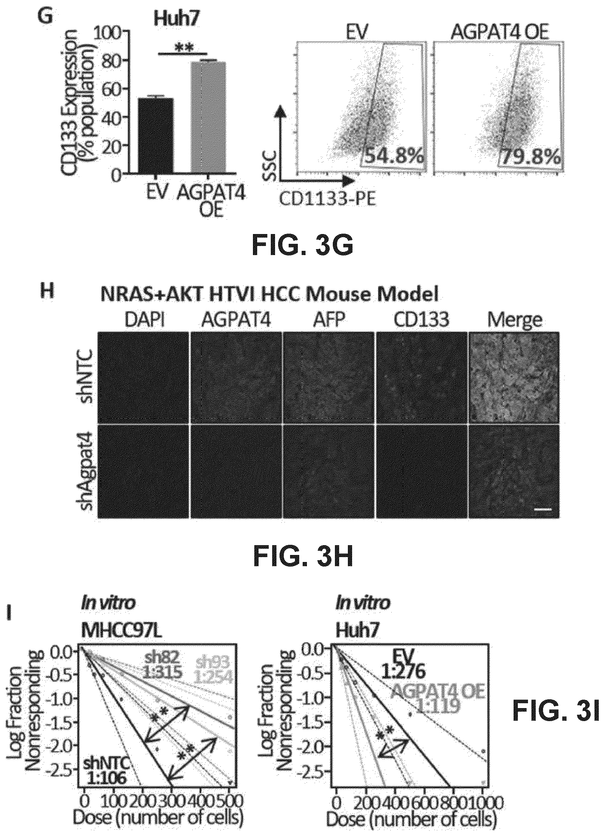

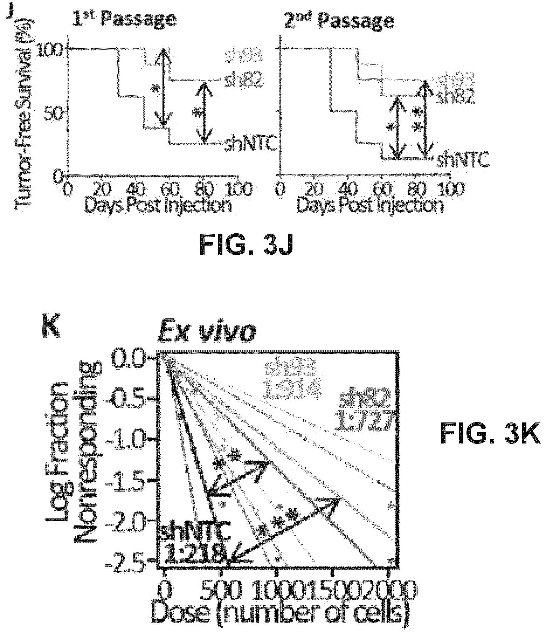

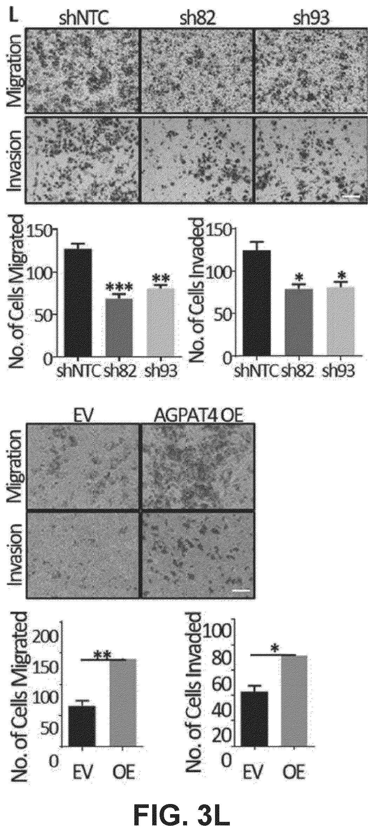

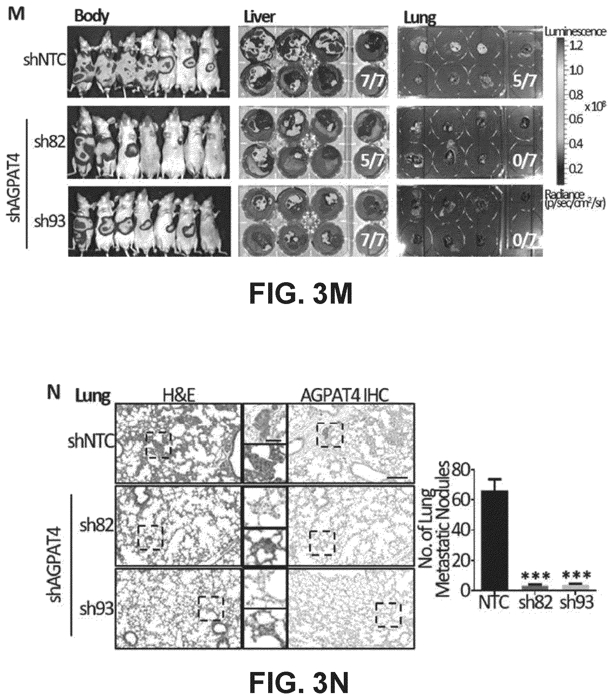

**FIG. 3A and 3B** are results from qPCR analysis of Agpat4/AGPAT4 expression in **(****FIG. 3A****)** fetal mouse livers and **(****FIG. 3B****)** human hepatocyte differentiation models at different developmental stages. **FIG. 3C** is a violin plot showing AGPAT4 expression in the TCGA-LIHC cohort stratified by histological grading (Edmondson-Steiner 4-tier). NT, non-tumor; G1, well-differentiated; G2, moderately differentiated; G3, poorly differentiated; G4, undifferentiated. **FIG. 3D** is a heatmap showing results when patients in TCGA-LIHC cohort were ranked based on AGPAT4 expression. High AGPAT4 = top 50% AGPAT4; low AGPAT4 = bottom 50% AGPAT4. Heatmap showing clustering of AGPAT4 expression with hepatic progenitor and mature hepatocyte markers. **FIG. 3E** are results from a Gene Set Enrichment Analysis (GSEA) showing that AGPAT4 high tumors demonstrate enrichment of stem cell-related gene set via. NES=normalized enrichment score. **FIG. 3F** shows the effect of manipulating AGPAT4 expression on CD133 and AFP levels by western blotting. **FIG. 3G** shows effect of manipulating AGPAT4 expression on cell surface expression of CD133 by flow cytometry. **FIG. 3H** are images from representative multiplex IHC staining for AGPAT4, AFP, and CD133 in the NRAS+AKT HTVI HCC mouse model with or without Agpat4 endogenously repressed. Scale bar = 25µm. **FIG. 3I** are results from *in vitro* limiting dilution assays for evaluating self-renewal abilities of MHCC97L and Huh7 cells with or without AGPAT4 expression manipulated. **FIG. 3J** are Kaplan-Meier survival estimate curves for tumor-free survival of mice xenografted with MHCC97L cells, with or without AGPAT4 suppression. **FIG. 3K** are results from *ex vivo* limiting dilution assay of tumors harvested from FIG. 3J. **FIG. 3L** are representative images and quantification of the number of MHCC97L and Huh7 cells that migrated and invaded with or without AGPAT4 expression manipulated. Scale bar = 20µm. **FIG. 3M** provides bioluminescence imaging of harvested livers and lungs from nude mice injected intrahepatically with luciferase labelled MHCC97L cells, with or without AGPAT4 suppression. *n =* 7 per group. **FIG. 3N** shows representative H&E staining of lung tissues harvested from the orthotopic MHCC97L metastasis model and quantification of the number of lung metastatic nodules. Scale bar = 100µm. Scale bar of magnified image = 50µm. **p*<0.05, ***p*<0.01, ****p*<0.001. OE, overexpression; NTC, shRNA scrambled non-targeting control; sh82 and sh93, two independent shRNA clones targeting AGPAT4.

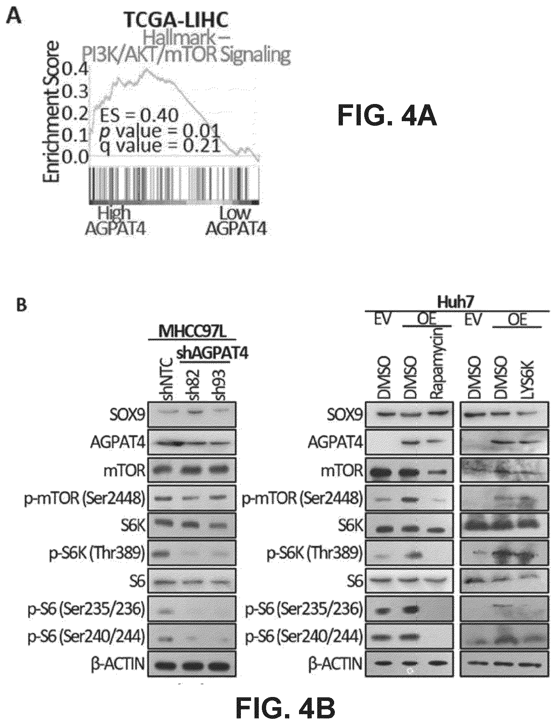

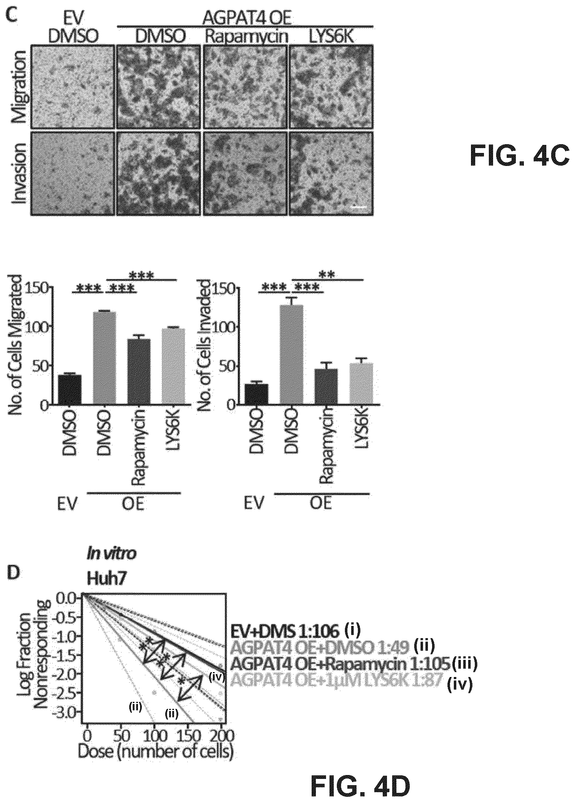

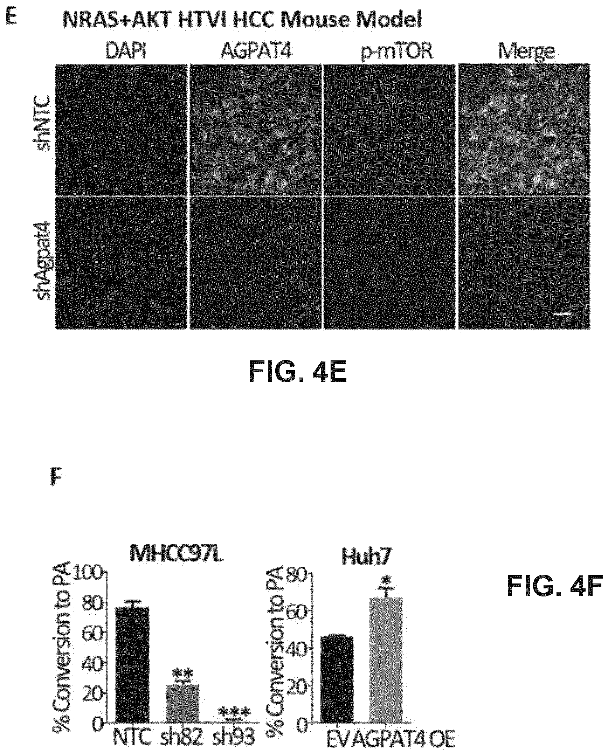

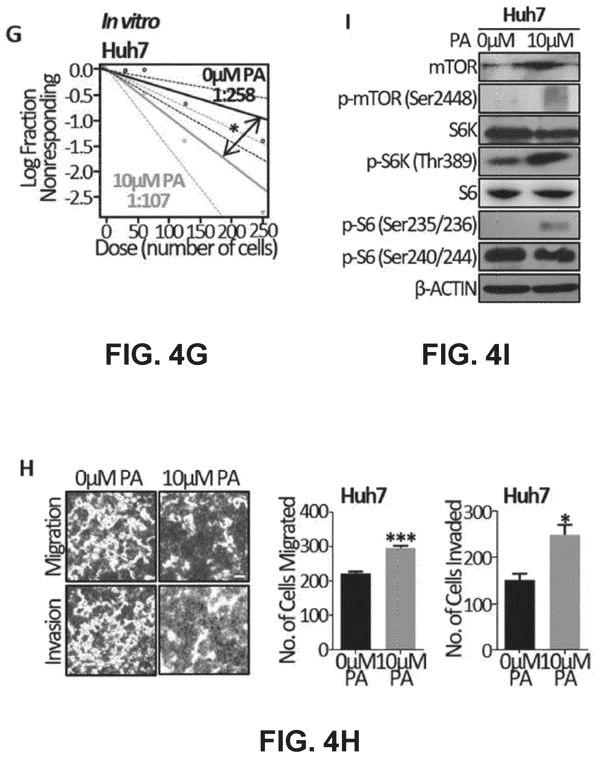

**FIG. 4A** are results from a gene set enrichment analysis (GSEA) of HCC tumors (TCGA-LIHC) segregated by high or low AGPAT4 expression levels. **FIG. 4B** are results from western blot analysis for expression of AGPAT4, total and phosphorylated forms of mTOR, S6K, and S6 in MHCC97L cells with or without AGPAT4 suppression or Huh7 cells with or without AGPAT4 overexpression, and in the absence or presence of rapamycin (mTOR inhibitor) or LYS6K (S6K inhibitor). **FIG. 4C** are representative images and quantification of the number of MHCC97L and Huh7 cells that migrated and invaded with or without AGPAT4 expression manipulated and in the absence or presence of rapamycin or LYS6K. Scale bar = 20µm. **FIG. 4D** are results from *in vitro* limiting dilution assays for evaluating self-renewal abilities of MHCC97L and Huh7 cells with or without AGPAT4 expression manipulated and in the absence or presence of rapamycin or LYS6K. **FIG. 4E** provides representative multiplex IHC staining for AGPAT4 and p-mTOR in the NRAS+AKT HTVI HCC mouse model, with or without Agpat4 endogenously repressed. Scale bar = 25µm. **FIG. 4F** shows the effect of manipulating AGPAT4 expression on the abundance of PA produced in the PA conversion assay. **FIG. 4G** are results from *in vitro* limiting dilution assays for evaluating the self-renewal abilities of Huh cells with or without the addition of exogenous PA. **FIG. 4H** are representative images and quantification of Huh7 cells that migrated or invaded in the presence or absence of exogenous PA. Scale bar = 20µm. **FIG. 4I** shows the effect of exogenous PA addition on the abundance of total and phosphorylated forms of mTOR, S6K, and S6 in Huh7 cells by western blotting. **p<0.05, **p<0.01, ***p<0.001.* OE, overexpression; NTC, shRNA scrambled non-targeting control; sh82 and sh93, two independent shRNA clones targeting AGPAT4.

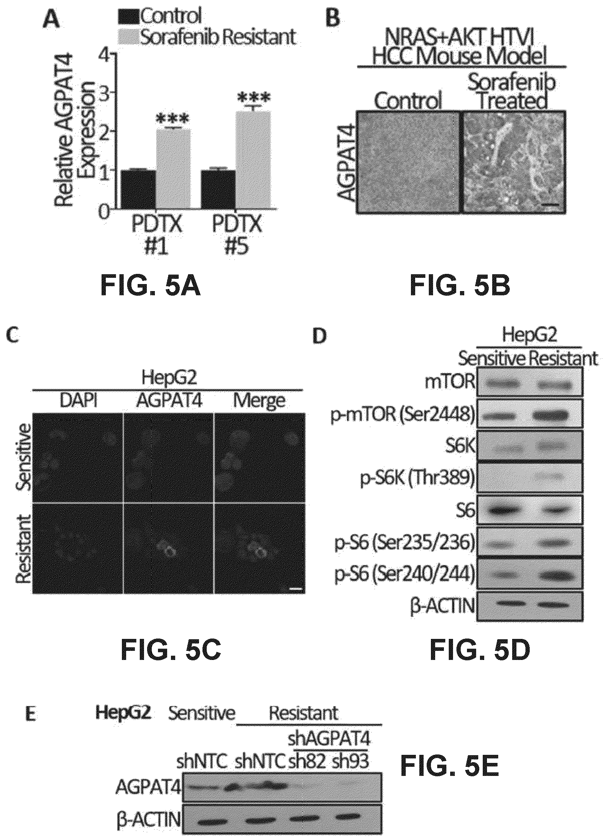

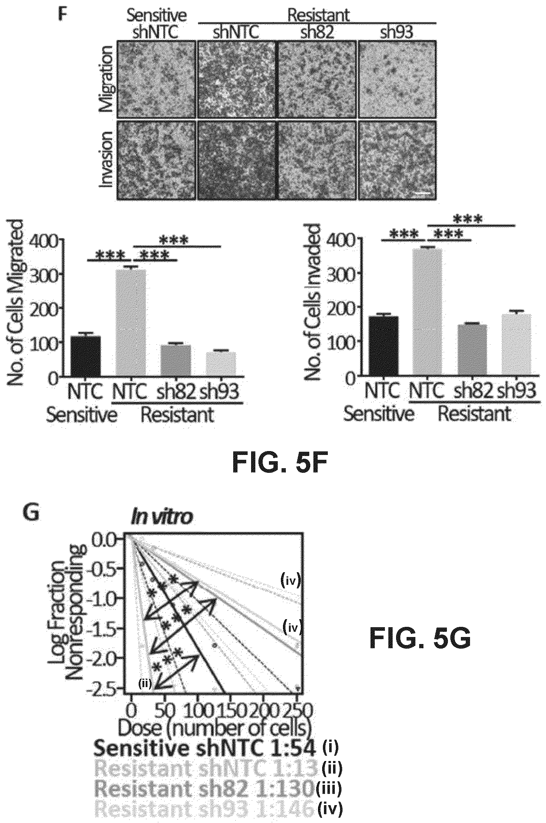

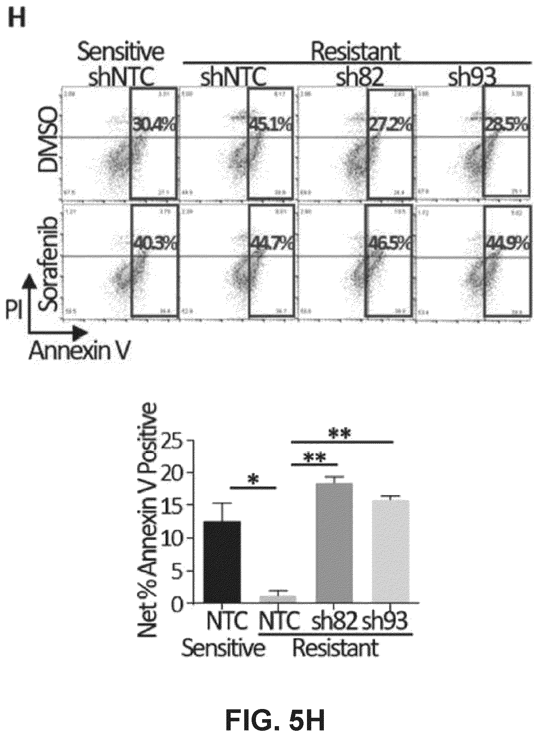

**FIG. 5A** is a bar graph showing genomic AGPAT4 expression in sorafenib-sensitive (control) and sorafenib-resistant HCC patient-derived xenografts (PDTX #1 and #5). **FIG. 5B** are representative images from immunohistochemical analysis for staining of AGPAT4 in control and sorafenib-treated NRAS+AKT HTVI HCC mouse tissues. Scale bar = 100µm. **FIG. 5C** are representative images from immunofluorescence analysis of proteomic AGPAT4 expression in sorafenib-sensitive and sorafenib-resistant HepG2 cells. Scale bar = 20µm. **FIG. 5D** are images from western blot analysis of the expression of total and phosphorylated forms of mTOR, S6K, and S6 in sorafenib-sensitive and sorafenib-resistant HepG2 cells. **FIG. 5E** shows the effect of manipulating AGPAT4 expression on the abundance of AGPAT4 in sorafenib-sensitive and sorafenib-resistant HepG2 cells by western blotting. **FIG. 5F** provides representative images and quantitation of the number of cells that migrated and invaded with or without AGPAT4 expression manipulated in sorafenib-sensitive and sorafenib-resistant HepG2 cells. Scale bar = 20µm. **FIG. 5G** are results from *in vitro* limiting dilution assays for evaluating self-renewal abilities of sorafenib-sensitive and sorafenib-resistant HepG2 cells with or without AGPAT4 expression manipulated. **FIG. 5H** provides representative flow cytometry analysis of Annexin V-PI staining and quantitation of net apoptosis in sorafenib-sensitive and sorafenib-resistant HepG2 cells with or without AGPAT4 expression manipulated. **p<0.05, **p<0.01, ***p<0.001.* NTC, shRNA scrambled non-targeting control; sh82 and sh93, two independent shRNA clones targeting AGPAT4.

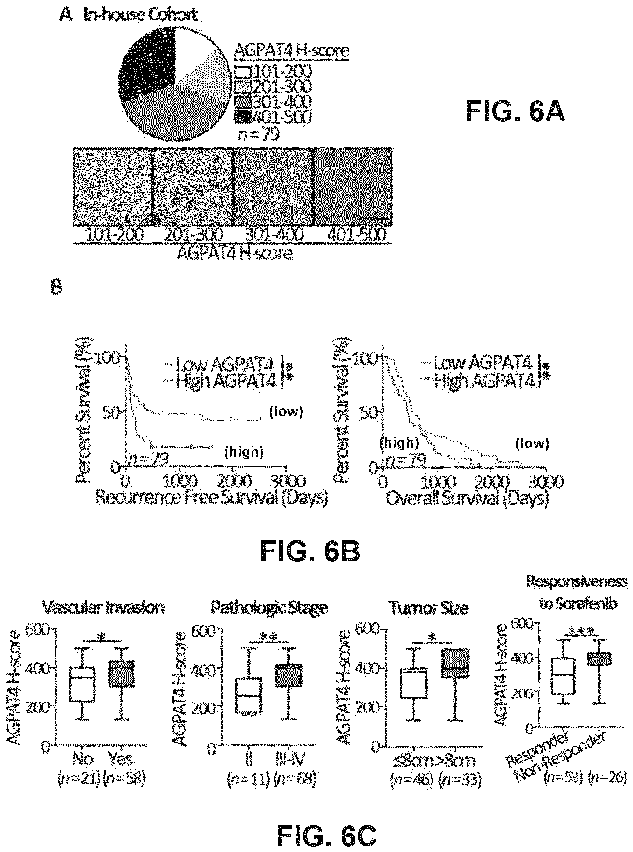

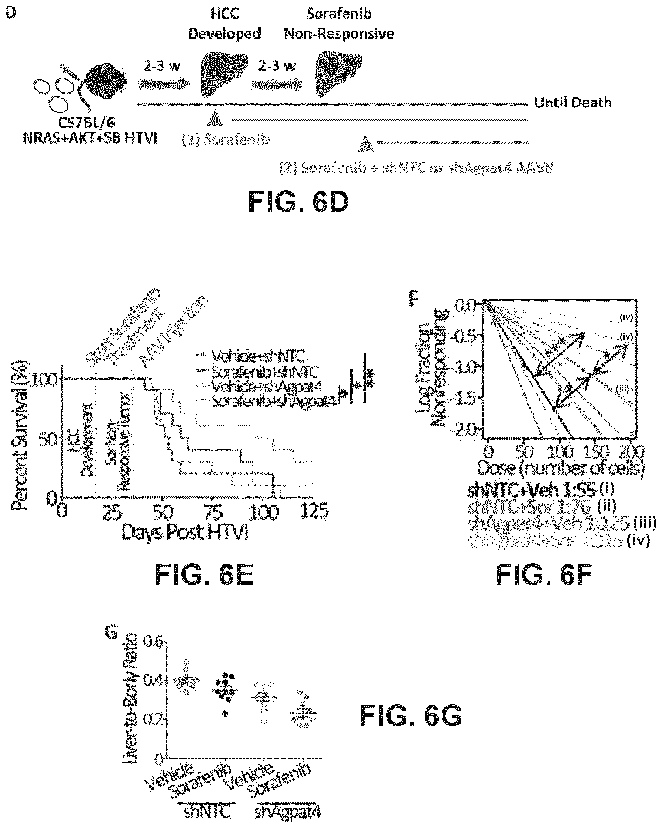

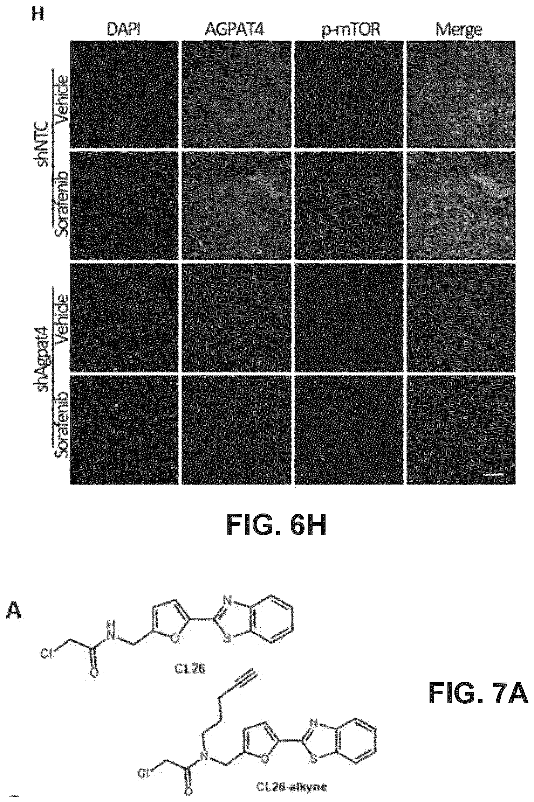

**FIG. 6A** are representative images of AGPAT4 immunohistochemical staining at different intensities in resected HCC samples from patients who underwent sorafenib treatment. Quantification of AGPAT4 staining was performed using the immunohistochemistry H-score, which combines the intensity score and percentage of positive cells. Scale bar = 100µm. **FIG. 6B** provides results from Kaplan-Meier survival analysis comparing the cumulative survival rate of HCC patients who underwent sorafenib treatment with different AGPAT4 expression levels (*n* = 79 cases). **FIG. 6C** are results from correlation analysis with various clinical parameters of HCC patients who had undergone sorafenib treatment with different AGPAT4 expression levels. **FIG. 6D** is an experimental schema for testing the effects of AAV8-mediated shAgpat4 and sorafenib treatment in the NRAS+AKT HTVI HCC mouse model. *n* = 10 mice per group. **FIG. 6E** is a Kaplan-Meier survival curve showing the survival percentage of each annotated group. **FIG. 6F** provides results from *ex vivo* limiting dilution analysis of the frequency of tumor-initiating cells in cells harvested from NTC or AAV8-shAgpat4 mice treated with DMSO or sorafenib. **FIG. 6G** provides quantitative data of liver-to-body weight ratio from NTC or AAV8-shAgpat4 mice treated with DMSO or sorafenib. **FIG. 6H** are representative images from multiplex IHC staining for AGPAT4 and p-mTOR in liver tissues from NTC and AAV8-shAgpat4 mice treated with DMSO or sorafenib. Scale bar = 25µm. **p*<0.05, ***p*<0.01, ****p*<0.001. NTC, shRNA scrambled non-targeting control.

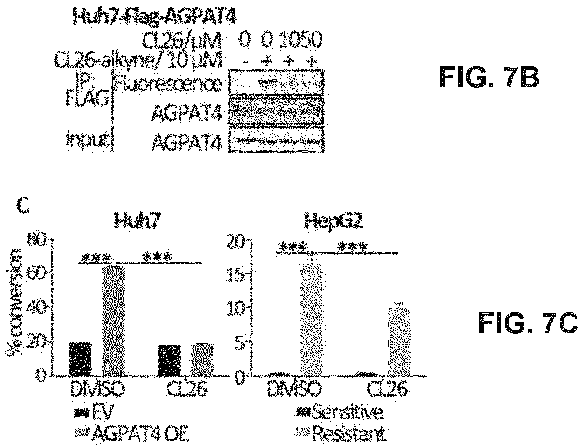

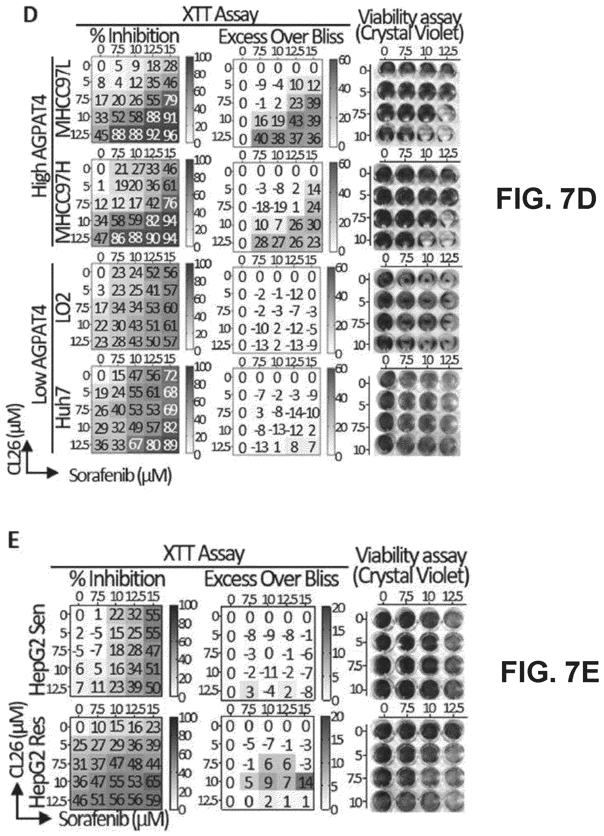

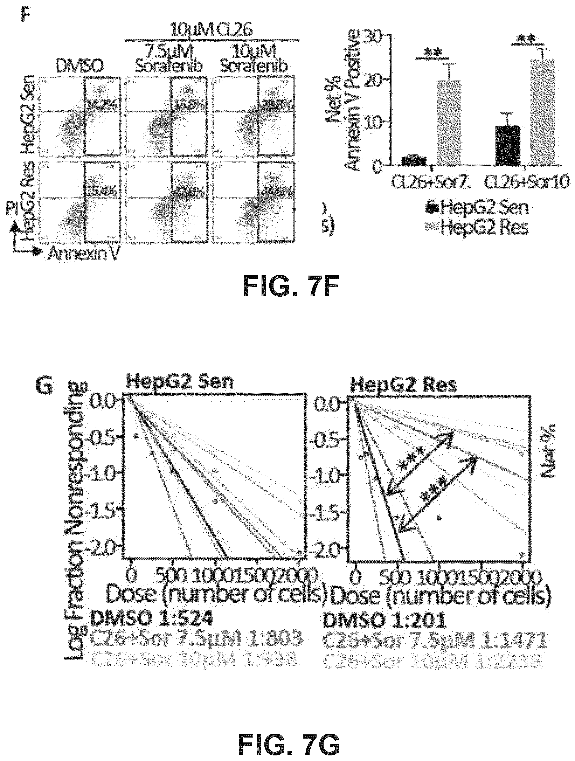

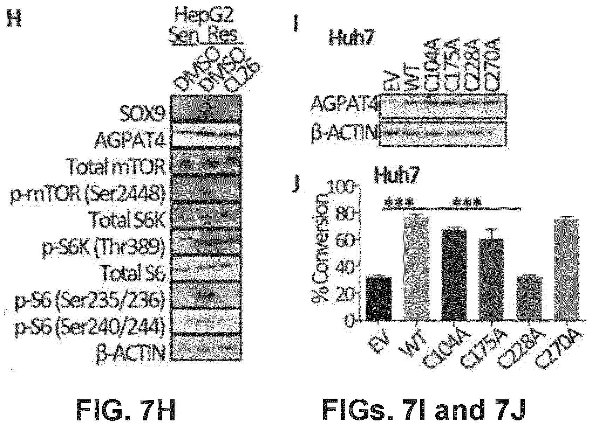

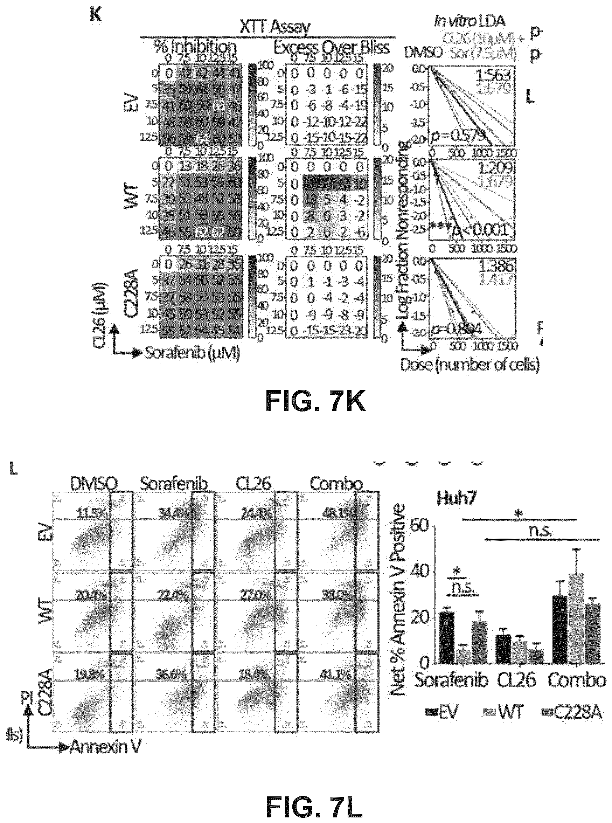

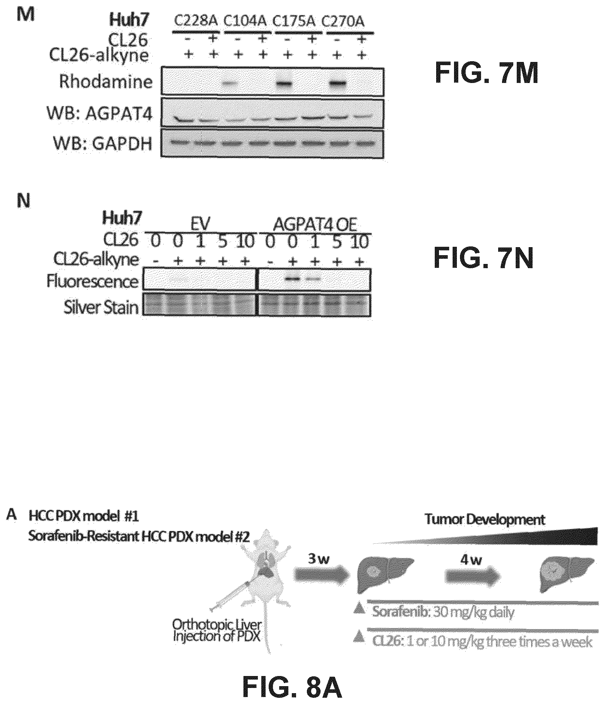

**FIG. 7A** shows the chemical structures of the AGPAT4-specific inhibitor CL26 and its molecular probe CL26-alkyne. **FIG. 7B** shows results from target engagement of CL26 with AGPAT4 in Huh7 cells, as demonstrated by comparing in-gel fluorescence intensity from CL26-alkyne-bound AGPAT4 upon CL26 pre-treatment. **FIG. 7C** are bar graphs showing LPA to PA conversion in Huh7 cells with or without AGPAT4 overexpression or sorafenib-sensitive and sorafenib-resistant HepG2 cells and in the presence of DMSO control of CL26. **FIGs. 7D-7E** shows the effects of sorafenib and CL26 in high AGPAT4 expressing MHCC97L and MHCC97H vs. low AGPAT4 expressing Huh7 and LO2 **(****FIG. 7D****)** as well as sorafenib-sensitive and sorafenib-resistant HepG2 cells **(****FIG. 7E****).** **FIG. 7F** shows cell apoptosis as demonstrated by Annexin V-PI flow cytometry analysis in sorafenib-sensitive and sorafenib-resistant HepG2 cells treated with DMSO control or various combinations of CL26 and sorafenib. **FIG. 7G** provides results from *in vitro* limiting dilution assays for evaluating self-renewal abilities in sorafenib-sensitive and sorafenib-resistant HepG2 cells treated with the DMSO control or various combinations of CL26 and sorafenib. **FIG. 7H** are images from western blot analysis for the expression of SOX9, AGPAT4, total and phosphorylated forms of mTOR, S6K, and S6 in sorafenib-sensitive and sorafenib-resistant HepG2 cells treated with DMSO control or a combination of CL26 and sorafenib. FIG. 7I provides images from western blot analysis for the expression of AGPAT4 in Huh7 cells transfected with empty vector (EV) control, wild-type (WT) AGPAT4, or AGPAT4 overexpressing various mutated cysteines. **FIG. 7J** is a bar graph showing LPA to PA conversion in Huh7 cells transfected with empty vector (EV) control, wild-type (WT) AGPAT4, or AGPAT4 overexpressing various mutated cysteines. **FIG. 7K****, left** shows the synergistic effects of sorafenib and CL26 in Huh7 cells overexpressing empty vector (EV) control, wild-type (WT) AGPAT4, or AGPAT4 with mutated cysteine at position 228 (C228A). **FIG. 7K****, right** provides results from *in vitro* limiting dilution assays for evaluating self-renewal abilities in Huh7 cells overexpressing empty vector (EV) control, wild-type (WT) AGPAT4, or AGPAT4 with mutated cysteine at position 228 (C228A), and when treated with CL26 and sorafenib. **FIG. 7L** shows cell apoptosis as demonstrated by Annexin V-PI flow cytometry analysis in Huh7 cells overexpressing empty vector (EV) control, wild-type (WT) AGPAT4, or AGPAT4 with mutated cysteine at position 228 (C228A), and when treated with CL26 and sorafenib. **FIG. 7M** is an image from a gel-based activity-based protein profiling (ABPP) experiment to investigate engagement of CL26 with different AGPAT4 proteins expressed in Huh7 cells. **FIG. 7N** is an image from a gel-based ABPP to investigate off-target binding of CL26 in Huh7 cells with low endogenous expression of AGPAT4. **p*<0.05, ***p*<0.01, ****p*<0.001. EV, empty vector; OE, overexpression.

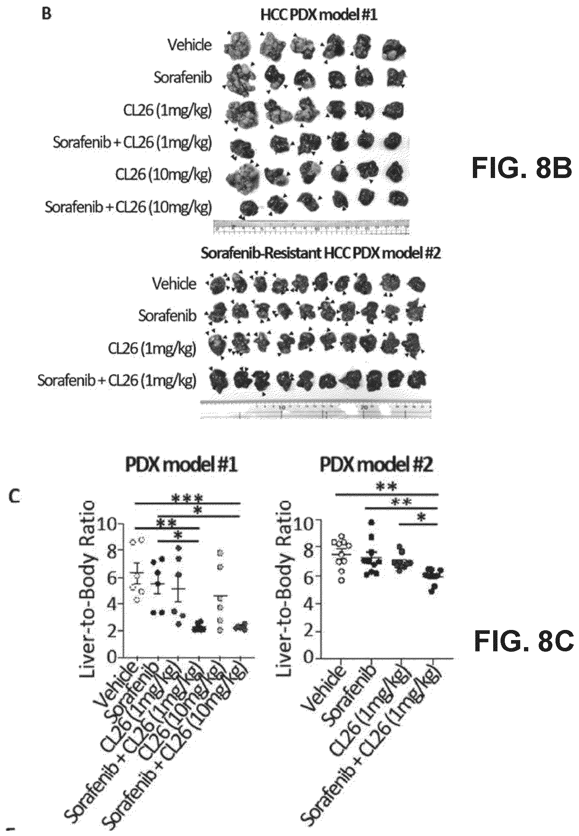

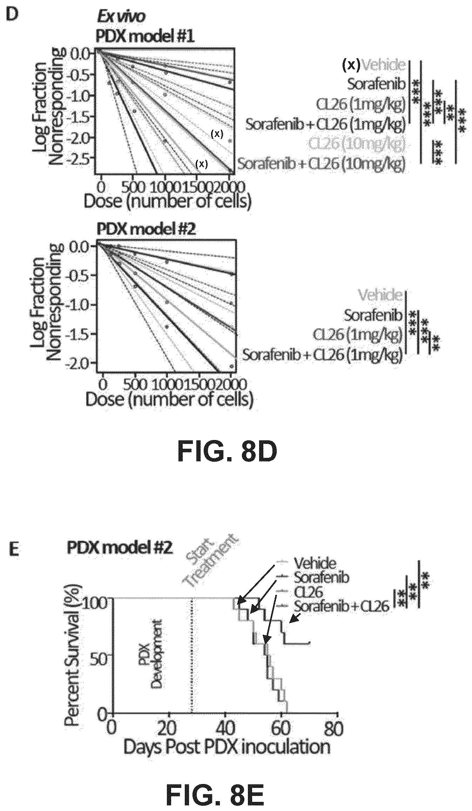

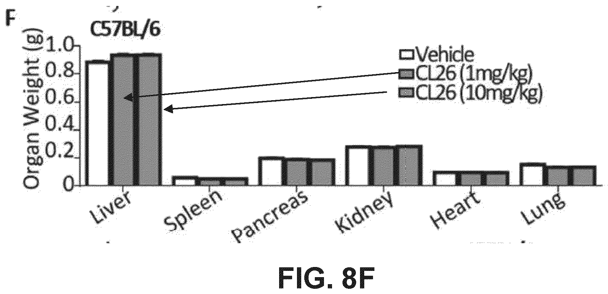

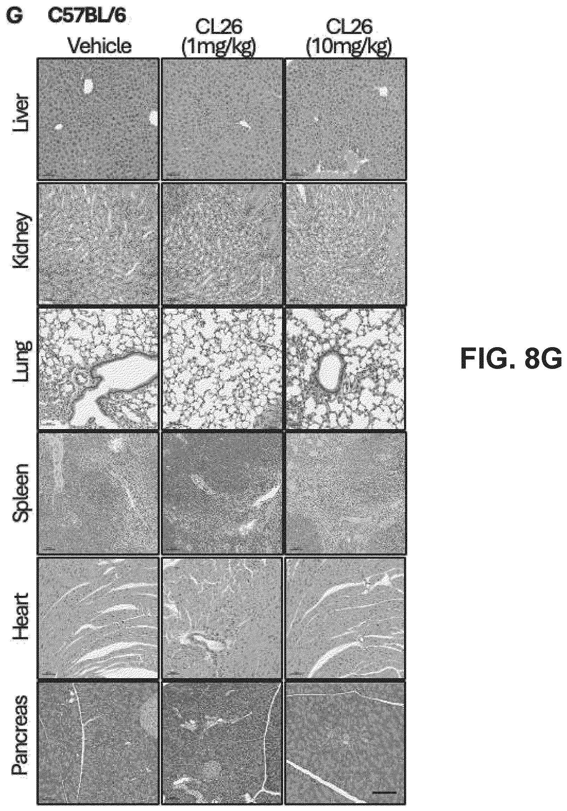

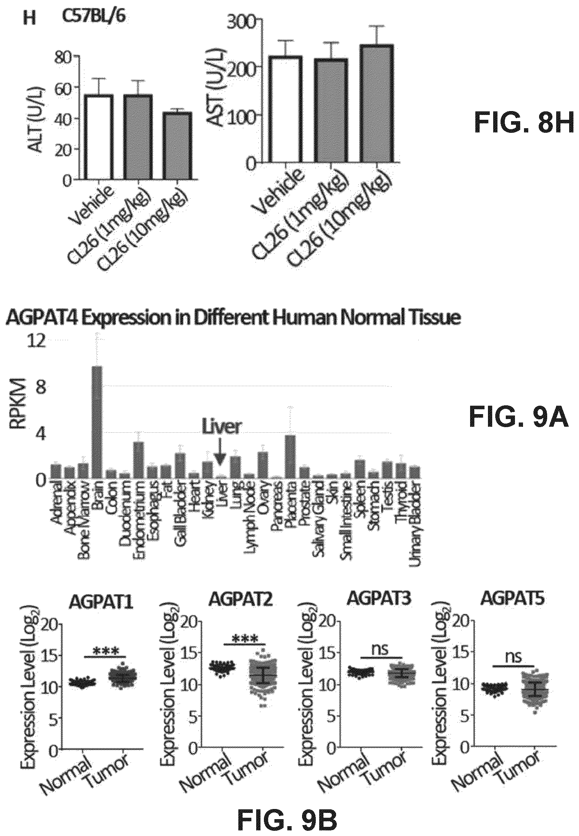

**FIG. 8A** is an experimental scheme for sorafenib and/or CL26 single or combination treatment in HCC patient-derived xenograft (PDX) model #1 and sorafenib-resistant HCC PDX model #2. **FIGs. 8B-8C** are representative images **(****FIG. 8B****)** and quantitative data **(****FIG. 8C****)** of the liver-to-body weight ratio. Arrows point to tumors. **FIG. 8D** provides results from *ex vivo* limiting dilution analysis for the frequency of tumor-initiating cells in cells harvested from each treatment group. **FIG. 8E** is a Kaplan-Meier survival curve showing the survival percentage of each annotated group in HCC PDX model #2. **FIG. 8F** is a bar graph showing weight of various major organs in C57BL/6 mice treated with vehicle control, and low/high doses of CL26. **FIG. 8G** are images from H&E staining of the major organs harvested from C57BL/6 mice treated with vehicle control, and low/high doses of CL26. Scale bar = 100µm. **FIG. 8H** are bar graphs showing ALT and AST levels in C57BL/6 mice treated with vehicle control, and low/high doses of CL26. **p*<0.05, ***p*<0.01, ****p*<0.001. NTC, shRNA scrambled non-targeting control.

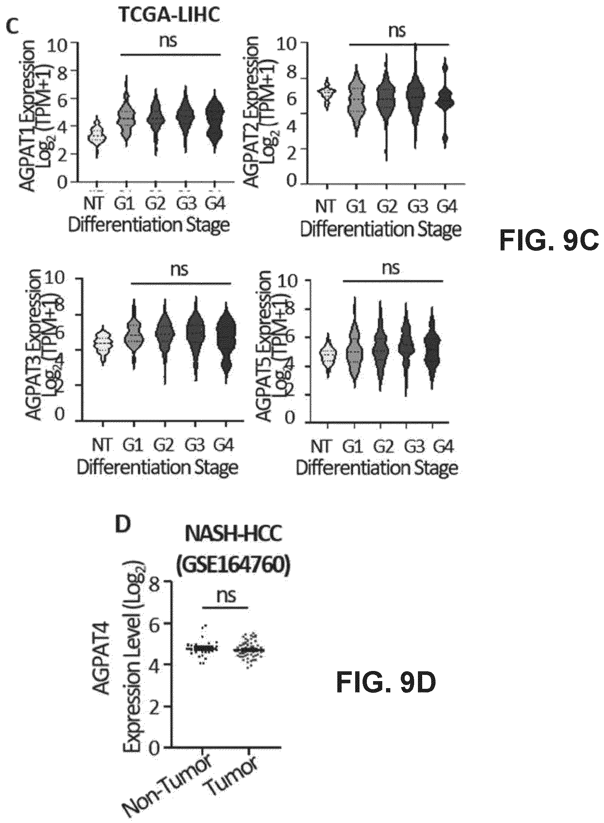

**FIG. 9A** is a bar graph showing AGPAT4 expression in normal human organs (44). **FIG. 9B** is a collection of dot plots showing AGPAT1, AGPAT2, AGPAT3 and AGPAT5 expression in The Cancer Genome Atlas (TCGA) Liver Hepatocellular Carcinoma (LIHC) dataset. **FIG. 9C** is a collection of violin plots showing AGPAT1, 2, 3 and 5 expressions in the TCGA-LIHC cohort stratified by histological grading (Edmondson-Steiner 4-tier). NT, non-tumor; G1, well-differentiated; G2, moderately differentiated; G3, poorly differentiated; G4, undifferentiated. **FIG. 9D** is a dot plot showing AGPAT4 expression in the GSE164760 dataset (comparing non-tumor liver and NASH-HCC) (45). ****p*<0.001. ns, not significant.

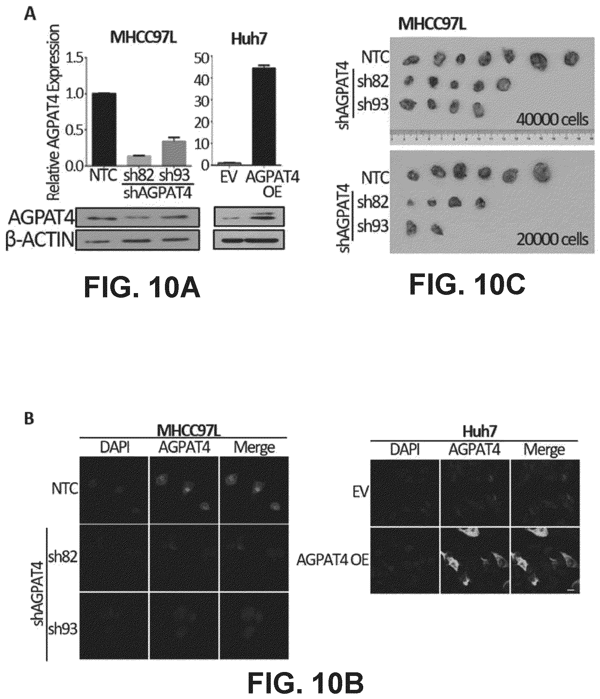

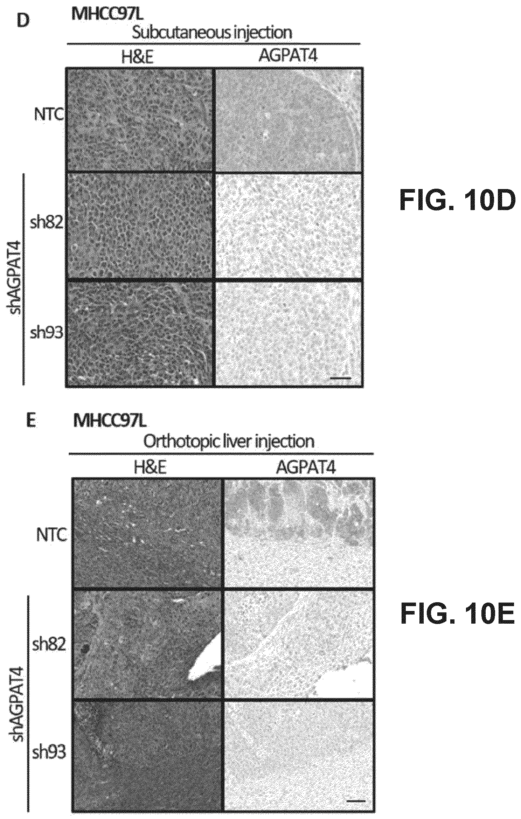

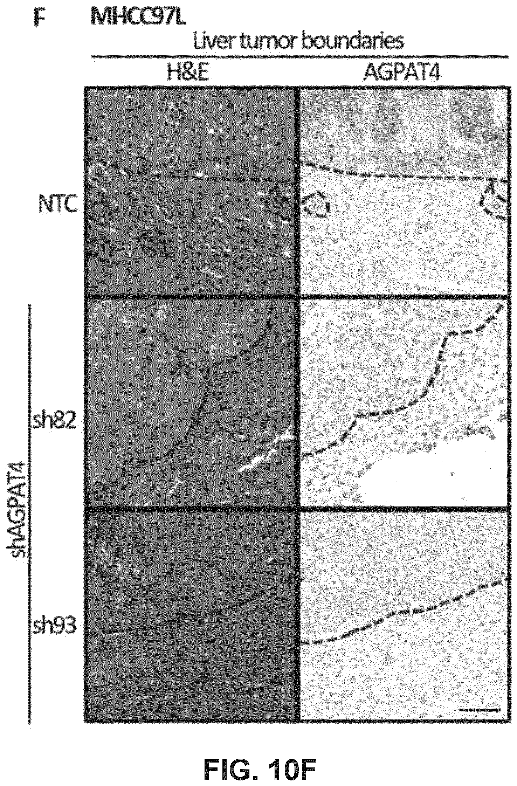

**FIG. 10A-B** provides validation of AGPAT4 expression in MHCC97L and Huh7 cells with or without AGPAT4 manipulation by qPCR, western blotting **(****FIG. 10A****),** and immunofluorescence staining **(****FIG. 10B****).** Scale bar = 20µm. **FIG. 10C** is a representative image of tumors formed by 40,000 and 20,000 MHCC97L cells, with or without AGPAT4 expression, transplanted subcutaneously into NOD-SCID mice. **FIG. 10D** are H&E and immunohistochemical images of AGPAT4 staining in subcutaneous HCC tumors harvested from each treatment group in FIG. 10C Scale bar = 100µm. **FIGs. 10E-10F** are H&E and immunohistochemistry images of AGPAT4 staining in orthotopic HCC tumors harvested from each treatment group, with liver tumor boundaries denoted in FIG. 10F. Scale bar = 100µm. OE, overexpression; NTC, shRNA scrambled non-targeting control; sh82 and sh93, two independent shRNA clones targeting AGPAT4.

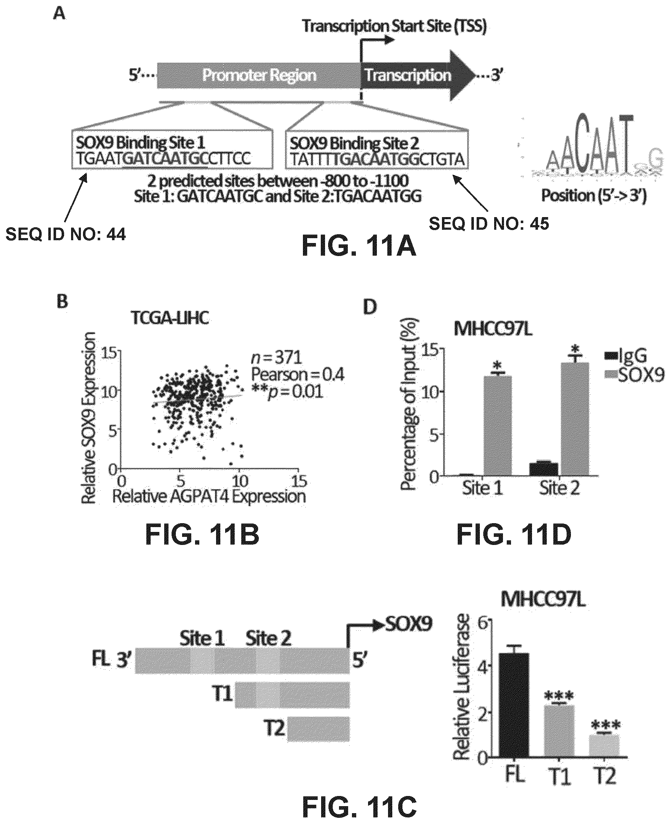

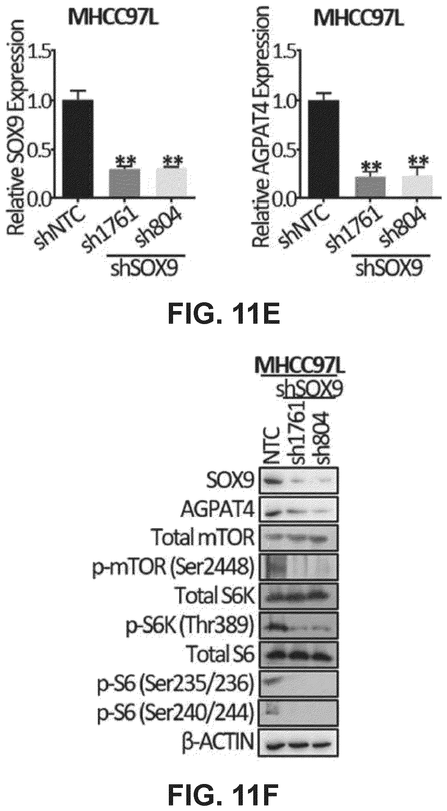

**FIG. 11A****,** left is a computational prediction of SOX9 binding sites (site 1: GATCAATGC at -1065 to -1057 and site 2: TGACAATGG at -862 to -854) on the AGPAT4 promoter region using the Gene Transcription Regulation Database (GTRD). **FIG. 11A****,** right shows consensus-binding motif of SOX9. **FIG. 11B** provides results from Pearson's correlation analysis of AGPAT4 and SOX9 mRNA expression in human HCC samples from the TCGA-LIHC dataset. **FIG. 11C** show reporter constructs generated by cloning full-length (FL) or truncated (T1 and T2) promoter fragments into a pGL3 vector encoding firefly luciferase. Luciferase reporter assays showing relative luciferase units (firefly/Renilla) in MHCC97L cells with FL or different truncations of AGPAT4 (T1 and T2) promoters. The pRL-CMV Renilla luciferase plasmid was cotransfected for normalization. **FIG. 11D** provides ChIP-qPCR confirmation of SOX9 binding to predicted sites (S1 and S2) on the AGPAT4 promoter in MHCC97L cells using SOX9 and IgG antibodies. **FIG. 11E** provides results from qPCR analysis of SOX9 and AGPAT4 expression in MHCC97L cells following lentivirus-mediated shRNA knockdown of SOX9. **FIG. 11F** are images from western blot analysis for the expression of SOX9, AGPAT4, total and phosphorylated forms of mTOR, S6K, and S6 in HCC cells with or without SOX9 expression repressed. **p*<0.05, ***p*<0.01, ****p*<0.001. TSS, transcription start site; NTC, shRNA scrambled non-targeting control; sh1761 and sh804, two independent shRNA clones targeting SOX9.

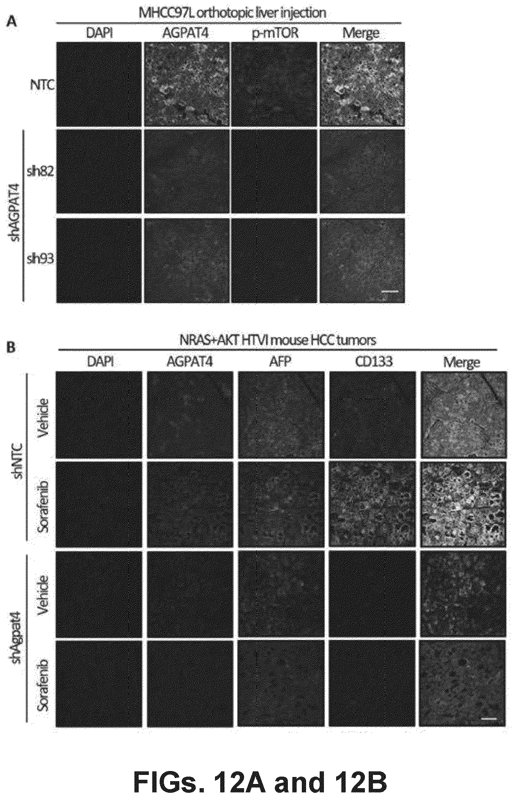

**FIG. 12A** are representative images from multiplex IHC staining for AGPAT4 and p-mTOR in orthotopic HCC tumors harvested from each treatment group (in reference to FIG.3M). Scale bar = 25µm. **FIG. 12B** are representative images from multiplex IHC staining for AGPAT4, AFP, and CD133 in liver tissues of NTC and AAV8-shAgpat4 mice treated with DMSO or sorafenib (FIG. 6D and 6H). Scale bar = 25µm.

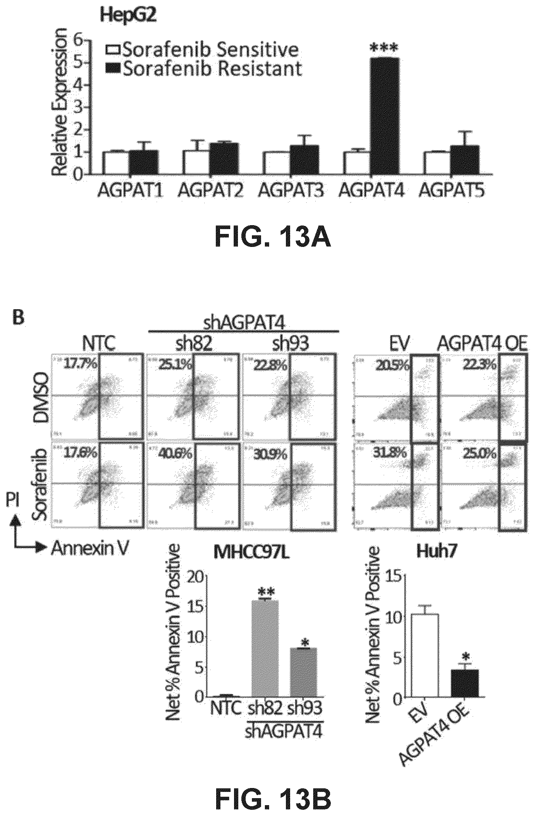

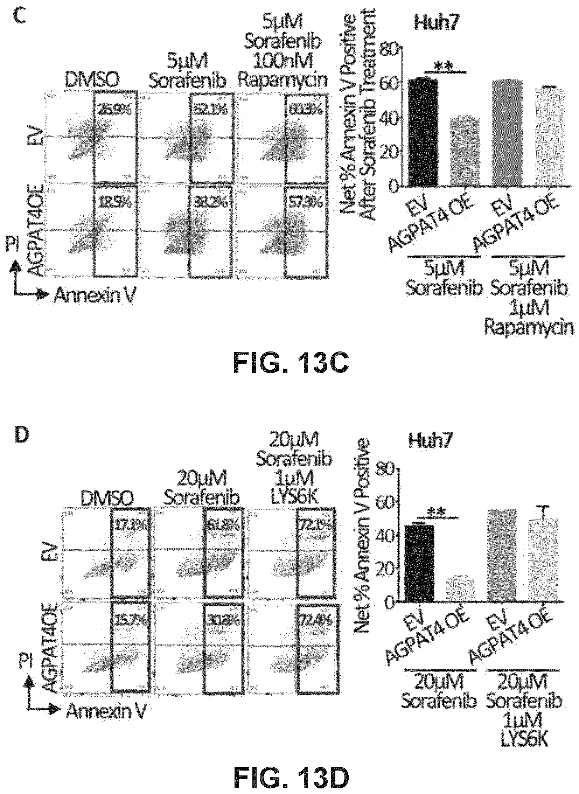

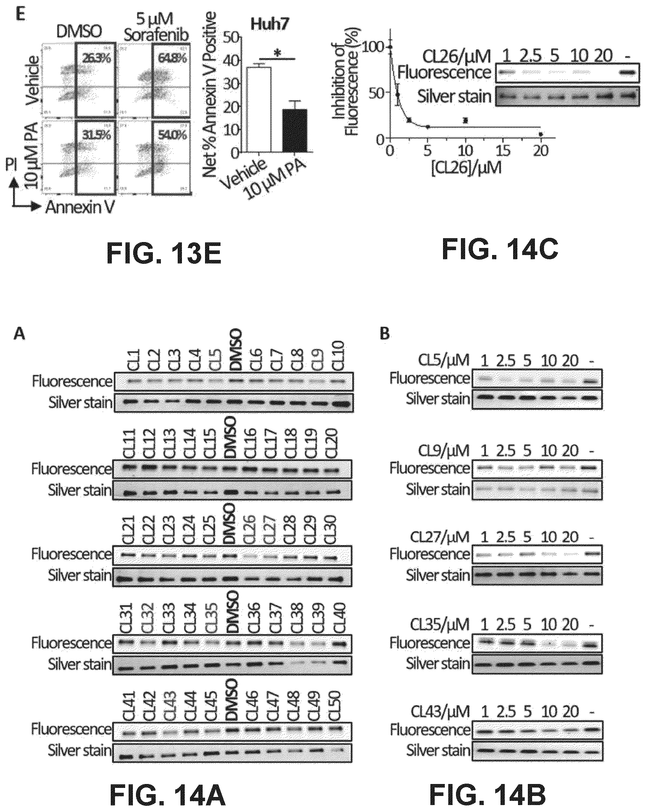

**FIG. 13A** is a bar graph showing qPCR analysis of AGPAT1, AGPAT2, AGPAT3, AGPAT4, and AGPAT5 expression in sorafenib-sensitive and-resistant HepG2 cells. **FIG. 13B** shows cell apoptosis upon sorafenib treatment as demonstrated by Annexin V-PI flow cytometry analysis in MHCC97L or Huh7 cells with or without AGPAT4 expression manipulated. **FIG. 13C-13D** shows cell apoptosis upon sorafenib treatment as demonstrated by Annexin V-PI flow cytometry analysis in Huh7 cells with or without AGPAT4 overexpressed and in the absence/presence of rapamycin **(****FIG. 13C****)** or LYS6K **(****FIG. 13D**). **FIG. 13E** shows the effect of exogenous PA on cell apoptosis following sorafenib treatment, as demonstrated by Annexin V-PI flow cytometry analysis in Huh7 cells. **p*<0.05, ***p*<0.01. NTC, shRNA scrambled non-targeting control; sh82 and sh93, two independent shRNA clones targeting AGPAT4.
**FIG. 14A** are blot images from gel based ABPP experiment for screening covalent ligands that strongly bind to AGPAT4 in vitro. Diminished in-gel fluorescence intensity upon compound treatment, as compared to the DMSO control, indicated compound binding to the AGPAT4 protein. **FIG. 14B and 14C** provide results from dose-dependent experiments investigating the binding affinity of lead compounds to AGPAT4.

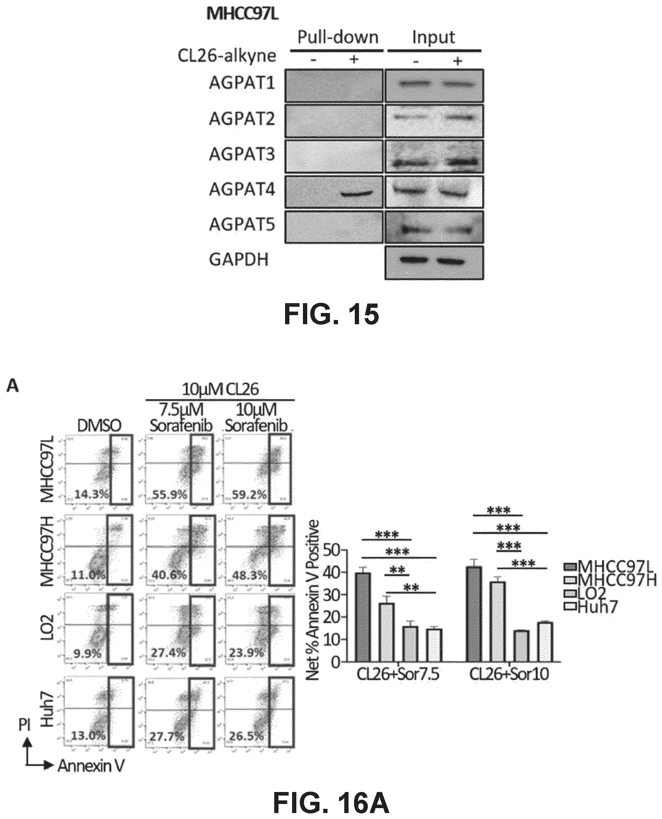

**FIG. 15** is a blot image showing isoform-specificity of CL26 as examined by enrichment and immunoblotting of CL26-alkyne-bound protein in MHCC97L cells.

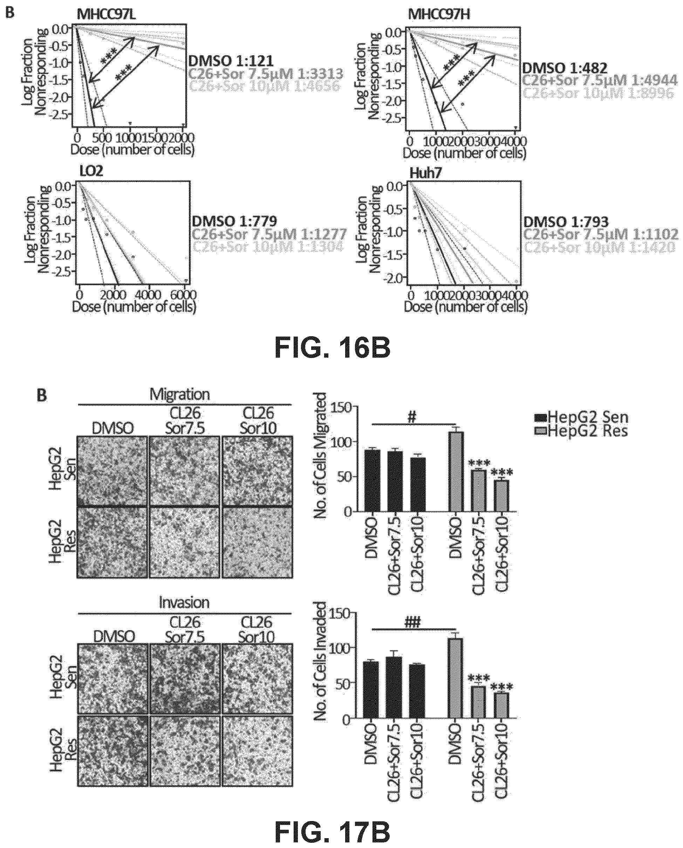

**FIG. 16A** shows cell apoptosis as demonstrated by Annexin V-PI flow cytometry analysis in high AGPAT4 expressing MHCC97L and MHCC97H vs. low AGPAT4 expressing Huh7 and LO2 cells treated with DMSO control or CL26 and various combinations of sorafenib. **FIG. 16B** shows results from *in vitro* limiting dilution assays for evaluating self-renewal abilities in high AGPAT4 expressing MHCC97L and MHCC97H vs. low AGPAT4 expressing Huh7 and LO2 cells when treated with DMSO control or CL26 and various combinations of sorafenib. ***p*<0.01, ****p*<0.001.

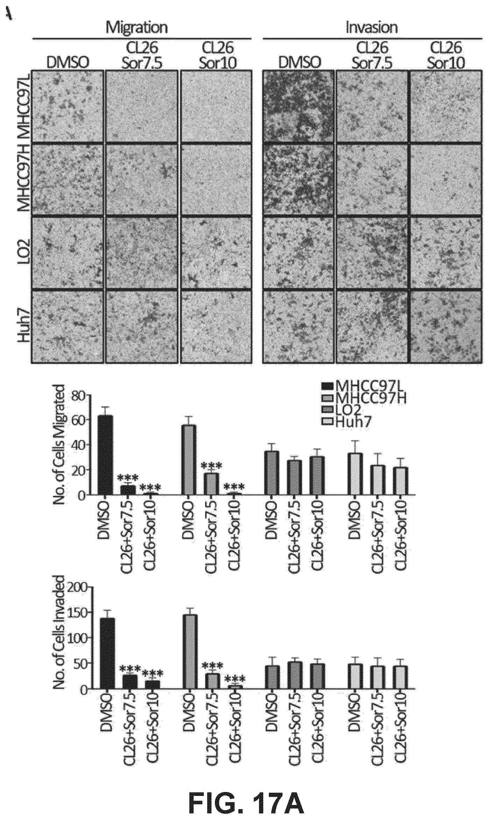

**FIG. 17A** are representative images and quantitation of the number of high AGPAT4 expressing MHCC97L and MHCC97H vs. low AGPAT4 expressing Huh7 and LO2 cells that migrated and invaded when treated with DMSO control or CL26 and various combinations of sorafenib. Scale bar = 20µm. **FIG. 17B** are representative images and quantitation of the number of sorafenib-sensitive and sorafenib-resistant HepG2 cells that migrated and invaded when treated with DMSO control or CL26 and various combinations of sorafenib. Scale bar = 20µm. ****p*<0.001, *#p*<0.05, ###*p*<0.001.

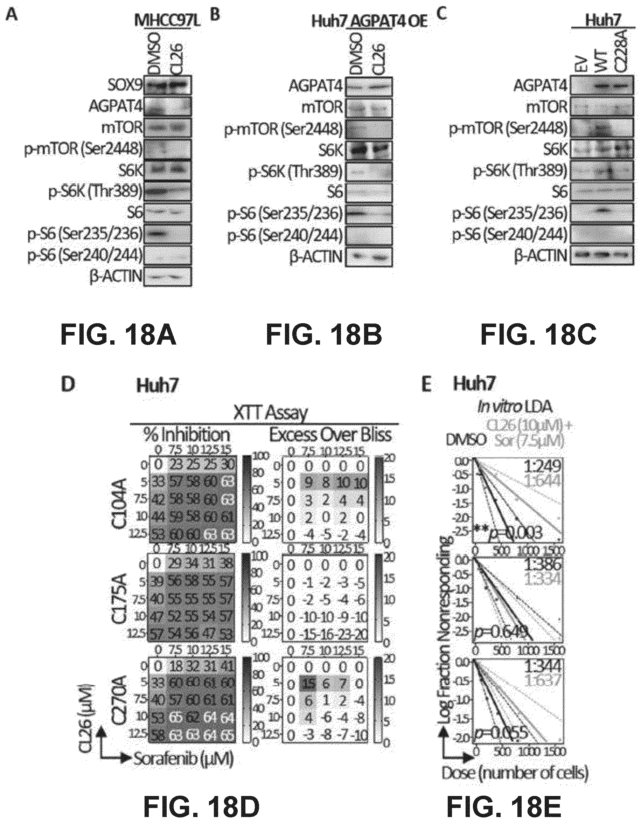

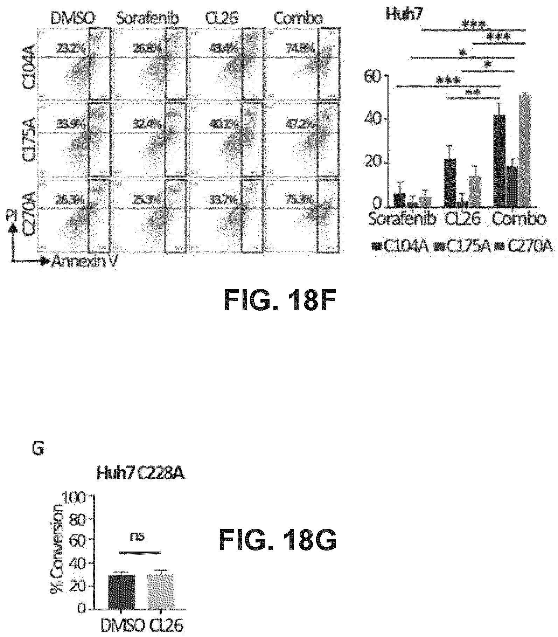

**FIGs. 18A-18C** are images from western blot analysis for expression of SOX9, AGPAT4, total and phosphorylated forms of mTOR, S6K, and S6 in high AGPAT4 expressing MHCC97L cells treated with DMSO or CL26 **(****FIG. 18A****),** Huh7 cells overexpressing AGPAT4 and treated with DMSO control or CL26 **(****FIG. 18B****),** and Huh7 cells overexpressing empty vector (EV) control, wild-type (WT) AGPAT4, or AGPAT4 with mutated cysteine at position 228 (C228A) **(****FIG. 18C). FIG. 18D** shows the synergistic effects of sorafenib and CL26 in Huh7 cells overexpressing AGPAT4 with mutated cysteine at positions 104 (C104A), 175 (C175A), or 270 (C270A). Refer to FIG. 7K for comparisons of the empty vector (EV) control and wild-type (WT) AGPAT4. **FIG. 18E** shows *in vitro* limiting dilution assays for evaluating self-renewal abilities in Huh7 cells that have AGPAT4 mutated C104A, C175A, or C270A, and when treated with CL26 and sorafenib. Refer to FIG. 7K for comparisons of the empty vector (EV) control and wild-type (WT) AGPAT4. **FIG. 18F** shows cell apoptosis as demonstrated by Annexin V-PI flow cytometry analysis in Huh7 cells overexpressing AGPAT4 with mutated C104A, C175A, or C270A, and when treated with CL26 and sorafenib. Refer to FIG. 7I for the empty vector (EV) control and wild-type (WT) AGPAT4 comparisons. **FIG. 18G** shows LPA to PA conversion in Huh7 cells transfected with AGPAT4 overexpressing mutated cysteine at position 228 (C288A), and treated with either DMSO control or CL26. **p*<0.05, ***p*<0.01, ****p*<0.001. ns, not significant.

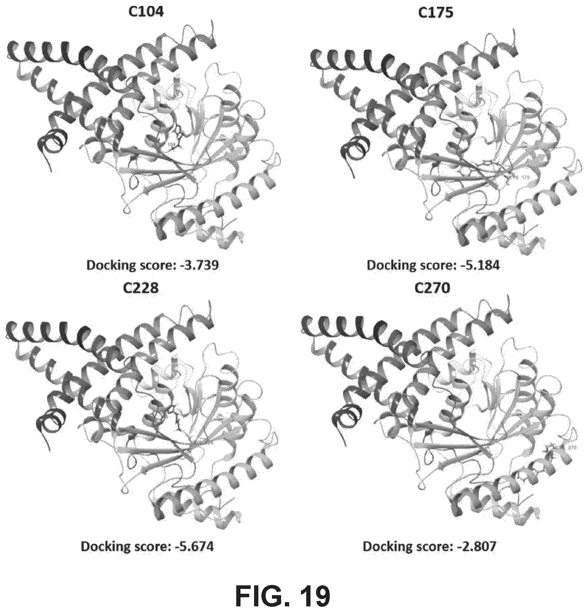

**FIG. 19** shows the covalent docking of CL26 onto different cysteine residues in AGPAT4 using the protein structure predicted by AlphaFold (AF-Q9NRZ5-F1). The lowest docking score for the binding model of CL26 with Cys228 indicates the strongest binding.

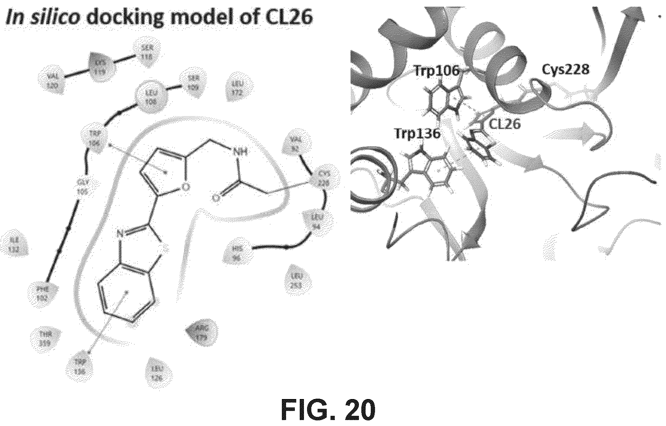

**FIG. 20** is a covalent docking model and ligand interaction map showing π-π interactions of the furan ring and phenyl ring on benzothiazole with Trp106 and Trp136 on AGPAT4, respectively, in addition to CL26-Cys228 covalent bond formation.

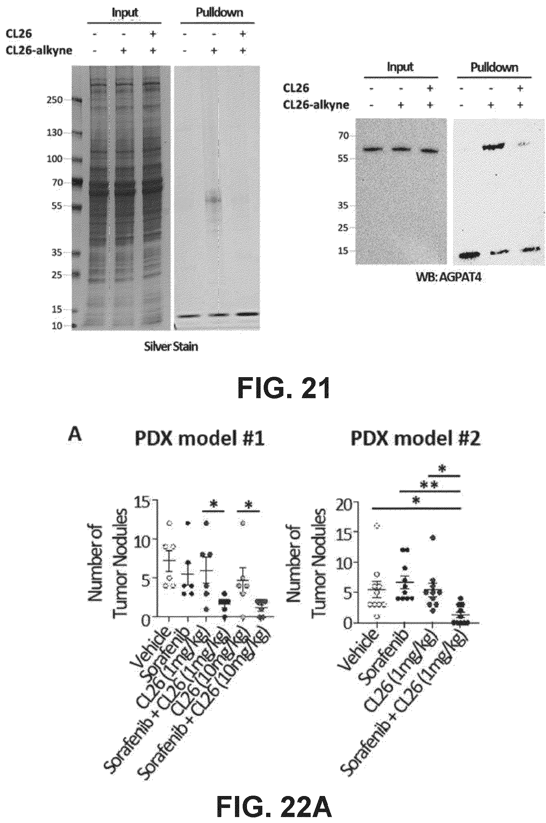

**FIG. 21** are blot images of CL26-alkyne-bound proteins in Huh7 cells expressing FLAG-AGPAT4 were enriched and silver-stained by SDS-PAGE. The major protein band found in the silver-stained gel was also examined by a parallel immunoblotting.

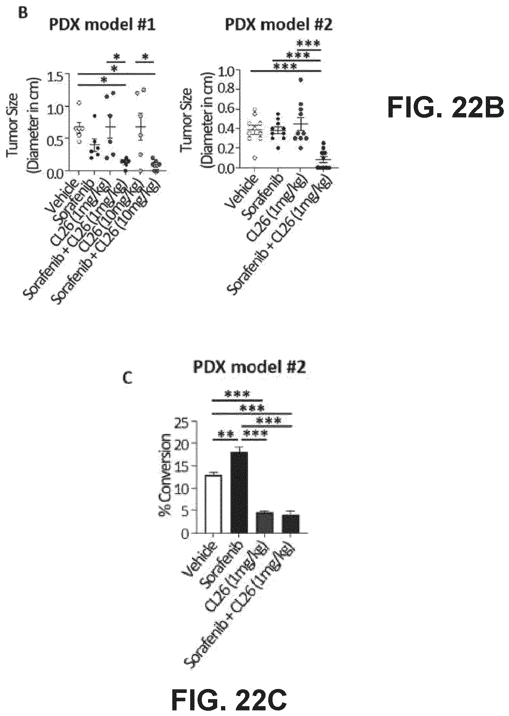

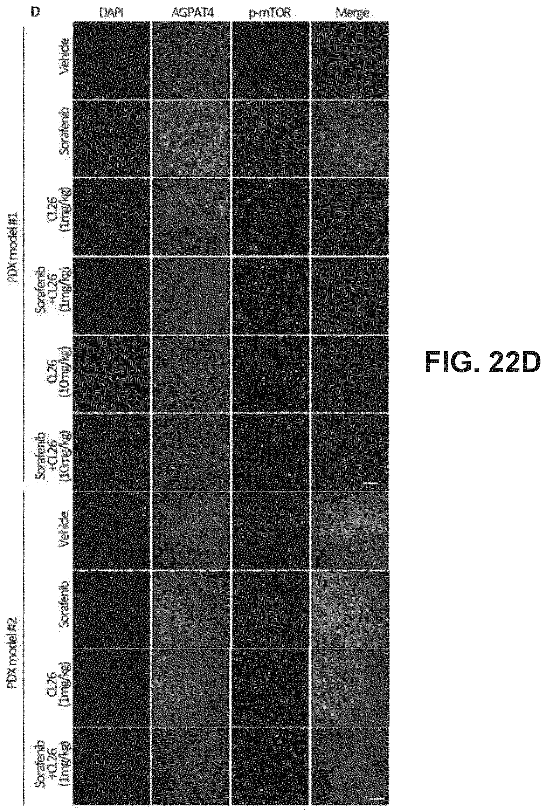

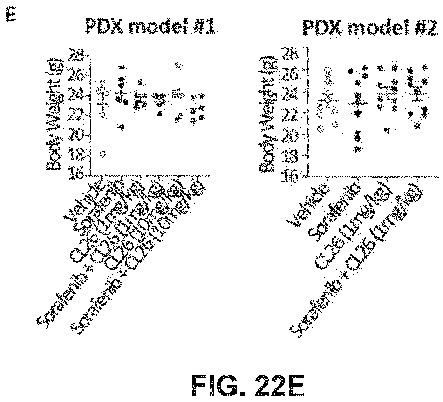

**FIG. 22A** **and** **22B** provide quantitative data on the number of tumor modules formed **(****FIG. 22A****)** and tumor size **(****FIG. 22B****)** in HCC PDX model #1 and sorafenib-resistant HCC PDX model #2 under various treatment conditions. **FIG. 22C**is a bar graph showing LPA to PA conversion in each treatment group of HCC PDX model #2. **FIG. 22D** are representative images from multiplex IHC staining for AGPAT4 and p-mTOR in orthotopic HCC tumors harvested from each treatment group in the two HCC PDX models. Scale bar = 25µm. **FIG. 22E** are dot plots showing body weight measurements in HCC PDX model #1 and sorafenib-resistant HCC PDX model #2 under various treatment conditions. **p*<0.05, ***p*<0.01, ****p*<0.001.

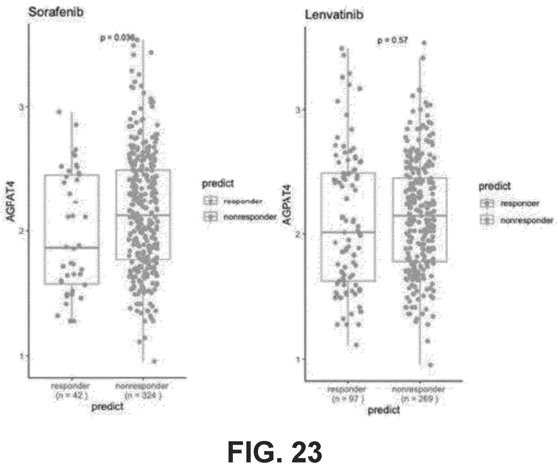

**FIG. 23** is a dot plot showing AGPAT4 expression in relation to sorafenib or lenvatinib responding and non-responding HCC patients via analysis on CARE: Computational Analysis of REsistance - http://care.dfci.harvard.edu/.

### DETAILED DESCRIPTION OF THE INVENTION

The disclosed compositions and methods are based at least on the discovery that that 1-acylglycerol-3-phosphate o-acyltransferase 4 (AGPAT4) upregulation in HCC is tightly correlated with aggressive clinical features, and that enhanced tumor lineage plasticity and cellular identity changes are induced by the AGPAT4-mediated phosphatidic acid production axis, leading to subsequent activation of the mTOR signaling pathway. AGPAT4 belongs to the AGPAT (also known as LPA acyltransferase) family of enzymes involved in de novo synthesis of triacylglycerol and glycerophospholipids. AGPAT contains five family members (namely AGPAT1, AGPAT2, AGPAT3, AGPAT4 and AGPAT5) and all are recognized for their lysophosphatidic acid acyltransferase activity that enables conversion of lysophosphatidic acid (LPA) to phosphatidic acid (PA) (Yamashita et al. Biology 2014). While AGPAT1, and 4 are all found elevated in HCC as compared to non-tumor liver, only AGPAT4 was found to be correlated with poor overall survival and cancer stemness. Further, only AGPAT4 was found to be elevated in sorafenib-resistant HCC.

### I. DEFINITIONS

"Hydrophilic" as used herein refers to substances that have strongly polar groups that readily interact with water.

"Hydrophobic" as used herein refers to substances that lack an affinity for water; tending to repel and not absorb water as well as not dissolve in or mix with water.

The term "combination therapy" refers to treatment of a disease or symptom thereof, or a method for achieving a desired physiological change, including administering to an animal, such as a mammal, especially a human being, an effective amount of two or more chemical agents or components to treat the disease or symptom thereof, or to produce the physiological change, wherein the chemical agents or components are administered together, such as part of the same composition, or administered separately and independently at the same time or at different times (*i.e.,* administration of each agent or component is separated by a finite period of time from each other).

The terms "single guide RNA" or "sgRNA" refer to the polynucleotide sequence comprising the guide sequence, tract sequence and the tract mate sequence. "Guide sequence" refers to the around 20 base pair (bp) sequence within the guide RNA that specifies the target site and may be used interchangeably with the terms "guide" or "spacer."

The term "dosing regimen" refers to drug administration regarding formulation, route of administration, drug dose, dosing interval and treatment duration.

The terms "individual", "host", "subject", and "patient" are used interchangeably, and refer to a mammal, including, but not limited to, murines, simians, humans, mammalian farm animals, mammalian sport animals, and mammalian pets. "Patient" or "subject" to be treated as used herein refers to either a human or non-human animal.

The term "effective amount" or "therapeutically effective amount" refers to the amount which is able to treat one or more symptoms of hepatocellular carcinoma (HCC), reverse the progression of one or more symptoms of HCC, halt the progression of one or more symptoms of HCC, or prevent the occurrence of one or more symptoms of HCC in a subject to whom the formulation is administered, for example, as compared to a matched subject not receiving the compound. The actual effective amounts of compound can vary according to the specific compound or combination thereof being utilized, the particular composition formulated, the mode of administration, and the age, weight, condition of the individual, and severity of the symptoms or condition being treated.

The term "pharmaceutically acceptable" refers to compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions, or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient.

The term "pharmaceutically acceptable salt" is art-recognized, and includes relatively non-toxic, inorganic, and organic acid addition salts of compounds. Examples of pharmaceutically acceptable salts include those derived from mineral acids, such as hydrochloric acid and sulfuric acid, and those derived from organic acids, such as ethanesulfonic acid, benzenesulfonic acid, and p-toluenesulfonic acid. Examples of suitable inorganic bases for the formation of salts include the hydroxides, carbonates, and bicarbonates of ammonia, sodium, lithium, potassium, calcium, magnesium, aluminum, and zinc. Salts may also be formed with suitable organic bases, including those that are non-toxic and strong enough to form such salts. For purposes of illustration, the class of such organic bases may include mono-, di-, and trialkylamines, such as methylamine, dimethylamine, and triethylamine; mono-, di- or trihydroxyalkylamines such as mono-, di-, and triethanolamine; amino acids, such as arginine and lysine; guanidine; N-methylglucosamine; N-methylglucamine; L-glutamine; N-methylpiperazine; morpholine; ethylenediamine; N-benzylphenethylamine;

The terms "inhibit" or "reduce" in the context of inhibition, mean to reduce, or decrease in activity and quantity. This can be a complete inhibition or reduction in activity or quantity, or a partial inhibition or reduction. Inhibition or reduction can be compared to a control or to a standard level. Inhibition can be measured as a % value, e.g., from 1% up to 100%, such as 5%, 10, 25, 50, 75, 80, 85, 90, 95, 99, or 100%. For example, compositions including lipocalin-2 antagonists may inhibit or reduce the activity and/or quantity of one or more lipocalin-2 protein or variants thereof by about 10%, 20%, 30%, 40%, 50%, 75%, 85%, 90%, 95%, or 99% from the activity and/or quantity of the same lipocalin-2 protein or variants thereof in subjects that did not receive or were not treated with the compositions. In some embodiments, the inhibition and reduction are compared according to the level of mRNAs, proteins, cells, tissues, and organs.

The terms "treating" mean to ameliorate, reduce or otherwise stop a disease, disorder or condition from occurring or progressing in an animal which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain. Desirable effects of treatment include decreasing the rate of disease progression, ameliorating, or palliating the disease state, and remission or improved prognosis. For example, an individual is successfully "treated" if one or more symptoms associated with HCC are mitigated or eliminated, including, but are not limited to, reducing and/or inhibiting rate of tumor cell proliferation/growth, increasing the quality of life of those suffering from the disease, decreasing the dose of other medications required to treat the disease, delaying the progression of the disease, and/or prolonging survival of individuals.

The term 'treating HCC" or "HCC treatment" means reducing, inhibiting, or alleviating one or more symptoms related to HCC in a subject suffering from HCC.

The term "alkyl" refers to the radical of saturated aliphatic groups (i.e., an alkane with one hydrogen atom removed), including straight-chain alkyl groups, branched-chain alkyl groups, cycloalkyl (alicyclic) groups, alkyl-substituted cycloalkyl groups, and cycloalkyl-substituted alkyl groups.

In preferred embodiments, a straight chain or branched chain alkyl has 30 or fewer carbon atoms in its backbone (e.g., C1-C30 for straight chains, and C3-C30 for branched chains), preferably 20 or fewer, more preferably 15 or fewer, most preferably 10 or fewer. Likewise, preferred cycloalkyls have 3-10 carbon atoms in their ring structure, and more preferably have 5, 6, or 7 carbons in the ring structure. The term "alkyl" (or "lower alkyl") as used throughout the specification, examples, and claims is intended to include both "unsubstituted alkyls" and "substituted alkyls", the latter of which refers to alkyl moieties having one or more substituents replacing a hydrogen on one or more carbons of the hydrocarbon backbone. Such substituents include, but are not limited to, halogen, hydroxyl, carbonyl (such as a carboxyl, alkoxycarbonyl, formyl, or an acyl), thiocarbonyl (such as a thioester, a thioacetate, or a thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or an aromatic or heteroaromatic moiety.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Throughout the application, preferred alkyl groups are lower alkyls. In preferred embodiments, a substituent designated herein as alkyl is a lower alkyl.

It will be understood by those skilled in the art that the moieties substituted on the hydrocarbon chain can themselves be substituted, if appropriate. For instance, the substituents of a substituted alkyl may include halogen, hydroxy, nitro, thiols, amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF3, -CN and the like. Cycloalkyls can be substituted in the same manner.

The term "heteroalkyl", as used herein, refers to straight or branched chain, or cyclic carbon-containing radicals, or combinations thereof, containing at least one heteroatom. Suitable heteroatoms include, but are not limited to, O, N, Si, P, Se, B, and S, wherein the phosphorous and sulfur atoms are optionally oxidized, and the nitrogen heteroatom is optionally quaternized. Heteroalkyls can be substituted as defined above for alkyl groups.

The terms "alkenyl" and "alkynyl", refer to unsaturated aliphatic groups analogous in length and possible substitution to the alkyls described above, but that contain at least one double or triple bond respectively.

The terms "alkoxyl" or "alkoxy" as used herein refers to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, and -O-alkynyl. The terms "aroxy" and "aryloxy", as used interchangeably herein, can be represented by -O-aryl or O-heteroaryl, wherein aryl and heteroaryl are as defined below. The alkoxy and aroxy groups can be substituted as described above for alkyl.

"Aryl", as used herein, refers to C5-C10-membered aromatic, heterocyclic, fused aromatic, fused heterocyclic, biaromatic, or bihetereocyclic ring systems. Broadly defined, "aryl", as used herein, includes 5-, 6-, 7-, 8-, 9-, and 10-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine, pyrimidine, and the like. Those aryl groups having heteroatoms in the ring structure may also be referred to as "aryl heterocycles" or "heteroaromatics". The aromatic ring can be substituted at one or more ring positions with one or more substituents including, but not limited to, halogen, azide, alkyl, aralkyl, alkenyl, alkynyl, cycloalkyl, hydroxyl, alkoxyl, amino (or quaternized amino), nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF3, -CN, and combinations thereof.

The term "aryl" also includes polycyclic ring systems having two or more cyclic rings in which two or more carbons are common to two adjoining rings (i.e., "fused rings") wherein at least one of the rings is aromatic, e.g., the other cyclic ring or rings can be cycloalkyls, cycloalkenyls, cycloalkynyls, aryls and/or heterocycles. Examples of heterocyclic rings include, but are not limited to, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH carbazolyl, carbolinyl, chromanyl, chromenyl, cinnolinyl, decahydroquinolinyl, 2H,6H-1,5,2-dithiazinyl, dihydrofuro[2,3 b]tetrahydrofuran, furanyl, furazanyl, imidazolidinyl, imidazolinyl, imidazolyl, 1H-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, and xanthenyl. One or more of the rings can be substituted as defined above for "aryl".

The term "aralkyl", as used herein, refers to an alkyl group substituted with an aryl group (e.g., an aromatic or heteroaromatic group).

The term "aralkyloxy" can be represented by -O-aralkyl, wherein aralkyl is as defined above.

The term "heteroatom" as used herein means an atom of any element other than carbon or hydrogen. Preferred heteroatoms are boron, nitrogen, oxygen, phosphorus, sulfur, and selenium. Other heteroatoms include silicon and arsenic.

As used herein, the term "nitro" means -NO2; the term "halogen" designates -F, - Cl, -Br, or -I; the term "sulfhydryl" means -SH; the term "hydroxyl" means -OH; and the term "sulfonyl" means -SO2-.

The term "substituted" as used herein, refers to all permissible substituents of the compounds described herein. In the broadest sense, the permissible substituents include acyclic and cyclic, branched, and unbranched, carbocyclic and heterocyclic, aromatic and nonaromatic substituents of organic compounds. Illustrative substituents include, but are not limited to, halogens, hydroxyl groups, or any other organic groupings containing any number of carbon atoms, preferably 1-14 carbon atoms, and optionally include one or more heteroatoms such as oxygen, sulfur, or nitrogen grouping in linear, branched, or cyclic structural formats. Representative substituents include alkyl, substituted alkyl, alkenyl, substituted alkenyl, alkynyl, substituted alkynyl, phenyl, substituted phenyl, aryl, substituted aryl, heteroaryl, substituted heteroaryl, halo, hydroxyl, alkoxy, substituted alkoxy, phenoxy, substituted phenoxy, aroxy, substituted aroxy, alkylthio, substituted alkylthio, phenylthio, substituted phenylthio, arylthio, substituted arylthio, cyano, isocyano, substituted isocyano, carbonyl, substituted carbonyl, carboxyl, substituted carboxyl, amino, substituted amino, amido, substituted amido, sulfonyl, substituted sulfonyl, sulfonic acid, phosphoryl, substituted phosphoryl, phosphonyl, substituted phosphonyl, polyaryl, substituted polyaryl, C3-C20 cyclic, substituted C3-C20 cyclic, heterocyclic, substituted heterocyclic, amino acid, peptide, and polypeptide groups.

### II. COMPOSITIONS

The disclosed compositions include AGPAT4 inhibitors (also referred to herein as "AGPAT4 inhibitors" or "compounds"), which can be provided in a pharmaceutical formulation. The AGPAT4 inhibitor include small molecules as well as nucleic acid molecules.

### A. Compounds

Compounds for inhibiting or reducing AGPAT4 activity are provided.

Through competitive binding as identified by gel-based and mass spectrometry (MS)-based ABPP experiments, the high-affinity and selective covalent inhibitor targeting AGPAT4, CL26 was discovered from a library of cysteine-reactive compounds. Activity-based protein profiling is a technology to identify the binding of small molecule probes with proteins and confirm direct interaction. Activity-Based Protein Profiling (ABPP) combines activity-based probe and proteomics technologies together to facilitate understanding of the mechanisms of compounds and their modes of action (reviewed in Wang et al., Front. Pharmacol., 9:353 (2018).

The disclosed compounds bind to the cysteine residue, Cys228 and engage in an π-π interaction with W106, thus reducing AGPAT4 activity (FIGs. 7A, 7B, 14A-C; and FIG. 15). The AGPAT4 inhibitors can covalently bind AGPAT4, and thereby achieve improved inhibitory efficacy (i.e., an IC₅₀ value of about 766 nM). The combination of high AGPAT4 inhibitory efficacy and high AGPAT4 specificity of the disclosed AGPAT4 inhibitors allow these compounds to show high cytotoxicity in cancer cells e.g., hepatocellular carcinoma (HCC) cells, with negligible toxicity in normal cells, in *vitro* and in *vivo.* For example, as demonstrated by the data in the Examples below, it has been demonstrated that AGPAT4 blockade by isoform-specific small-molecule inhibitors targeting the cysteine residue Cys228 which resides adjacent to the catalytic motif, sensitizes HCC tumors, and elicits a robust and synergistic response to sorafenib (FIGs. 7A-7K).

The disclosed compounds generally have a structure of Formula I, and include a pharmaceutically acceptable salt, a prodrug, analog, or derivative thereof; or a pharmaceutically acceptable salt of a prodrug, analog, or derivative of the inhibitor represented by Formula I. 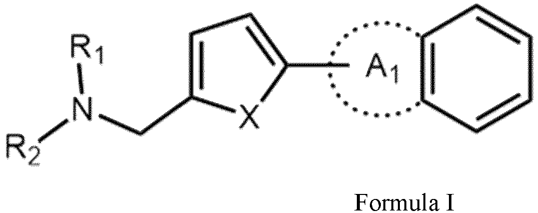 wherein:
(i) A₁ is a C₅-C₆ aromatic ring or a C₄-C₅ heteroaromatic ring;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4;
(iv) X is O, S, NR₃, PR₃, CR₃R₄ or SiR₃R₄ where R₃ and R₄ are independently a hydrogen, a deuterium, a substituted or unsubstituted alkyl, or a substituted or unsubstituted aryl.

In some forms, the compounds generally have a structure of Formula II: 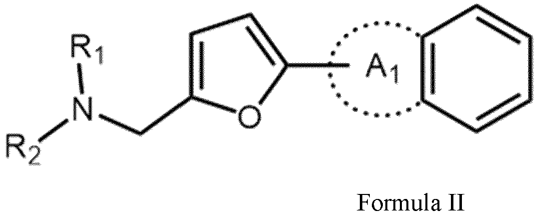 wherein:
(i) A₁ is a C₅-C₆ aromatic ring or a C₄-C₅ heteroaromatic ring;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4.

"Analog" or "derivative" as relates to a given compound, refers to another compound that is structurally similar, functionally similar, or both, to the specified compound. Structural similarity can be determined using any criterion known in the art, such as the Tanimoto coefficient which provides a quantitative measure of similarity between two compounds based on their molecular descriptors. Preferably, the molecular descriptors are 2D properties such as fingerprints, topological indices, and maximum common substructures, or 3D properties such as overall shape, and molecular fields. Tanimoto coefficients range between zero and one, inclusive, for dissimilar and identical pairs of molecules, respectively. A compound can be considered an analog of a specified compound, if it has a Tanimoto coefficient with the selected compound is between 0.5 and 1.0, inclusive, preferably between 0.7 and 1.0, inclusive, most preferably between 0.85 and 1.0, inclusive. A compound is functionally similar to a specified, if it induces the same pharmacological effect, physiological effect, or both, as the specified compound. "Analog" can also refer to a modification including, but not limited to, hydrolysis, reduction, or oxidation products, of the disclosed compounds. Hydrolysis, reduction, and oxidation reactions are known in the art.

In some forms, the compounds generally have a structure of Formula III: 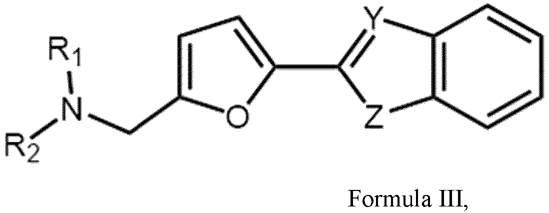 wherein:
(i) Y is N, P, CR3 or SiR3 where R3 is a hydrogen, a deuterium, a substituted or unsubstituted alkyl, or a substituted or unsubstituted aryl;
(ii) Z is O, S, NR4, PR4, CR4R5 or SiR4R5 where R4 and R5 are independently a hydrogen, a deuterium, a substituted or unsubstituted alkyl, or a substituted or unsubstituted aryl;
(ii) R1 is a hydrogen, a deuterium, a substituted or unsubstituted alkyl, or a substituted or unsubstituted aryl;
(iii) R2 is a cysteine-reactive group (such as haloacetamide, acrylamide, maleimide, vinyl sulfone and epoxide), which allows chemical reaction with cysteines on AGPAT4.

In some forms, the compounds generally have a structure of Formula IV: 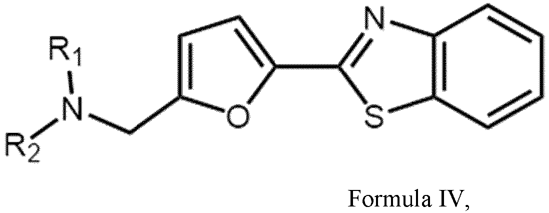 wherein:
(i) R₁ is a hydrogen, a deuterium, a substituted or unsubstituted alkyl, or a substituted or unsubstituted aryl;
(ii) R₂ is a cysteine-reactive group (such as haloacetamide, acrylamide, maleimide, vinyl sulfone and epoxide), which allows chemical reaction with cysteines on AGPAT4.

In some forms, the compounds generally have a structure of Formula V: 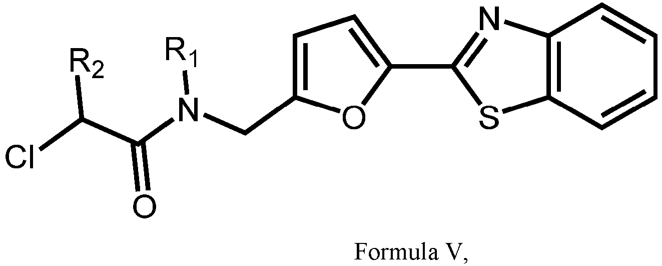 wherein:
(i) R₁ is a hydrogen, a deuterium, a substituted or unsubstituted alkyl, or a substituted or unsubstituted aryl;
(ii) R₂ is a hydrogen, a deuterium, a halogen, a substituted or unsubstituted alkyl, or a substituted or unsubstituted aryl.

A representative compound, N-[[5-(2-benzothiazolyl)-2-furanyl]methyl]-2-chloroacetamide or termed as CL26,

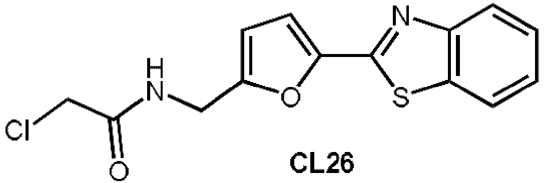

In some forms, the compound is a derivative of CL26. Derivatives that do not diminish binding to Cys228 are preferred. The term "derivative" does not mean that the derivative is synthesized from the parent compound either as a starting material or intermediate, although this may be the case. The term "derivative" can include salts, prodrugs, or metabolites of the parent compound. Derivatives include compounds in which free amino groups in the parent compound have been derivatized to form amine hydrochlorides, p-toluene sulfoamides, benzoxycarboamides, t-butyloxycarboamides, thiourethane-type derivatives, trifluoroacetylamides, chloroacetylamides, or formamides. Derivatives include replacing one or more amino substituents or hydrogen groups with substituted or unsubstituted alkyl, aminoalkyl, aryl, or heteroaryl groups having from 1 to 30 carbon atoms.

In some forms, the disclosed AGPAT4 inhibitors include a probe, such as an alkyne group, for biological studies of AGPAT4 functions and activities in cancers, as well as studies of the disclosed AGPAT4 inhibitors in cancer therapy. For example, as shown in the Examples below, a molecular probe of CL26, CL26-alkyne, was used to validate the specific binding of CL26 with AGPAT4. The structure of an exemplary molecular probe is presented below.

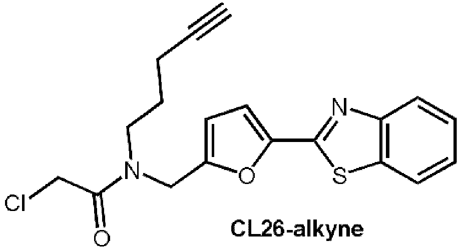

### 1. Pharmaceutically Acceptable Salts

Also disclosed herein are pharmaceutically acceptable salts of the compound represented by Formula I, and the derivatives thereof as well as other compounds that were determined to bind the cysteine residue Cys228. Examples of pharmaceutically acceptable salts include but are not limited to mineral or organic acid salts of basic residues such as amines; and alkali or organic salts of acidic residues such as carboxylic acids. The pharmaceutically acceptable salts include the conventional non-toxic salts or the quaternary ammonium salts of the parent compound formed, for example, from non-toxic inorganic or organic acids. Such conventional non-toxic salts include those derived from inorganic acids such as hydrochloric, hydrobromic, sulfuric, sulfamic, phosphoric, and nitric acids; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, naphthalenesulfonic, methanesulfonic, ethane disulfonic, oxalic, and isethionic salts.

The compounds described herein may be neutral or may be one or more pharmaceutically acceptable salts, crystalline forms, non-crystalline forms, hydrates, or solvates, or a combination thereof. References to the compounds may refer to the neutral molecule, and/or those additional forms thereof collectively and individually from the context. Pharmaceutically acceptable salts of the compounds include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include aluminum, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

The pharmaceutically acceptable salts of the compounds can be synthesized from the parent compound, which contains a basic or acidic moiety, by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 20th ed., Lippincott Williams & Wilkins, Baltimore, MD, 2000, p. 704; and "Handbook of Pharmaceutical Salts: Properties, Selection, and Use," P. Heinrich Stahl and Camille G. Wermuth, Eds., Wiley-VCH, Weinheim, 2002.

### 2. Intermediates

The compounds identified herein using virtual ligand screening can be used as a starting compound or intermediate compound to produce a final compound that has increased binding to a residue, for example Cys228, or increased inhibition of the residue, for example Cys228, relative to starting compound or intermediate compound. The compounds identified herein can be modified to increase bioavailability, increase half-life in the blood stream, increase solubility, increase hydrophilicity, increase hydrophobicity, or a combination thereof using conventional techniques.

The compounds can be modified to incorporate polar functional groups, such as the alcohol, amine, amide, carboxylic acid, sulfonic acid, and phosphate groups, which either ionize or are capable of relatively strong intermolecular forces of attraction with water (hydrogen bonding), usually resulting in analogues with an increased water solubility. Acidic and basic groups are particularly useful, since these groups can be used to form salts, which would give a wider range of dosage forms for the final product. However, the formation of zwitterions by the introduction of either an acid group into a structure containing a base or a base group into a structure containing an acid group can reduce water solubility. Introduction of weakly polar groups, such as carboxylic acid esters, aryl halides and alkyl halides, will not significantly improve water solubility and can result in enhanced lipid solubility.

The incorporation of acidic residues into the compound is less likely to change the type of activity, but it can result in the analogue exhibiting hemolytic properties. Furthermore, the introduction of an aromatic acid group usually results in antiinflammatory activity, whilst carboxylic acids with an alpha functional group may act as chelating agents. Basic water solubilizing groups tend to change the mode of action, since bases often interfere with neurotransmitters and biological processes involving amines. However, their incorporation does mean that the analogue can be formulated as a wide variety of acid salts. Non-ionizable groups do not have the disadvantages of acidic and basic groups.

Groups that are bound directly to the carbon skeleton of the lead compound by less reactive C-C, C-O and C-N bonds are likely to be irreversibly attached to the lead structure. Groups that are linked to the compound by ester, amide, phosphate, sulfate, and glycosidic bonds are more likely to be metabolized from the resulting analogue to reform the parent compound as the analogue is transferred from its point of administration to its site of action. Compounds with this type of solubilizing group are acting as prodrugs and so their activity is more likely to be the same as the parent compound. However, the rate of loss of the solubilizing group will depend on the nature of the transfer route, and this could affect the activity of the drug.

To preserve the type of activity exhibited by the compound, a water solubilizing group should be attached to a part of the structure that is not involved in the drug-receptor interaction. Consequently, the route used to introduce a new water solubilizing group and its position in the lead compound will depend on the relative reactivities of the compound and the rest of the molecule. Examples of water solubilizing structures and the routes used to introduce them into the lead structures. O-alkylation, N-alkylation, O-acylation, and N-acylation reactions are used to introduce both acidic and basic groups. Acetylation methods use both the appropriate acid chloride and anhydride.

Examples of water solubilizing structures and the routes used to introduce them into lead compounds include but are not limited to phosphate acid halides for introducing phosphate groups into compounds. Structures containing hydroxy groups have been introduced by reaction of the corresponding monochlorinated hydrin and the use of suitable epoxides. Sulphonic acid groups may be introduced by either direct sulfonation or the addition of bisulfite to reactive C = C bonds.

### B. Inhibitory Nucleic Acids

The data in the present application demonstrates that knockdown of endogenous AGPAT4 expression significantly reduces tumorigenicity, with a decrease in tumor incidence and tumor-initiating frequency (Table 1), reduces the frequency of tumor-initiating cells capable of forming spheres (FIG. 2G), and extends survival (FIG. 2E).

Therefore, inhibitory nucleic acid compositions for reducing AGPAT4 expression such as siRNA and ShRNA are provided.

The human AGPAT4 nucleic acid sequence contains 144,095 base pairs (NCBI Gene ID: 56895). AGPAT4 expression can be reduced using a functional nucleic acid (herein, AGPAT4 RNA-inhibiting NA), or vector encoding the same, which reduces expression of AGPAT4. Examples include but are not limited to antisense oligonucleotides (ASOs), small interfering RNAs (siRNAs), short hairpin RNAs (shRNA), microRNAs (miRNA), antisense oligoribonucleotides (EGSs), ribozymes, and aptamers (nucleic acid and peptide aptamers). In some forms, AGPAT4 expression is reduced in a subject in need thereof, using an siRNA or shRNA.

### 1. RNA Interference

In some embodiments, AGPAT4 RNA expression is reduced through RNA interference (RNAi). This silencing was originally observed with the addition of double stranded RNA (dsRNA) (Fire, et al. (1998) Nature, 391:806-11; Napoli, et al. (1990) Plant Cell 2:279-89; Hannon, (2002) Nature, 418:244-51). Once dsRNA enters a cell, it is cleaved by an RNase III -like enzyme, Dicer, into double stranded small interfering RNAs (siRNA) 21-23 nucleotides in length that contains 2 nucleotide overhangs on the 3' ends (Elbashir, et al. (2001) Genes Dev., 15:188-200; Bernstein, et al. (2001) Nature, 409:363-6; Hammond, et al. (2000) Nature, 404:293-6). In an ATP dependent step, the siRNAs become integrated into a multi-subunit protein complex, commonly known as the RNAi induced silencing complex (RISC), which guides the siRNAs to the target RNA sequence (Nykanen, et al. (2001) Cell, 107:309-21). At some point, the siRNA duplex unwinds, and it appears that the antisense strand remains bound to RISC and directs degradation of the complementary mRNA sequence by a combination of endo and exonucleases (Martinez, et al. (2002) Cell, 110:563-74). However, the effect of RNAi or siRNA or their use is not limited to any type of mechanism.

Short Interfering RNA (siRNA) is a double-stranded RNA that can induce sequence-specific post-transcriptional gene silencing, thereby decreasing or even inhibiting gene expression. In one example, a siRNA triggers the specific degradation of homologous RNA molecules, such as mRNAs, within the region of sequence identity between both the siRNA and the target RNA. For example, WO 02/44321 discloses siRNAs capable of sequence-specific degradation of target mRNAs when base-paired with 3' overhanging ends, herein incorporated by reference for the method of making these siRNAs.

Sequence specific gene silencing can be achieved in mammalian cells using synthetic, short double-stranded RNAs that mimic the siRNAs produced by the enzyme dicer (Elbashir, et al. (2001) Nature, 411:494 498) (Ui-Tei, et al. (2000) FEBS Lett 479:79-82). SiRNA can be chemically or *in* vitro-synthesized or can be the result of short double-stranded hairpin-like RNAs (shRNAs) that are processed into siRNAs inside the cell. Synthetic siRNAs are generally designed using algorithms and a conventional DNA/RNA synthesizer. Suppliers include Ambion (Austin, Texas), ChemGenes (Ashland, Massachusetts), Dharmacon (Lafayette, Colorado), Glen Research (Sterling, Virginia), MWB Biotech (Esbersberg, Germany), Proligo (Boulder, Colorado), and Qiagen (Vento, The Netherlands). SiRNA can also be synthesized *in vitro* using kits such as Ambion's SILENCER^{®} siRNA Construction Kit.

The production of siRNA from a vector is more commonly done through the transcription of a short hairpin RNAse (shRNAs). Kits for the production of vectors comprising shRNA are available, such as, for example, Imgenex's GENESUPPRESSOR^{™} Construction Kits and Invitrogen's BLOCK-IT^{™} inducible RNAi plasmid and lentivirus vectors.

In one preferred form, the shRNA is a nucleic acid having the sequence 5'-CCGCACCAAAGGCTTTGCTATTACTTCAAGAGAGTAATAGCAAAGCCTTT GGTGTTTTTTG -3' (SEQ ID NO:2). Variant nucleotide sequences include sequences that differ by one or more nucleotide substitutions, additions, or deletions, such as allelic variants. In some forms, the shRNA is from about 70% to about 95% identical to SEQ ID NO:2. In some forms, the nucleic acid is about 70%, about 80%, about 85%, about 90%, about 95% identical to SEQ ID NO:2. An isolated nucleic acid sequence or a recombinant nucleic acid sequence that is 97%, 98%, 99%, or 100% identical is also contemplated. In some forms, the shRNA reduces the AGPAT4 expression by about 5%, about 10%, about 25%, about 50%, about 75%, about 80%, about 85%, about 90%, about 95%, about 99%, or about 100%.

### 2. Antisense

AGPAT4 expression can be reduced using can be antisense molecules. Antisense molecules are designed to interact with a target nucleic acid molecule through either canonical or non-canonical base pairing. The interaction of the antisense molecule and the target molecule is designed to promote the destruction of the target molecule through, for example, RNAse H mediated RNA-DNA hybrid degradation. Alternatively, the antisense molecule is designed to interrupt a processing function that normally would take place on the target molecule, such as transcription or replication. Antisense molecules can be designed based on the sequence of the target molecule. There are numerous methods for optimization of antisense efficiency by finding the most accessible regions of the target molecule. Exemplary methods include *in vitro* selection experiments and DNA modification studies using DMS and DEPC. It is preferred that antisense molecules bind the target molecule with a dissociation constant (K_{d}) less than or equal to 10⁻⁶, 10⁻⁸, 10⁻¹⁰, or 10⁻¹².

An "antisense" nucleic acid sequence (antisense oligonucleotide) can include a nucleotide sequence that is complementary to a "sense" nucleic acid encoding a protein, e.g., complementary to the AGPAT4. Antisense nucleic acid sequences and delivery methods are well known in the art (Goodchild, Curr. Opin. Mol. Ther., 6(2):120-128 (2004); Clawson, et al., Gene Ther., 11(17):1331-1341 (2004). The antisense nucleic acid can be complementary to an entire coding strand of a target sequence, or to only a portion thereof. An antisense oligonucleotide can be, for example, about 7, 10, 15, 20, 25, 30, 35, 40, 45, 50, 55, 60, 65, 70, 75, 80, or more nucleotides in length.

An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

Other examples of useful antisense oligonucleotides (AONs/ASOs) include an alpha-anomeric nucleic acid. An alpha-anomeric nucleic acid molecule forms specific double-stranded hybrids with complementary RNA in which, contrary to the usual beta-units, the strands run parallel to each other (Gaultier et al., Nucleic Acids. Res. 15:6625-6641 (1987)). The antisense nucleic acid molecule can also comprise a 2'-o-methylribonucleotide (Inoue et al. Nucleic Acids Res. 15:6131-6148 (1987)) or a chimeric RNA-DNA analogue (Inoue et al. FEBS Lett., 215:327-330 (1987)).

### 3. Triplex forming molecules

AGPAT4 expression can be reduced using triplex forming molecules. Triplex forming functional nucleic acid molecules are molecules that can interact with either double-stranded or single-stranded nucleic acid. When triplex molecules interact with a target region, a structure called a triplex is formed in which there are three strands of DNA forming a complex dependent on both Watson-Crick and Hoogsteen base-pairing. Triplex molecules are preferred because they can bind target regions with high affinity and specificity. It is preferred that the triplex forming molecules bind the target molecule with a Kd less than 10⁻⁶, 10⁻⁸, 10⁻¹⁰, or 10⁻¹².

### 4. External guide sequences

AGPAT4 expression RNA expression can be reduced using external guide sequences. External guide sequences (EGSs) are molecules that bind a target nucleic acid molecule forming a complex, which is recognized by Rnase P, which then cleaves the target molecule. EGSs can be designed to specifically target an RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. Similarly, eukaryotic EGS/RNAse P-directed cleavage of RNA can be utilized to cleave desired targets within eukaryotic cells. Representative examples of how to make and use EGS molecules to facilitate cleavage of a variety of different target molecules are known in the art. Methods for delivering nucleic acid payloads are known in the art (reviewed in Paunovska, et al. Nat. Rev. Gen,23:265-280 (2022).

### C. Inhibitory Peptides and/or Proteins

In some forms, the AGPAT4 inhibitor is an inhibitory peptide or protein. Typically, the inhibitory peptide or protein inhibits or decreases the synthesis and/or activity of the AGPAT4 protein. In preferred forms, the inhibitory peptide or protein inhibits or decreases the synthesis and/or activity of the Human AGPAT4 Protein. Human AGPAT4 Proteins are represented by SEQ ID NO:38 and SEQ ID NO:39.
Human AGPAT4 Isoform 1 (378 amino acids; UniProt ID: QRNRZ5-1): 
MDLAGLLKSQFLCHLVFCYVFIASGLIINTIQLFTLLLWPINKQLFRKINCRLSYCI
SSQLVMLLEWWSGTECTIFTDPRAYLKYGKENAIVVLNHKFEIDFLCGWSLSER
FGLLGGSKVLAKKELAYVPIIGWMWYFTEMVFCSRKWEQDRKTVATSLQHLRD
YPEKYFFLIHCEGTRFTEKKHEISMQVARAKGLPRLKHHLLPRTKGFAITVRSLR
NVVSAVYDCTLNFRNNENPTLLGVLNGKKYHADLYVRRIPLEDIPEDDDECSA
WLHKLYQEKDAFQEEYYRTGTFPETPMVPPRRPWTLVNWLFWASLVLYPFFQF
LVSMIRSGSSLTLASFILVFFVASVGVRWMIGVTEIDKGSAYGNSDSKQKLND
(SEQ ID NO:38).
Human AGPAT4 Isoform 2 (216 amino acids; UniProt ID: QRNRZ5-2):

MDLAGLLKSQFLCHLVFCYVFIASGLIINTIQLFTLLLWPINKQLFRKINCRLSYCI
SSLSAVYDCTLNFRNNENPTLLGVLNGKKYHADLYVRRIPLEDIPEDDDECSAW
LHKLYQEKDAFQEEYYRTGTFPETPMVPPRRPWTLVNWLFWASLVLYPFFQFL
VSMIRSGSSLTLASFILVFFVASVGVRWMIGVTEIDKGSAYGNSDSKQKLND
(SEQ ID NO:39).

In some forms, the AGPAT4 inhibitory peptide or protein reduces synthesis and/or activity of a derivative of the AGPAT4 protein. In some forms, the AGPAT4 inhibitory peptide or protein inhibits or reduces the synthesis and/or activity of a derivative of SEQ ID NO:38 or SEQ ID NO:39. Exemplary derivatives of the AGPAT4 protein are represented by SEQ ID NO:40, SEQ ID NO:41, SEQ ID NO:42, and SEQ ID NO:43.
Human AGPAT4 (159 amino acids; UniProt ID: Q5TEE8): 
MDLAGLLKSQFLCHLVFCYVFIASGLIINTIQLFTLLLWPINKQLFRKINCRLSYCI
SSQLVMLLEWWSGTECTIFTDPRAYLKYGKENAIVVLNHKFEIDFLCGWSLSER
FGLLGVSQKCIPPCLTHFSGSAPPLVFLLLVIQNLQKNQQSFYLMKWS (SEQ ID
NO:40).
Human AGPAT4 (118 amino acids; UniProt ID: Q6AI25): 
MDLAGLLKSQFLCHLVFCYVFIASGLIINTIQLFTLLLWPINKQLFRKINCRLSYCI
SSRLQGPGQERAGLCPNYRLDVVLHRDGLLFAQVGAGSQDGCHQFAAPPGLPR
EVFFPDSL (SEQ ID NO:41).
Human AGPAT4 (105 amino acids; UniProt ID: H0Y5R3): 
XSAVYDCTLNFRNNENPTLLGVLNGKKYHADLYVRRIPLEDIPEDDDECSAWLH
KLYQEKGWGDTEFSKQYVSVSRPGRLALCPSEKIAEQQVEVVHGFSKQSGE
(SEQ ID NO:42).
Human AGPAT4 (133 amino acids; UniProt ID: G3XAF1):

MDLAGLLKSQFLCHLVFCYVFIASGLIINTIQLFTLLLWPINKQLFRKINCRLSYCI
SSQLVMLLEWWSGTECTIFTDPRAYLKYGKENAIVVLNHKFEIDFLCGWSLSER
FGLLGFDLLHVACMPLELGLFS (SEQ ID NO:43).

In some forms, the inhibitory peptide or protein inhibits or reduces expression and/or function of a variant AGPAT4 protein. Typically, the AGPAT4 protein variant includes a molecule or sequence that is modified from a native protein but still retains the pharmacological activity of interest. Thus, the term "protein variant" contains a molecule or sequence in which non-native residues substitute for native residues, non-native residues are added, or native residues are deleted. Any native residue may be removed because it provides structural features or biological activity that are not required for the pharmacological activity of interest of the fusion molecules of the present invention. Thus, the term "protein variant" includes a molecule or sequence that lacks one or more native protein sites or residues that affect or are involved in any number of cellular processes including but not limited to (1) intracellular signaling, (2) incompatibility with a selected host cell (3) N-terminal heterogeneity upon expression in a selected host cell, (4) glycosylation, (5) interaction with other proteins (e.g., dimerization domains), (6) binding to a receptor or other protein that does not affect the pharmacological activity of interest, or (7) antibody-dependent cellular cytotoxicity (ADCC).

### D. Pharmaceutical Formulations

The NAs and small molecules described herein can be formulated for administration to a subject in need thereof.

The NA agent is mixed or admixed with a transfection agent (or mixture thereof) and the resulting mixture is employed to transfect cells. Preferred transfection agents are cationic lipid compositions, particularly monovalent and polyvalent cationic lipid compositions, more particularly LIPOFECTIN^{®}, LIPOFECTACE^{®}, LIPOFECTAMINE^{™}, CELLFECTIN^{®}, DMRIE-C, DMRIE, DOTAP, DOSPA, and DOSPER, and dendrimer compositions, particularly G5-G10 dendrimers, including dense star dendrimers, PAMAM dendrimers, grafted dendrimers, and dendrimers known as dendrigrafts and SUPERFECT^{®}.

### 1. Parenteral Formulations

The compositions described herein (i.e., compounds reducing or inhibiting AGPAT4, and AGPAT4 inhibiting nucleic acids) can be formulated for parenteral administration.

For example, parenteral administration may include administration to a patient intravenously, intradermally, intraperitoneally, intramuscularly, subcutaneously, by injection, by infusion, etc.

Parenteral formulations can be prepared as aqueous compositions using techniques is known in the art. Typically, such compositions can be prepared as injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, or emulsomes.

The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof. The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Solutions and dispersions of the active compounds as the free acid or base or pharmacologically acceptable salts thereof can be prepared in water or another solvent or dispersing medium suitably mixed with one or more pharmaceutically acceptable excipients including, but not limited to, surfactants, dispersants, emulsifiers, pH modifying agents, viscosity modifying agents, and combination thereof.

Suitable surfactants may be anionic, cationic, amphoteric, or nonionic surface-active agents. Suitable anionic surfactants include, but are not limited to, those containing carboxylate, sulfonate, and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer^{®} 401, stearoyl monoisopropanolamide, and polyoxyethylene hydrogenated tallow amide. Examples of amphoteric surfactants include sodium N-dodecyl-.beta.-alanine, sodium N-lauryl-.beta.-iminodipropionate, myristoamphoacetate, lauryl betaine and lauryl sulfobetaine.

The formulation can contain a preservative to prevent the growth of microorganisms. Suitable preservatives include, but are not limited to, parabens, chlorobutanol, phenol, sorbic acid, and thimerosal. The formulation may also contain an antioxidant to prevent degradation of the active agent(s).

The formulation is typically buffered to a pH of 3-8 for parenteral administration upon reconstitution. Suitable buffers include, but are not limited to, phosphate buffers, acetate buffers, and citrate buffers.

Water-soluble polymers are often used in formulations for parenteral administration. Suitable water-soluble polymers include, but are not limited to, polyvinylpyrrolidone, dextran, carboxymethylcellulose, and polyethylene glycol.

Sterile injectable solutions can be prepared by incorporating the active compounds in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required other ingredients from those listed above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The powders can be prepared in such a manner that the particles are porous in nature, which can increase dissolution of the particles. Methods for making porous particles are well known in the art.

### i. Controlled Release Formulations

The parenteral formulations described herein can be formulated for controlled release including immediate release, delayed release, extended release, pulsatile release, and combinations thereof.

### a. Nano- and microparticles

For parenteral administration, the one or more compounds, and optional one or more additional active agents, can be incorporated into microparticles, nanoparticles, or combinations thereof that provide controlled release of the compounds and/or one or more additional active agents. In embodiments wherein the formulations contains two or more agents, the agents can be formulated for the same type of controlled release (e.g., delayed, extended, immediate, or pulsatile) or the agents can be independently formulated for different types of release (e.g., immediate and delayed, immediate and extended, delayed, and extended, delayed and pulsatile, etc.).

For example, the compounds and/or one or more additional active agents can be incorporated into polymeric microparticles, which provide controlled release of the drug(s). Release of the agent(s) is controlled by diffusion of the agent(s) out of the microparticles and/or degradation of the polymeric particles by hydrolysis and/or enzymatic degradation. Suitable polymers include ethylcellulose and other natural or synthetic cellulose derivatives.

Like DNA and mRNA, siRNA and miRNA can be delivered via nanocarriers. For example, Benoit et al. Biomacromolecules. 2012; 1311:3841-3849 developed a di-block co-polymer (pDMAEMA-b-p(DMAEMA-co-PAA-co-BMA)) consisting of a siRNA complexation block (pDMAEMA) and an endosomal escape block (tercopolymer of PAA, BMA, and DMAEMA) for efficient siRNA delivery.

Polymers, which are slowly soluble and form a gel in an aqueous environment, such as hydroxypropyl methylcellulose or polyethylene oxide, can also be suitable as materials for drug containing microparticles. Other polymers include, but are not limited to, polyanhydrides, poly(ester anhydrides), polyhydroxy acids, such as polylactide (PLA), polyglycolide (PGA), poly(lactide-co-glycolide) (PLGA), poly-3-hydroxybutyrate (PHB) and copolymers thereof, poly-4-hydroxybutyrate (P4HB) and copolymers thereof, polycaprolactone and copolymers thereof, and combinations thereof.

Alternatively, the agent(s) can be incorporated into microparticles prepared from materials which are insoluble in aqueous solution or slowly soluble in aqueous solution but are capable of degrading within the GI tract by means including enzymatic degradation, surfactant action of bile acids, and/or mechanical erosion. As used herein, the term "slowly soluble in water" refers to materials that are not dissolved in water within a period of 30 minutes. Preferred examples include fats, fatty substances, waxes, wax-like substances, and mixtures thereof. Suitable fats and fatty substances include fatty alcohols (such as lauryl, myristyl stearyl, cetyl or cetostearyl alcohol), fatty acids and derivatives, including but not limited to fatty acid esters, fatty acid glycerides (mono-, di- and tri-glycerides), and hydrogenated fats. Specific examples include, but are not limited to hydrogenated vegetable oil, hydrogenated cottonseed oil, hydrogenated castor oil, hydrogenated oils available under the trade name Sterotex^{®}, stearic acid, cocoa butter, and stearyl alcohol. Suitable waxes and wax-like materials include natural or synthetic waxes, hydrocarbons, and normal waxes. Specific examples of waxes include beeswax, glycowax, castor wax, carnauba wax, paraffins and candelilla wax. As used herein, a wax-like material is defined as any material, which is normally solid at room temperature and has a melting point of from about 30 to 300°C.

In some cases, it may be desirable to alter the rate of water penetration into the microparticles. To this end, rate-controlling (wicking) agents can be formulated along with the fats or waxes listed above. Examples of rate-controlling materials include certain starch derivatives (e.g., waxy maltodextrin and drum dried corn starch), cellulose derivatives (e.g., hydroxypropylmethyl-cellulose, hydroxypropylcellulose, methylcellulose, and carboxymethyl-cellulose), alginic acid, lactose, and talc. Additionally, a pharmaceutically acceptable surfactant (for example, lecithin) may be added to facilitate the degradation of such microparticles.

Proteins, which are water insoluble, such as zein, can also be used as materials for the formation of agent containing microparticles. Additionally, proteins, polysaccharides, and combinations thereof, which are water-soluble, can be formulated with agent into microparticles and subsequently cross-linked to form an insoluble network. For example, cyclodextrins can be complexed with individual agent molecules and subsequently cross-linked.

### 2. Method of making Nano- and Microparticles

Encapsulation or incorporation of agent into carrier materials to produce agent-containing microparticles can be achieved through known pharmaceutical formulation techniques. In the case of formulation in fats, waxes or wax-like materials, the carrier material is typically heated above its melting temperature and the agent is added to form a mixture comprising agent particles suspended in the carrier material, agent dissolved in the carrier material, or a mixture thereof. Microparticles can be subsequently formulated through several methods including, but not limited to, the processes of congealing, extrusion, spray chilling or aqueous dispersion. In a preferred process, wax is heated above its melting temperature, agent is added, and the molten wax-agent mixture is congealed under constant stirring as the mixture cools. Alternatively, the molten wax-agent mixture can be extruded and spheronized to form pellets or beads. These processes are known in the art. For some carrier materials it may be desirable to use a solvent evaporation technique to produce agent-containing microparticles. In this case agent and carrier material are co-dissolved in a mutual solvent and microparticles can subsequently be produced by several techniques including, but not limited to, forming an emulsion in water or other appropriate media, spray drying or by evaporating off the solvent from the bulk solution and milling the resulting material.

In some embodiments, agent in a particulate form is homogeneously dispersed in a water-insoluble or slowly water-soluble material. To minimize the size of the agent particles within the composition, the agent powder itself may be milled to generate fine particles prior to formulation. The process of jet milling, known in the pharmaceutical art, can be used for this purpose. In some embodiments, a drug in a particulate form is homogeneously dispersed in a wax or wax like substance by heating the wax or wax like substance above its melting point and adding the drug particles while stirring the mixture. In this case a pharmaceutically acceptable surfactant may be added to the mixture to facilitate the dispersion of the drug particles.

The particles can also be coated with one or more modified release coatings. Solid esters of fatty acids, which are hydrolyzed by lipases, can be spray coated onto microparticles or drug particles. Zein is an example of a naturally water-insoluble protein. It can be coated onto drug containing microparticles or drug particles by spray coating or by wet granulation techniques. In addition to naturally water-insoluble materials, some substrates of digestive enzymes can be treated with cross-linking procedures, resulting in the formation of non-soluble networks. Many methods of cross-linking proteins, initiated by both chemical and physical means, have been reported. One of the most common methods to obtain cross-linking is the use of chemical cross-linking agents. Examples of chemical cross-linking agents include aldehydes (gluteraldehyde and formaldehyde), epoxy compounds, carbodiimides, and genipin. In addition to these cross-linking agents, oxidized and native sugars have been used to cross-link gelatin. Cross-linking can also be accomplished using enzymatic means; for example, transglutaminase has been approved as a GRAS substance for cross-linking seafood products. Finally, cross-linking can be initiated by physical means such as thermal treatment, UV irradiation and gamma irradiation.

To produce a coating layer of cross-linked protein surrounding drug containing microparticles or drug particles, a water-soluble protein can be spray coated onto the microparticles and subsequently cross-linked by the one of the methods described above. Alternatively, drug-containing microparticles can be microencapsulated within protein by coacervation-phase separation (for example, by the addition of salts) and subsequently cross-linked. Some suitable proteins for this purpose include gelatin, albumin, casein, and gluten.

Polysaccharides can also be cross-linked to form a water-insoluble network. For many polysaccharides, this can be accomplished by reaction with calcium salts or multivalent cations, which cross-link the main polymer chains. Pectin, alginate, dextran, amylose, and guar gum are subject to cross-linking in the presence of multivalent cations. Complexes between oppositely charged polysaccharides can also be formed; pectin and chitosan, for example, can be complexed via electrostatic interactions.

### Enteral Formulations

Suitable oral dosage forms include tablets, capsules, solutions, suspensions, syrups, and lozenges. Tablets can be made using compression or molding techniques well known in the art. Gelatin or non-gelatin capsules can be prepared as hard or soft capsule shells, which can encapsulate liquid, solid, and semi-solid fill materials, using techniques well known in the art.

Oral mRNA delivery using capsules is described in Abramson, et al., Matter, 5(3):975-987 (2022) (incorporated herein by reference), using branched hybrid poly(β-amino ester) mRNA nanoparticles.

Formulations may be prepared using a pharmaceutically acceptable carrier. As generally used herein "carrier" includes, but is not limited to, diluents, preservatives, binders, lubricants, disintegrators, swelling agents, fillers, stabilizers, and combinations thereof.

Carrier also includes all components of the coating composition, which may include plasticizers, pigments, colorants, stabilizing agents, and glidants.

Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT^{®} (Roth Pharma, Westerstadt, Germany), zein, shellac, and polysaccharides.

Additionally, the coating material may contain conventional carriers such as plasticizers, pigments, colorants, glidants, stabilization agents, pore formers and surfactants.

"Diluents", also referred to as "fillers," are typically necessary to increase the bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar.

"Binders" are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

"Lubricants" are used to facilitate tablet manufacture. Examples of suitable lubricants include, but are not limited to, magnesium stearate, calcium stearate, stearic acid, glycerol behenate, polyethylene glycol, talc, and mineral oil.

"Disintegrants" are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginate, gums or cross-linked polymers, such as cross-linked PVP (Polyplasdone^{®} XL from GAF Chemical Corp).

"Stabilizers" are used to inhibit or retard drug decomposition reactions, which include, by way of example, oxidative reactions. Suitable stabilizers include, but are not limited to, antioxidants, butylated hydroxytoluene (BHT); ascorbic acid, its salts, and esters; Vitamin E, tocopherol and its salts; sulfites such as sodium metabisulphite; cysteine and its derivatives; citric acid; propyl gallate, and butylated hydroxyanisole (BHA).

### i. Controlled Release Enteral Formulations

Oral dosage forms, such as capsules, tablets, solutions, and suspensions, can for formulated for controlled release. For example, the one or more compounds and optional one or more additional active agents can be formulated into nanoparticles, microparticles, and combinations thereof, and encapsulated in a soft or hard gelatin or non-gelatin capsule or dispersed in a dispersing medium to form an oral suspension or syrup. The particles can be formed of the agent and a controlled release polymer or matrix. Alternatively, the agent particles can be coated with one or more controlled release coatings prior to incorporation into the finished dosage form.

In another embodiment, the one or more compounds and optional one or more additional active agents are dispersed in a matrix material, which gels or emulsifies upon contact with an aqueous medium, such as physiological fluids. In the case of gels, the matrix swells entrapping the active agents, which are released slowly over time by diffusion and/or degradation of the matrix material. Such matrices can be formulated as tablets or as fill materials for hard and soft capsules.

In still another embodiment, the one or more compounds, and optional one or more additional active agents are formulated into a sold oral dosage form, such as a tablet or capsule, and the solid dosage form is coated with one or more controlled release coatings, such as a delayed release coatings or extended-release coatings. The coating or coatings may also contain the compounds and/or additional active agents.

### a. Extended-release dosage forms

The extended-release formulations are generally prepared as diffusion or osmotic systems, which are known in the art. A diffusion system typically consists of two types of devices, a reservoir, and a matrix, and is well known and described in the art. The matrix devices are generally prepared by compressing the agent with a slowly dissolving polymer carrier into a tablet form. The three major types of materials used in the preparation of matrix devices are insoluble plastics, hydrophilic polymers, and fatty compounds. Plastic matrices include, but are not limited to, methyl acrylate-methyl methacrylate, polyvinyl chloride, and polyethylene. Hydrophilic polymers include, but are not limited to, cellulosic polymers such as methyl and ethyl cellulose, hydroxyalkylcelluloses such as hydroxypropyl-cellulose, hydroxypropylmethylcellulose, sodium carboxymethylcellulose, and Carbopol^{®} 934, polyethylene oxides and mixtures thereof. Fatty compounds include, but are not limited to, various waxes such as carnauba wax and glyceryl tristearate and wax-type substances including hydrogenated castor oil or hydrogenated vegetable oil, or mixtures thereof.

In certain preferred embodiments, the plastic material is a pharmaceutically acceptable acrylic polymer, including but not limited to, acrylic acid and methacrylic acid copolymers, methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer poly(methyl methacrylate), poly(methacrylic acid)(anhydride), polymethacrylate, polyacrylamide, poly(methacrylic acid anhydride), and glycidyl methacrylate copolymers.

In certain preferred embodiments, the acrylic polymer is comprised of one or more ammonio methacrylate copolymers. Ammonio methacrylate copolymers are well known in the art and are described in NF XVII as fully polymerized copolymers of acrylic and methacrylic acid esters with a low content of quaternary ammonium groups.

In one preferred embodiment, the acrylic polymer is an acrylic resin lacquer such as that which is commercially available from Rohm Pharma under the tradename EUDRAGIT t^{®}. In further preferred embodiments, the acrylic polymer comprises a mixture of two acrylic resin lacquers commercially available from Rohm Pharma under the tradenames EUDRAGIT^{®} RL30D and EUDRAGIT ^{®} RS30D, respectively. EUDRAGIT^{®} RL30D and EUDRAGIT ^{®} RS30D are copolymers of acrylic and methacrylic esters with a low content of quaternary ammonium groups, the molar ratio of ammonium groups to the remaining neutral (meth)acrylic esters being 1:20 in EUDRAGIT ^{®} RL30D and 1:40 in EUDRAGIT^{®} RS30D. The mean molecular weight is about 150,000. EUDRAGIT ^{®} S-100 and EUDRAGIT ^{®} L-100 are also preferred. The code designations RL (high permeability) and RS (low permeability) refer to the permeability properties of these agents. EUDRAGIT ^{®} RL/RS mixtures are insoluble in water and in digestive fluids. However, multi-particulate systems formed to include the same are swellable and permeable in aqueous solutions and digestive fluids.

The polymers described above such as EUDRAGIT ^{®} RL/RS may be mixed in any desired ratio in order to ultimately obtain a sustained-release formulation having a desirable dissolution profile. Desirable sustained release multi-particulate systems may be obtained, for instance, from 100% EUDRAGIT^{®} RL, 50% EUDRAGIT^{®} RL and 50% EUDRAGIT t^{®} RS, and 10% EUDRAGIT^{®} RL and 90% EUDRAGIT^{®} RS. One skilled in the art will recognize that other acrylic polymers may also be used, such as, for example, EUDRAGIT^{®} L.

Alternatively, extended-release formulations can be prepared using osmotic systems or by applying a semi-permeable coating to the dosage form. In the latter case, the desired agent release profile can be achieved by combining low permeable and high permeable coating materials in suitable proportion.

The devices with different agent release mechanisms described above can be combined in a final dosage form comprising single or multiple units. Examples of multiple units include, but are not limited to, multilayer tablets and capsules containing tablets, beads, or granules. An immediate release portion can be added to the extended-release system by means of either applying an immediate release layer on top of the extended-release core using a coating or compression process or in a multiple unit system such as a capsule containing extended and immediate release beads.

Extended-release tablets containing hydrophilic polymers are prepared by techniques commonly known in the art such as direct compression, wet granulation, or dry granulation. Their formulations usually incorporate polymers, diluents, binders, and lubricants as well as the active pharmaceutical ingredient. The usual diluents include inert powdered substances such as starches, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin, and sugars such as lactose, fructose, and glucose. Natural and synthetic gums, including acacia, alginates, methylcellulose, and polyvinylpyrrolidone can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid, and hydrogenated vegetable oils.

Extended-release tablets containing wax materials are generally prepared using methods known in the art such as a direct blend method, a congealing method, and an aqueous dispersion method. In the congealing method, the agent is mixed with a wax material and either spray- congealed or congealed and screened and processed.

### b. Delayed release dosage forms

Delayed release formulations can be created by coating a solid dosage form with a polymer film, which is insoluble in the acidic environment of the stomach, and soluble in the neutral environment of the small intestine.

The delayed release dosage units can be prepared, for example, by coating an agent or an agent-containing composition with a selected coating material. The agent-containing composition may be, e.g., a tablet for incorporation into a capsule, a tablet for use as an inner core in a "coated core" dosage form, or a plurality of agent-containing beads, particles, or granules, for incorporation into either a tablet or capsule. Preferred coating materials include bioerodible, gradually hydrolyzable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename Eudragit^{®} (Rohm Pharma; Westerstadt, Germany), including EUDRAGIT^{®} L30D-55 and L100-55 (soluble at pH 5.5 and above), EUDRAGIT^{®} L-100 (soluble at pH 6.0 and above), EUDRAGIT^{®} S (soluble at pH 7.0 and above, as a result of a higher degree of esterification), and EUDRAGITS^{®} NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different coating materials may also be used. Multi-layer coatings using different polymers may also be applied.

The preferred coating weights for particular coating materials may be readily determined by those skilled in the art by evaluating individual release profiles for tablets, beads and granules prepared with different quantities of various coating materials. It is the combination of materials, method and form of application that produce the desired release characteristics, which one can determine only from the clinical studies.

The coating composition may include conventional additives, such as plasticizers, pigments, colorants, stabilizing agents, glidants, etc. A plasticizer is normally present to reduce the fragility of the coating and will generally represent about 10 wt. % to 50 wt. % relative to the dry weight of the polymer. Examples of typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil and acetylated monoglycerides. A stabilizing agent is preferably used to stabilize particles in the dispersion. Typical stabilizing agents are nonionic emulsifiers such as sorbitan esters, polysorbates and polyvinylpyrrolidone. Glidants are recommended to reduce sticking effects during film formation and drying and will generally represent approximately 25 wt. % to 100 wt. % of the polymer weight in the coating solution. One effective glidant is talc. Other glidants such as magnesium stearate and glycerol monostearates may also be used. Pigments such as titanium dioxide may also be used. Small quantities of an anti-foaming agent, such as a silicone (e.g., simethicone), may also be added to the coating composition.

### III. METHODS OF DIAGNOSIS AND TREATMENT

It has been discovered that patients with liver cancer generally have an increased level of AGPAT4 in the liver tissue and/or in the plasma sample. Therefore, methods for assisting in the detection and diagnosis of cancers exhibiting an increased level of AGPAT4 such as liver cancer e.g. HCC, in a subject are provided.

### A. Methods for Identifying Subjects with Cancer

Methods for characterizing tumors and/or for characterizing the tumor microenvironment are provided. In particular forms, the methods characterize tumors so as to assess the extent to which the tumor cells and/or tumor infiltrating cells or tumor associated cells express genes associated with sensitivity to one or more AGPAT4 inhibitors alone, or in combination with one or more inhibitors of a kinase, preferably a receptor tyrosine kinase. For example, tumor cells and/or tumor infiltrating cells or tumor associated cells that are sensitive to the effects of an AGPAT4 inhibitor alone or in combination with one or more inhibitors of a receptor tyrosine kinase can express genes involved in the PI3K/AKT/mTOR signaling pathway prior to treatment, including phosphorylated *mTOR, S6K, and S6,* and phosphatidic acid (PA; an upstream regulator of mTOR signaling), prior to treatment. In some forms, the methods diagnose and treat cancer and other diseases. Therefore, in some forms, the methods characterize a cell of a tumor. In particular forms, the methods include determining whether a cell of the tumor expresses one or more of the components of genes and signaling pathways involved in cancer stemness (including *Agpat4* and *Sox9*)*.* Additionally, CD133 and AFP are downregulated when AGPAT4 is inhibited (as shown by WB, flow cytometry and multiplex IHC staining (FIG. 3).

Methods for assessing the amenability of the subject to a proposed anti-cancer therapy are also provided. In some forms, the methods include characterizing cells of a tumor of the subject by determining whether the cells of the tumor express genes that are associated with sensitivity to the effects of the AGPAT4 inhibitor or to more than additive effects of an AGPAT4 inhibitor in combination with one or more inhibitors of a receptor tyrosine kinase. For example, in some embodiments, subjects having cancer cells that express genes involved in the PI3K/AKT/mTOR signaling pathway are selected for treatment with an AGPAT4 inhibitor or combination therapy with an AGPAT4 inhibitor and one or more inhibitors of a receptor tyrosine kinase. Methods for assessing the efficacy of an anti-cancer therapy provided to a subject are also disclosed. In some forms the methods include characterizing cells of a tumor of the patient during the course of the therapy or after the completion thereof, wherein said characterization can include determining whether the cells of the tumor express genes involved in the PI3K/AKT/mTOR signaling pathway, or show increased expression of phosphorylated *mTOR, S6K, and S6,* and phosphatidic acid in response to the treatment.

Methods for selecting patients for anti-cancer therapy based on characterization of the tumor or tumor microenvironment are also provided. In some embodiments, cancer patient tumor samples are characterized prior to and following treatment with specific chemotherapeutic and/or biologic therapies, and/or other therapeutic interventions (*e.g.* radiation, cryoablation, surgical resection of the tumor, *etc*.), in order to determine if the expression patterns of one or more of the components of genes and signaling pathways involved in cancer stemness e.g., *Agpat4* and genes involved in the PI3K/AKT/mTOR signaling pathway including *mTOR, S6K, and S6,* within the tumor microenvironment have changed.

The disclosed methods can comprise the steps of a) measuring the level of AGPAT4 in a sample of a subject; b) comparing the amount of AGPAT4 in the sample to a control; c) determining whether the sample has an increased level of AGPAT4 compared to the control to provide an assay output, and d) identifying the subject as having liver cancer if the level of AGPAT4 is increased compared to the control.

In one form, the methods include the step of obtaining a subject sample. For example, this step could be performed by someone other than the person or machine measuring the levels of the biomarkers. Obtaining the sample can include obtaining the sample directly from the subject or obtaining the sample from a storage area.

In some forms, disclosed are methods that include the step of obtaining the assay output, and prescribing an anti-cancer drug for the subject in a prescription if the amount of the AGPAT4 is greater than the control. The levels of AGPAT4 in the disclosed methods can be at least 5%, 10%, 15%, 20%, 25%, 30%, 40%, 50%, 60%, 70%, 80%, 90%, 100% or greater than the control levels. In some forms, the levels of AGPAT4 can be at least 1.1x, 1.5x, 2x, 2.5x, 3x, 4x, 5x, 6x, 7x, 8x, 9x, 10x, 20x, 30x, 40x, 50x, 60x, 70x, 80x, 90x, 100x or greater than the control levels. In one form, the level of AGPAT4 can be at least 10% greater than the control levels.

The sample used in the disclosed methods can be a blood sample or plasma sample.

In some forms, the methods include performing an assay. The assay can be done to measure the levels of AGPAT4. Exemplary assays include an enzyme-linked immunosorbent assay (ELISA), radioimmunoassay (RIA), western blot, and dot blot.

The disclosed methods can include a control which can be a standard. The control can be a sample from a healthy subject or a subject without liver cancer.

In some forms, the methods further comprise the step of transmitting the assay output to a recipient.

Further disclosed are methods where increased levels of AGPAT4 can provide at least 50%, 60%, 70%, 80%, 90%, 95%, 98%, 99% or 100% confidence or accuracy of the diagnosis or identification of a subject with liver cancer.

Also disclosed are methods of diagnosing cancer in a subject which includes measuring the levels of AGPAT4 in a sample from the subject, where an increased level of AGPAT4 in the subject indicates cancer in the subject and producing a diagnosis result. In preferred forms, the cancer is liver cancer e.g. hepatocellular carcinoma.

As shown in the Examples, AGPAT4 is linked with chemotherapy resistance and poor prognosis. Thus, in some forms, the methods stratify HCC patients into a high survival group and a low survival group based on the levels of AGPAT4 in samples from the subjects. In other forms, the methods stratify cancer patients into responders and non-responders to chemotherapy. In some forms, the patients in the high survival group are responders to conventional chemotherapy. In other forms, the patients in the low survival group are non-responders to conventional chemotherapy.

### B. Methods for Treating Cancer

Methods of treating one or more symptoms of a cancer in a subject are provided. In some forms, the methods include administering to a subject with cancer an effective amount of an AGPAT4 inhibitor disclosed herein in a therapeutically effective amount to reduce, inhibit the activity and/or quantity of AGPAT4 in the subject. In some forms, AGPAT4 inhibitors are a small molecule inhibitors such as CL26, a derivative thereof or a pharmaceutically acceptable salt thereof. In some forms the AGPAT4 inhibitor is an inhibitory nucleic acid. A preferred inhibitory nucleic acid is SEQ ID NO:2 or a variant thereof, having more than 90%, 91%, 92%, 93%, 94%, 95%, 96%, 97%, 98%, or 99% sequence identify to SEQ ID NO:2.

In some forms, the methods include contacting one or more cancer cells expressing AGPAT4 with an effective amount of a therapeutic agent, to decrease or inhibit the proliferation and/or viability of the cancer cells compared to untreated control cancer cells. The therapeutic agent(s) can be administered locally or systemically to the subject, or coated or incorporated onto, or into a device.

In some forms, the cancer may have developed a resistance to the previously administered chemotherapeutic agent(s). Therefore, in some forms, the subject population being treatment is one in which the cancer being treated is resistant or insensitive to one or more conventional chemotherapeutic agents prior to treating with a therapeutic agent that reduces, inhibits the activity and/or quantity of AGPAT4.

An effective amount of each of the agents can be administered as a single unit dosage (*e.g*., as dosage unit), or sub-therapeutic doses that are administered over a finite time interval. Such unit doses may be administered on a daily basis for a finite time period, such as up to 3 days, or up to 5 days, or up to 7 days, or up to 10 days, or up to 15 days or up to 20 days or up to 25 days, are all specifically contemplated.

### 1. Hepatocellular Carcinoma

In some forms, the methods are effective for treatment of hepatocellular carcinoma (HCC). HCC is the most common primary liver malignancy and is a leading cause of cancer-related death worldwide. It originates in the hepatocytes, which are the main functional cells of the liver.

In its early stages, HCC may not cause noticeable symptoms. As it progresses, common symptoms may include abdominal pain or discomfort, jaundice (yellowing of the skin and eyes), unexplained weight loss, fatigue, nausea and vomiting and a feeling of fullness in the abdomen.

"Locally advanced" hepatocellular carcinoma (HCC) is a term used to describe tumor that has progressed beyond its initial site of origin but has not yet spread extensively to distant parts of the body. This term encompasses a heterogeneous morphology with no regard to stage of prognosis of the disease. In other words, locally advanced cancer has grown into nearby tissues, organs, or structures, but it has not metastasized, which means it has not spread to distant organs or distant lymph nodes.

The exact definition of "locally advanced" can also depend on the stage of cancer, but it generally means that the tumor has reached a stage where it is often challenging to treat with localized therapies like surgery or radiation therapy alone. Treatment for locally advanced HCC can combine chemotherapy, embolization, chemoembolization (such as TACE), radiation therapy (including SBRT), immunotherapy (such as immune checkpoint inhibitors), or any combination of these in any sequence or manner. "Unresectable" HCC can be defined as a liver tumor or cancerous growth that cannot be completely removed or excised given the extent of disease, including patients that are not suitable for surgery for location of tumor(s) in the liver, or patients who were older than 75 years, or those who refused surgical therapies. This also means that if surgically removed, it can cause excessive damage to vital organs, tissues, or structures.

### 2. Other Cancers

In some forms, methods of administering an AGPAT4 inhibitor, alone or in combination with a kinase inhibitor are effective for treatment of multiple types of cancer. For example, the Agpat4/LPA axis in colorectal cancer cells regulates antitumor responses through p38/p65 signaling in macrophages (35).

Further, AGPAT4 is reported among breast cancer-expressed genes involved in endo-/exocytosis (EEC) (Wittkowski et al., PLoS ONE, 13:e0199012 (2018). Thus, in some forms, the AGPAT4 inhibitors can be used to treat colorectal cancer and/or breast cancer. AGPAT4 has also been previously reported to be frequently overexpressed in other tumor types including colorectal cancer (Zhang D et al. Signal Transduct Target Ther 2020). Analysis of TCGA data using the GTEx Portal (https://gtexportal.org/home/) also found AGPAT4 to be significantly overexpressed in head and neck squamous cell carcinoma as well as pancreatic adenocarcinoma in addition to HCC (p<0.01). LGG (Brain Lower Grade Glioma), GBM (Glioblastoma multiforme), SARC (Sarcoma), KIRP (Kidney renal papillary cell carcinoma), and UCEC (Uterine Corpus Endometrial Carcinoma) have also demonstrated high AGPAT4 expression of AGPAT4 in their tumors.

It has been established that an AGPAT4 inhibitor, or a derivative, analog or prodrug, or a pharmacologically active salt thereof, alone or in combination with one or more inhibitors of receptor tyrosine kinase can provide greater than additive killing of cancer cells in cancers that are associated with increased activities and/or gene expression of one or more components in the signaling pathways involved in cancer stemness, e.g., *Agpat4,* specifically genes involved in the PI3K/Akt/mTOR signaling pathway including LPA. Therefore, multiple non-hormonal cancers can be treated using the compositions and methods described herein.

The combination is particularly effective in treating chemotherapy-resistant cancers e.g., sorafenib resistant cancers.

### 3. Dosage and Effective Amounts

Dosage and dosing regimens are dependent on the severity of the disorder and/or methods of administration, and can be determined by those skilled in the art. A therapeutically effective amount of compositions targeting AGPAT4 or pharmaceutical formulation thereof used in the treatment of liver cancer is typically sufficient to reduce or alleviate one or more symptoms associated with the disease or disorder.

In some forms, the compositions targeting AGPAT4 are administered in an amount effective to reduce and/or inhibit amount and/or activities of AGPAT4. In preferred forms, the compositions are effective to reduce or inhibit proliferation, migration, invasion, motility, and/or metastatic abilities of the cancer cells. In further preferred forms, the compositions are effective to reduce or inhibit tumor growth, tumor burden, and/or increase survival of the subject.

For example, following the administration step or all of the administration steps (if the administration occurs more than one time), an effective amount of the AGPAT4 inhibitor is administered to the subject, such that the tumor volume and the tumor weight in the subject is at least 10%, at least 20%, at least 30%, at least 40%, or at least 50% smaller/less than a control that is administered with the pharmaceutically acceptable excipient(s) only.

For example, following the administration step or all of the administration steps (if the administration occurs more than one time), an effective amount of the AGPAT4 inhibitor is administered to the subject, such that the tumor volume in the subject does not change compared to before administration of the pharmaceutical composition or the increase of tumor volume compared to before administration of the pharmaceutical composition in the subject is less than the increase of tumor volume in a control that is administered with the pharmaceutically acceptable excipient(s) only; and the tumor weight in the subject is at least 10%, at least 20%, at least 30%, at least 40%, or at least 50% less than the control.

The cancer treatment effect as described above can occur within 3 days, within 5 days, within 7 days, within 10 days, within 2 weeks, within one month, within three months, or within six months following the administration step or all of the administration steps. Optionally, the cancer treatment effect occurs without any observable toxicity to the subject, as indicated by body vital measurements (e.g., body weight), standard hematology markers, and/or blood biochemical parameters compared to the control.

In some forms, the disclosed therapeutic agents are administered in an amount effective to reduce or inhibit the number of cancer stemness markers in HCC, or the number of cancer cells expressing one or more of PROM1 (CD133) and AFP.

Epithelial-mesenchymal transition (EMT) is a reversible cellular process, characterized by changes in gene expression and activation of proteins, favoring the trans-differentiation of the epithelial phenotype to a mesenchymal phenotype. This process increases cell migration and invasion of tumor cells, progression of the cell cycle, and resistance to apoptosis and chemotherapy, all of which support tumor progression. Thus, in some forms, the disclosed therapeutic agents are administered in an amount effective to reduce or inhibit EMT. In other forms, the compositions are effective to reduce and/or inhibit mTOR, S6K, and S6 phosphorylation. In further forms, the compositions are effective to increase the sensitivity of cancer cells to chemotherapy.

Preferably, the compositions do not target or otherwise modulate the activity or quantity of healthy cells not within or associated with the diseased or target tissues or do so at a reduced level compared to target cells. In this way, by-products and other side effects associated with the compositions are reduced.

The actual effective amounts can vary according to factors including the specific agent administered, the particular composition formulated, the mode of administration, and the age, weight, condition of the subject being treated, as well as the route of administration and the disease or disorder.

For example, following the administration step or all of the administration steps, the effective amount of the AGPAT4 inhibitor administered to the subject is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 ng, from about 1 µg to about 10 µg, from about 2 µg to about 20 µg, or from about 5 µg to about 50 µg, per g of the subject.

In general, the timing and frequency of administration will be adjusted to balance the efficacy of a given treatment schedule with the side-effects of the given delivery system. Exemplary dosing frequencies include continuous infusion, single and multiple administrations such as daily, weekly, monthly, or yearly dosing.

The composition or pharmaceutical formulation thereof can be administered daily, biweekly, weekly, every two weeks, monthly, or less frequently in an amount to provide a therapeutically effective increase in the blood level of the therapeutic agent. Alternatively, the compositions can be formulated for controlled release, wherein the composition is administered as a single dose that is repeated on a regimen of once a week, or less frequently.

Dosage can vary, and can be administered in one or more doses daily, once daily, twice weekly, once a week, once every two weeks, once monthly, or less frequently. Guidance can be found in the literature for appropriate dosages for given classes of pharmaceutical products. Optimal dosing schedules can be calculated from measurements of drug accumulation in the body of the subject or patient. Persons of ordinary skill can easily determine optimum dosages, dosing methodologies and repetition rates. Optimum dosages can vary depending on the relative potency of individual pharmaceutical compositions and can generally be estimated based on EC₅₀s found to be effective in *in vitro* and *in vivo* animal models.

In some forms, the compositions are administered to a subject for between 1 to 20 years, *e.g.,* 1 year, 2 years, 3 years, 4 years, 5 years, 6 years, 7 years, 8 years, 9 years, 10 years, 11 years, 12 years, 13 years, 14 years, 15 years, 16 years, 17 years, 18 years, 19 years, or 20 years. Optionally, the compositions are administered for 10 years. In one form, the effects of treatment last for at least 1 year.

In some forms, the regimen includes one or more cycles of a round of therapy followed by a drug holiday (*e.g.,* no drug). The drug holiday can be 1, 2, 3, 4, 5, 6, or 7 days, or 1, 2, 3, 4 weeks, or 1, 2, 3, 4, 5, or 6 months.

In some forms, the amount of compositions administered to a subject change over time following an initial dose. Therefore, in some forms, the amount of compositions administered to a subject change over time following an initial dose.

### 4. Combination Therapy

Early-stage HCC can be treated curatively by local ablation, surgical resection, or liver transplantation. Treatment selection depends on tumor characteristics, the severity of underlying liver dysfunction, age, other medical comorbidities, and available medical resources and local expertise. Catheter-based locoregional treatment is used in patients with intermediate-stage cancer. Treatment with one or more active agents that reduce or inhibit the expression and/or activity of AGPAT4 can increase the sensitivity of cancer cells to conventional chemotherapy. Thus, in some forms, the therapy includes administering one or more active agents such as small molecules that reduce or inhibit AGPAT4 expression and/or activity, or inhibitory nucleic acid that knockdown AGPAT4, in combination with one or more conventional chemotherapeutic agents such as sorafenib, cisplatin, and 5-flurouracil.

In some forms, the combination therapies include one or more kinase inhibitors. Kinase and immune checkpoint inhibitors have been shown to be effective treatment options in patients with advanced-stage HCC. Multiple cellular kinases are involved in the development and progression of the HCC by promoting angiogenesis, cellular differentiation, proliferation, and survival.

Kinases are a class of enzymes that promote phosphorylation. Protein enzymes are chains of amino acids and when a phosphoryl group, *i.e.,* PO32-, is covalently attached to one of the amino acids, it changes the three-dimensional configuration and function of the enzyme. Proteins are constantly being phosphorylated and dephosphorylated in living cells. Apoptosis, proliferation, and differentiation are all affected by phosphorylation. When these kinase enzymes go "wrong", normal cellular function can go awry, and kinase deregulation can contribute to the growth of cancer cells. Drugs given to stop kinases can slow the proliferation of malignant cells and angiogenesis (growth of blood vessels). There are many kinases (538 in the human body) and many kinase inhibitor compounds have been identified. Some of these have proved to be useful in cancer treatment.

In some embodiments, the kinase inhibitor is a tyrosine kinase inhibitor (TKI). Most kinase inhibitors work on tyrosine kinases. Tyrosine kinases are important cellular signaling proteins that have a variety of biological activities, including cell proliferation and migration. Multiple kinases are involved in angiogenesis, including receptor tyrosine kinases such as the vascular endothelial growth factor receptor (VEGFR). Tyrosine kinase inhibitors (TKIs) are a class of chemotherapy medications that inhibit, or block, one or more of the enzyme tyrosine kinases. Cell membrane receptors are molecular structures that send and receive signals from the environment. Some of the receptors are enzymes and catalyze biochemical reactions.

In some embodiments, the kinase inhibitor is an inhibitor of a Receptor Tyrosine Kinase (RTK). RTKs span the cell membrane with an intracellular (internal) and extracellular (external) portion. The intracellular portion removes a phosphate group, a process called de-phosphorylation, from the coenzyme messenger ATP. The extracellular portion has sites to which signal sending proteins and hormones can bind. Many of these signaling binders are growth factors.

Tyrosine kinase inhibitors treat cancer by correcting this deregulation. Imatinib, for example, blocks a kinase receptor from binding to ATP, preventing the phosphorylation that would benefit the cancerous cell and promote cell division. For example, Gefitinib inhibits EGFRs, preventing that signal from being stuck "on" and creating uncontrolled proliferation. Over 30 TKI medications, including imatinib and gefitinib, have been approved by the Food and Drug Administration for use in humans. One TKI, Toceranib (Palladia), was approved for the treatment of cancer in dogs. The human medications may inhibit one or more tyrosine kinases. Erlotinib (Tarceva), like Gefitinib, inhibits EGFR and a subclass called Human EGFR type 2. EGFR is not the only growth factor targeted. Sunitinib (Sutent) is multi-targeted, inhibiting PDGFR and VEGF. Lapatinib (Tykerb) is a dual inhibitor, inhibiting EGFR and HER2.

Other tyrosine kinase inhibitors are more specialized. Sorafenib (Nexavar) targets a complex pathway that would lead to a kinase signaling cascade. Nilotinib (Tasinga) inhibits the fusion protein bcr-abl and is typically prescribed when a patient has shown resistance to imatinib.

In some embodiments, the combination therapy includes one or more inhibitors targeting one or more of FLT4, FGFR, EGFR, ERBB2, VEGFRs, Kit, PDGFRs, ABL, SRC, mTOR, and combinations thereof. In some embodiments, the combination therapy includes one or more of Crizotinib, Ceritinib, Alectinib, Brigatinib, Bosutinib, Dasatinib, Imatinib, Nilotinib, Vemurafenib, Dabrafenib, Ibrutinib, Palbociclib, Sorafenib, Ribociclib, Cabozantinib, Gefitinib, Erlotinib, Lapatinib, Vandetanib, Afatinib, Osimertinib, Ruxolitinib, Tofacitinib, Trametinib, Axitinib, Toceranib, Lenvatinib, Nintedanib, Pazopanib, Regorafenib, Sunitinib, Dacomitinib, and Ponatinib.

In some embodiments, the combination therapy includes a kinase receptor antagonist that is a pan-kinase receptor inhibitor. In other embodiments, the combination therapy includes a kinase receptor antagonist that is a specific inhibitor of one or more kinases. In preferred embodiments, the combination therapy includes one or more inhibitors targeting mammalian target of rapamycin complex 1 (mTORC1), mTORC2, p70 ribosomal S6 kinase 1 (S6K1), and FK506-binding protein (FKBP12). The combination therapy can also include one or mor inhibitors of PROM1 (CD133) and AFP, in addition to mTOR signaling pathway.

### i. Sorafenib

In some forms, the combination therapy includes the kinase inhibitor sorafenib. Sorafenib is an oral bi-aryl urea, which inhibits multiple cell surface and downstream kinases involved in tumor progression. Two phase III randomized placebo-controlled trials, the SHARP trial, conducted mainly in America and Europe (Llovet JM, *et al.*

(2008) N Engl J Med. 359(4): pages 378-390), and a similar trial conducted in Asia (Cheng AL, et al. (2009) Lancet Oncol. 10(1): pages 25-34) reported improved overall survival with sorafenib. In the SHARP trial, the median overall survival was 10.7 months with sorafenib and 7.9 months with placebo. In the Asian study, the median overall survival was 6.5 months with sorafenib and 4.2 months with placebo. Sorafenib was generally well tolerated; toxicities were mild to moderate in severity, predominantly including diarrhea, fatigue, and hand-foot skin reaction.

Accordingly, in some forms, the combination therapy includes an effective amount of sorafenib and an effective amount of one or more of AGPAT4 inhibitors for preventing, treating, or alleviating one or more symptoms of cancer, particularly liver cancer.

The chemical structure of sorafenib is shown below.

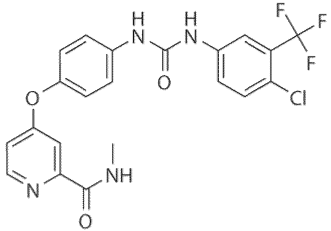

### ii. Lenvatinib

In some forms, the combination therapy includes the kinase inhibitor lenvatinib. Lenvatinib is a multireceptor tyrosine kinase inhibitor that inhibits the kinase activities of vascular endothelial growth factor (VEGF) receptors VEGFR1 (FLT1), VEGFR2 (KDR), and VEGFR3 (FLT4). Lenvatinib also inhibits other receptor tyrosine kinases that have been implicated in pathogenic angiogenesis, tumor growth, and cancer progression in addition to their normal cellular functions, including the fibroblast growth factor receptors FGFR1, 2, 3, and 4; and the platelet-derived growth factor receptor alpha, KIT, and RET.

In some embodiments, combination therapy includes an effective amount of lenvatinib and an effective amount of one or more of AGPAT4 inhibitors for preventing, treating, or alleviating one or more symptoms of cancer, particularly liver cancer.

The chemical structure of lenvatinib is shown below.

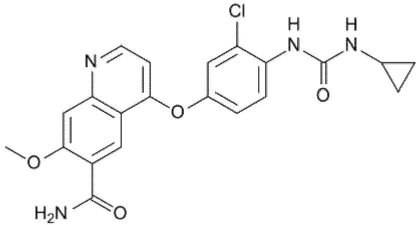

### iii. Linifanib

In some forms, the kinase inhibitor is linifanib. Linifanib is a multi-kinase inhibitor targeting VEGFR and PDGFR along with other kinases. It was found to be effective in the treatment of the HCC with an acceptable safety profile in a single arm phase II clinical trial (Toh HC, et al. (2013), Cancer.119: pages 380-387).

### iv. Tivantinib

In some forms, the kinase inhibitor is tivantinib. Tivantinib is an oral MET receptor tyrosine kinase inhibitor. When added to sorafenib, it had synergistic effect against HCC as noted in a phase I clinical trial (Martell RE, et al. (2012) Proc Am Soc Clin Oncol. 30(No. 15 Suppl): Abstract 4117). In a randomized, placebo-controlled, double-blind, phase II trial, tivantinib was used as a second line agent for the treatment of HCC in previously unresectable HCC who progressed or could not tolerate the first line systemic therapy (Santoro A, et al. (2013) Lancet Oncol. 14(1): pages 55-63).

### v. Everolimus

In some forms, the kinase inhibitor is everolimus. Everolimus is an inhibitor of mTOR. A phase I/II single arm trial using everolimus in advanced HCC patients (unresectable) with and without prior systemic therapy for HCC showed that the median progression free survival of 28 patients was 3.8 months (95%CI: 2.1-4.6) and overall survival was 8.4 months (95%CI: 3.9-21.1) (Zhu AX, et al. (2011) Cancer 117(22): pages 5094-5102).

In some forms, the compositions and methods are used prior to or in conjunction with surgical removal of tumors, for example, in preventing primary tumor metastasis. In some forms, the compositions and methods are used to enhance body's own anti-tumor immune functions.

### C. Methods for Monitoring and Evaluating Treatment Efficacy

The methods include steps of monitoring the level of AGPAT4 in a subject. In some forms, the methods include the steps of treating the subject for a cancer marked by increased AGPAT4 expression and/or activity such as liver cancer, and then performing any of the disclosed methods to monitor progress of treatment, and/or to detect the level of AGPAT4 following the treatment.

In some forms, the method further includes discontinuing treatment of the subject if the level of AGPAT4 is reduced, for example by 10%, 20%, 30%, 40%, 50%, 60%, 70%, 80%, 90%, or more than 90% compared to the level prior to treatment.

### D. Methods for Drug Screening

There is a severe lack of effective treatments which target AGPAT4 expression and/or activity for treating cancers such as liver cancer e.g. HCC. The systems and methods are useful to investigate the activity or applicability of one or more test compounds to treat or alleviate or prevent one or more symptoms of cancers marked by increased AGPAT4 expression and/or activity such as liver cancer e.g., HCC. Therefore, in some forms, the methods include one or more steps for assessing the quantity and/or activities of AGPAT4 in the presence of one or more active agents, where the quantity and/or activities of AGPAT4 in the presence of the active agent is assessed by comparison with the quantity and/or activities of AGPAT4 in the absence of the active agent. In an exemplary form, an active agent is selected if it is effective to reduce the quantity and/or activities of AGPAT4.

It is to be understood that the disclosed method and compositions are not limited to specific synthetic methods, specific analytical techniques, or to particular reagents unless otherwise specified, and, as such, can vary. It is also to be understood that the terminology used herein is for the purpose of describing particular embodiments only and is not intended to be limiting.

### IV. Kits

Medical kits are also disclosed. The medical kits can include, for example, a dosage supply of one or more AGPAT4 inhibitors. In some forms, the medical kit can also include one or more kinase inhibitors, separately or together in the same admixture for combination therapy. The active agents can be supplied alone (*e.g*., lyophilized), or in a pharmaceutical composition. The active agents can be in a unit dosage, or in a stock that should be diluted prior to administration. In some embodiments, the kit includes a supply of pharmaceutically acceptable carriers. The kit can also include devices for administration of the active agents or compositions, for example, syringes. The kits can include printed instructions for administering the AGPAT4 inhibitor in a method for use as described in Section IV below.

### EXAMPLES

### Materials and Methods

### Patients and tissue samples

Formalin-fixed paraffin-embedded primary human HCC and adjacent non-tumor liver tissue samples were obtained from HCC patients undergoing hepatectomy at the Sun Yat-Sen University Cancer Centre (Guangzhou, China) or The Second Affiliated Hospital of Air Force Military Medical University (Shanxi, China) with informed consent obtained from all patients and protocols approved by the Institutional Review Board of the University Cancer Centre or the Fourth Military Medical University. Samples were collected from patients who had not received any local or systemic treatment prior to the operation.

### Reagents

Sorafenib was purchased from LC Laboratories. Rapamycin was purchased from Sigma-Aldrich. 18:1 phosphatidic acid (PA) was purchased from Avanti Polar Lipids. LYS6K (LY2584702 Tosylate) was purchased from TargetMol. Recombinant Human AGPAT4 protein was purchased from Cusabio.

### Animal experiments

All animal study protocols were approved by and performed in accordance with the Committee of the Use of Live Animals in Teaching and Research (CULATR) at The University of Hong Kong and the Animals (Control of Experiments) Ordinance of Hong Kong. All mice (Mus musculus) were housed in the Association for Assessment and Accreditation of Laboratory Animal Care International (AAALAC)-credited facility in 12 hours light/dark cycle (07:00-19:00 light, 19:00-07:00 dark), with controlled room temperature (23 ± 2°C) and humidity (30-70%) in groups according to stocking density as recommended in the guidelines. According to the CULTAR guidelines, the diameter of a single tumor should not exceed 15 mm in mice for therapeutic studies, the decrease in body weight should not exceed 20% from baseline, and the mouse should not exhibit signs of being moribund, unconscious, or comatose, nor display a prolonged or irreversible inability to eat or drink. To ensure compliance with these guidelines, the tumor volume and weight were monitored every other day during the study. At the endpoint, the animals were euthanized by cervical dislocation under anaesthesia, as approved by CULTAR. Male mice were exclusively utilized in all animal experiments in this study, as males exhibit a significantly higher incidence rate of HCC than females.

### TCGA (The Cancer Genome Atlas) and Gene Expression Omnibus (GEO) data

Gene expression profiling studies involving normal liver or non-tumor liver and HCC tissue samples were analysed for the expression of AGPAT4 and/or SOX9 transcripts available in Liver Hepatocellular Carcinoma (LIHC) of TCGA Research Network and GEO (GSE25097, GSE40367, and GSE164760) of the National Centre for Biotechnology Information (NCBI). HCC tissue samples with AGPAT4 transcripts available under TCGA-LIHC were segregated into two groups: top and bottom 50%, using the mean of all AGPAT4 expression levels as the cut-off point. DAVID analysis was used to predict pathway enrichment. Similar analyses were performed other cancer types.

### Statistics

Unless otherwise specified, all statistical analyses were performed using GraphPad Prism 9. All data were expressed as the mean ± SEM from at least three independent experiments. To compare differences between two groups, an unpaired Student's t-test was performed, whereas for comparisons involving more than two groups, a one-way ANOVA with Tukey's post-test was conducted. Survival curves were calculated using the Kaplan-Meier method, and *p-values* were determined using the log-rank test. Recurrence-free survival was calculated from the date of HCC resection to the time of the first recurrence or death. Patients lost to follow-up were treated as censored events. Limiting dilution analysis (http://bioinf.wehi.edu.au/software/elda/) was performed using the chi-square test for pairwise differences in active cell frequency between groups. Statistical significance was set at *p* < 0.05, and all statistical tests were two-tailed. **p<*0.05*, **p<*0.01*,* and ****p*<0.001. The number of animals included per group is shown in each figure.

### Cell lines and culture conditions

The HCC cell line Huh7 was obtained from the Japanese Collection of the Research Biosciences Cell Bank. The HCC cell lines, MHCC97L and MHCC97H, were obtained from the Liver Cancer Institute, Fudan University (China) (40). The human liver cell line LO2 was obtained from the Institute of Virology, Chinese Academy of Medical Sciences (China). The 293T cell line was purchased from the American Type Culture Collection, whereas the 293FT cell line was purchased from Invitrogen. Sorafenib-resistant clones were established by subjecting HepG2 cells to continuous administration of gradually increasing sorafenib concentrations and trained up to 5.5 µM sorafenib (20-22). All cell lines used in this study were authenticated using STR profiling and evaluated in the absence of mycoplasma contamination.

### Tumor-initiating and self-renewal animal studies

Tumor-initiating and self-renewal abilities were investigated using limiting dilution and serial transplantation assays. Four- to six-week-old male NOD-SCID (NOD.Cg-*Prkdc^{scid} IL2rg^{tm1wjl}*/SzJ0) mice were subcutaneously injected with various numbers of cells. For secondary implantation, only one tumor was dissociated from each treatment group for passage into an additional eight mice. Tumor incidence and latency were recorded. The tumor-initiating frequency was calculated using extreme limiting dilution analysis. Tumor sizes were measured every three days using callipers, and tumor volumes were calculated as volume (cm³) = L × W² × 0.5, where L and W represent the largest and smallest diameters, respectively. Tumor initiation and incidence were determined as positive when the tumor volume reached 13.5 mm³. The tumors formed were harvested for histological analysis. Only tumors with sizes less than 200 mm³ were harvested and dissociated for subsequent passage to secondary mouse recipients or for *in vitro* limiting dilution analysis to minimize the number of necrotic cells within the tumor bulk. Animals that were injected with tumor cells but showed no signs of tumor burden were generally terminated six months after tumor cell inoculation, and animals were opened up at the injection sites to confirm that there was no tumor development.

### Metastasis animal studies

Metastasis was assessed by orthotopic injection into the liver to observe extrahepatic metastasis to the lungs. Luciferase-labelled cells were injected into the left lobes of the livers of 6-week-old BALB/c nude mice (n = 7 per group). Six to eight weeks after implantation, mice were administered 100 mg/kg D-luciferin (Gold Biotechnology) via peritoneal injection 5 min before bioluminescence imaging (IVIS 100 Imaging System, Xenogen). The livers and lungs were harvested for *ex vivo* imaging and histological analyses. The metastatic nodules in the lungs were counted.

### NRAS+AKT hydrodynamic tail vein injection (HTVI) mouse model of HCC

Six- to eight-week-old male wildtype C57BL/6 mice (Jackson Laboratory) were used in this study. HTVI procedure was conducted as previously described (41, 42). Briefly, 20 µg of plasmids encoding human AKT1 (myristylated AKT1 or myr-AKT1) and human neuroblastoma Ras viral oncogene homolog (N-RasV12) along with sleeping beauty (SB) transposase at a ratio of 25:1 was diluted in 2 ml of saline (0.9% NaCl), filtered through a 0.22 µm filter and injected into the lateral tail vein of C57BL/6 mice in 5-7 s. The constructs used in this study showed long-term expression of genes via hydrodynamic injection (42). Four weeks after hydrodynamic tail vein injection of proto-oncogenes and SB transposase, the mice were separated into two groups and administered 1 × 10¹¹ transducing units of AAV8 virus encoding either shNTC or shAgpat4 in 100 µL PBS via tail vein injection. At 5.5 weeks, mice were administered another dose of the shNTC or shAgpat4 AAV8 virus. Mouse shNTC and mouse shAgpat4 target sequences were cloned into the LV3 vector: 5'-CCGCGTGATGATTCTCACAAATTATCAAGAGTAATTTGTGAGAATCATCACG TTTTTTG-3' (SEQ ID NO:1) and 5'-CCGCACCAAAGGCTTTGCTATTACTTCAAGAGAGTAATAGCAAAGCCTTT GGTGTTTTTTG -3' (SEQ ID NO:2), respectively.

### Sorafenib non-responsive mouse model

Sorafenib (30 mg/kg/day, delivered orally daily) administration began at 2-3 weeks post HTVI injection, at which point tumors started to develop. Sorafenib treatment lasted for 2.5 weeks, when tumors became non-responsive to the treatment (21). The mice were then randomized for treatment with DMSO, sorafenib, AAV8-shNTC, or AAV8-shAgpat4 as described above. Tumor-bearing mice were euthanized at the humane endpoint. The livers were harvested for histological and ex vivo limiting dilution analyses.

### CL26 and sorafenib combination studies in HCC patient-derived xenografts

HCC PDX #1 were first expanded in NOD-SCID mice as described previously (21). Sorafenib-resistant HCC PDX #2 was established and expanded as described previously (29). Dissociated PDX tumors (5 × 10⁵ cells in 100% Matrigel) were orthotopically injected into the left lobes of the livers of 6-week-old BALB/c nude mice (n = 6-10 per group). Drug administration was initiated three weeks after inoculation. The mice were then randomized for treatment with DMSO, sorafenib, 30% PET (30%PEG400/15% ethanol/5% Tween 80 in saline), or CL26 (1 mg/kg or 10 mg/kg, delivered intraperitoneally thrice per week). Seven weeks post-inoculation, tumor-bearing mice were sacrificed. The livers were harvested for histological and ex vivo limiting dilution analyses.

### Animal toxicity studies

C57BL/6 mice were administered CL26 at a low dose (1 mg/kg) or high dose (10 mg/kg) intraperitoneally to evaluate potential tissue damage associated with CL26 exposure. Major organs, including the liver, spleen, pancreas, kidney, heart, and lungs, were weighed prior to fixation in 10% formalin for subsequent paraffin embedding and H&E staining. Additionally, the serum concentrations of alanine aminotransferase (ALT) and aspartate aminotransferase (AST) were measured. The above analysis was performed at the Veterinary Diagnostic Laboratory of the City University of Hong Kong.

### XTT assay and excess over bliss synergy analysis

Cells (1 × 10³ cells/well) were seeded in 96-well plates and cultured in complete medium for 24 h. Subsequently, sorafenib and/or CL26 was added to the culture and incubated for 48 h. The XTT tetrazolium reagent (Gold Biotechnology) and phenazine methosulfate (Thermo Fisher Scientific) were premixed and added to each well. The absorbance at 450 nm was measured using a Victor 3 microplate reader (PerkinElmer). Drug interactions were evaluated using the bliss independence model. According to this model, the expected growth inhibition (Eexp) resulting from the combination of drugs A and B was calculated using the following equation: Eexp = Ea + Eb - Ea × Eb, with Ea representing the growth inhibition caused by drug A alone and Eb representing the growth inhibition caused by drug B alone. To assess drug interactions, the difference between the observed growth inhibition (Eobs) and expected growth inhibition (Eexp) was calculated. A value of ΔE (Eobs - Eexp) exceeding 5 suggests a synergistic interaction between the two drugs.

### Viability assay

1 x 10⁴ cells were seeded in a 6-well plate and cultured in complete medium for 24 h. Subsequently, sorafenib and/or CL26 was added to the culture and incubated for 48 h. Dead cells were carefully washed with PBS, and the remaining viable cells were fixed using 4% paraformaldehyde (Sigma-Aldrich) and visualized by staining with 2% crystal violet (Sigma-Aldrich).

### Limiting dilution spheroid formation assay

Cells at limited dilutions were cultured in 100 µL serum-free DMEM/F12 medium (Invitrogen) supplemented with 20 ng/mL human recombinant epidermal growth factor (Sigma-Aldrich), 10 ng/mL human recombinant basic fibroblast growth factor (Sigma-Aldrich), 4 mg/mL insulin (Sigma-Aldrich), B27 (1:50; Invitrogen), 500 U/mL penicillin, 500 mg/mL streptomycin (Invitrogen), and 0.25%methylcellulose (Sigma-Aldrich). Cells were cultured in suspension in polyHEMA-coated 96-well plates. The cells were replenished with 30 µL of the supplemented medium every second day. The wells were monitored for sphere formation for 7-10 days. The tumor-initiating cell frequency was calculated using extreme limiting dilution analysis.

### LPA to PA conversion assay

Enzyme activity was measured by quantifying the conversion of [3H]-LPA to [3H]-PA following the protocol outlined in a previous study (43). Briefly, the enzymatic reaction was prepared by combining 10 µmol/L 18:1 Lyso PA (Avanti Polar Lipids), 50 µmol/L oleoyl CoA (Sigma-Aldrich), 1 µL 1-oleoyl-[oleoyl-9,10-³H]-LPA (specific activity 30-60 Ci/mmol; PerkinElmer), and 1 mg/mL fatty acid-free BSA in 200 µL 100 mM Tris-HCl buffer at pH 7.4. The reaction was initiated by adding 30 µg of total protein (cell lysate) and incubating the mixture at 37 °C for 10 min. To stop the reaction, 0.5 mL of 1-butanol containing 1 M HCl was added to extract phospholipids to stop the reaction. The butanol extract was then dried under vacuum, and the LPA and PA were separated using a solvent system consisting of chloroform, methanol, acetic acid, and water (25:10:3:1). Radioactive spots were identified by comparing their migration with unlabelled LPA and PA standards and visualized using iodine vapours. The spots corresponding to [3H]-LPA and [3H]-PA were scraped, and their radioactivity was measured using a Tri-Carb Liquid Scintillation Counter 4810 (PerkinElmer Life Sciences).

### RNA extraction, cDNA synthesis and quantitative real-time PCR (qPCR)

Total RNA was extracted using RNA-IsoPlus (TaKaRa) and cDNA was synthesized using PrimeScript RT Master Mix (TaKaRa). qPCR was performed with EvaGreen qPCR Master Mix (ABM) and the following primers:
AGPAT1:
   forward 5'-AGGACGCAACGTCGAGAAC-3' (SEQ ID NO:3) and
   reverse 5'-GCAGTACCTCCATCATCCCAAG-3'(SEQ ID NO:4);
AGPAT2:
   forward 5'-GCCGAGTTCTACGCCAAGG-3' (SEQ ID NO:5)
   reverse 5'-CGAACCAGCCGATGATGCT-3'; (SEQ ID NO:6)
AGPAT3:
   forward 5'-CTGCTGGTCGGCTTTGTCTT-3' (SEQ ID NO:7)
   reverse 5'-TCCAGAGTGAGTAGGCGAGG-3' (SEQ ID NO:8);
AGPAT4:
   forward 5'-CTCAGGGCTAATCATCAACACC-3' (SEQ ID NO:9)
   reverse 5'-GCTTGAGATGCAATAGGACAGT-3' (SEQ ID NO:10);
AGPAT5:
   forward 5'-CCGGCTCTACTGCGTCTA C-3' (SEQ ID NO:11)
   reverse 5'-GTCCTAGCGCATTCTGCCT-3' (SEQ ID NO:12);
SOX9:
   forward 5'-AGCGAACGCACATCAAGAC-3' (SEQ ID NO:13)
   reverse 5'-CTGTAGGCGATCTGTGGGG-3' (SEQ ID NO:14); and
β-actin:
   forward 5'-CATCCACGAAACTACCTTCAACTCC-3' (SEQ ID NO:15)
   reverse 5'-GAGCCGCCGATCCACACG-3' (SEQ ID NO:16) on a LightCycler 480 II analyser (Roche), with data analysed with the LightCycler 480 II software (Roche). The relative expression differences were calculated using the 2-ΔΔCt method.

### Protein extraction and western blot

Cells were lysed in RIPA buffer (Cell Signalling Technology) containing a complete protease inhibitor cocktail (Roche) and PMSF (Sigma-Aldrich). Protein lysates were quantified and resolved on an SDS-PAGE gel, transferred onto a PVDF membrane (Pall), immunoblotted with a primary antibody, and incubated with a secondary antibody. The antibody signal was detected using the Amersham ECL Select Western Blotting Detection Reagent (GE Healthcare). The following antibodies were used: anti-AGPAT1 (1:1000, Abcam, ab67018), anti-AGPAT2 (1:1000, Cell Signalling Technology, 14937), anti-AGPAT3 (1:1000, Thermo Fisher Scientific, PA5-101343), anti-AGPAT4 (1:1000, Thermo Fisher Scientific, PA5-62768), anti-AGPAT5 (1:1000, Abcam, AB127749), anti-β-actin (1:5000, Sigma-Aldrich, A5316), anti-GAPDH (1:5000, Cell Signalling Technology, 5174), anti-CD133 (1:500, Miltenyi Biotec, 130-092-395), anti-AFP (1:500, Dako, A0008), anti-SOX9 (1:1000; Millipore, ab5535), anti-p-mTOR (Ser2448) (1:1000, Cell Signalling Technology, 2971), anti-mTOR (1:1000, Cell Signalling Technology, 2983), anti-p-p70S6K (1:1000, Cell Signalling Technology, 9234), anti-p70S6K (1:1000, Cell Signalling Technology, 2708), anti-p-S6 (Ser235/236) (1:1000, Cell Signalling Technology, 4858), anti-p-S6 (Ser240/244) (1:1000, Cell Signaling Technology, 5364) and anti-S6 (1:1000, Cell Signaling Technology, 2217). Images were captured using Bio-Rad ImageLab Touch software (version 2.4.0.03).

### Lentiviral production and cell transduction

Human AGPAT4-specific and SOX9-specific shRNA expression vectors (pLKO.1) and shRNA scrambled non-targeting control (NTC) were purchased from Sigma-Aldrich. The sequences of the two shRNAs directed against human AGPAT4 and SOX9 are as follows:
AGPAT4 clone ID NM_020133.3 (clone 82): 
5’-CCGGATGATTGGTGTGACGGAAATTCTCGAGAATTTCCGTCACACCAAT
CATTTTTTG-3’ (SEQ ID NO:17)
AGPAT4 clone ID NM_020133.3 (clone 93): 
5’-CCGGGTGATCATAGAAAGGGTATTTCTCGAGAAATACCCTTTCTATGATC
ACTTTTTG-3’ (SEQ ID NO:18)
SOX9 clone ID NM_000346.4 (clone 1761): 
5’-CCGGCTCCACCTTCACCTACATGAACTCGAGTTCATGTAGGTGAAGGTGG
AGTTTTTG-3’ (SEQ ID NO:19)
SOX9 clone ID NM_000346.4 (clone 804):

5’-CCGGACTTCTGAACGAGAGCGAGAACTCGAGTTCTCGCTCTCGTTCAGAA
GTTTTTT G-3’ (SEQ ID NO:20)

The sequence of shRNA scrambled non-targeting control (NTC) is CCGGCAACAAGATGAAGAGCACAACTCGAGTTGGTGCTCTTCATCTTGTTGT TTTT (SEQ ID NO:21). Sequences were transfected into 293FT cells and packaged using the MISSION Lentiviral Packaging Mix (Sigma-Aldrich). The open reading frame (ORF) encoding the mRNA of AGPAT4 was cloned into the pDONR-221 donor vector (Hitrobio). It was then shuttled from pDONR-221 to pEZ-Lv199 (GeneCopoeia) through LR reaction. pEZ-Lv199 was used as an empty vector control. Sequences were transfected into 293T cells and packaged using the Lenti-Pac HIV expression packaging mix (GeneCopoeia). Virus-containing supernatants were collected for subsequent transduction to establish cells with stable AGPAT4 or SOX9 repression, or AGPAT4 overexpression. Puromycin was used for cell selection.

### Site-directed mutagenesis

PCR was performed using pLV199-AGPAT4 plasmid as a template and with the following primers:
C104A:
   forward 5'-TTGAAATTGACTTTCTGGCCGGCT-3' (SEQ ID NO:22),
   reverse 5'-GCTCCAGCCGGCCAGAA-3' (SEQ ID NO:23),
C175A:
   forward 5'-TGATTCACGCCGAGGGC-3' (SEQ ID NO:24),
   reverse 5'-GTGCCCTCGGCGTGA-3' (SEQ ID NO:25),
C228A:
   forward 5'-AGCTGTATATGACGCCACACT-3' (SEQ ID NO:26),
   reverse 5'-CTGAAATTGAGTGTGGCGTCA-3' (SEQ ID NO:27),
C270A:
   forward 5'-ACGATGACGAGGCCTCGG-3' (SEQ ID NO:28),
   reverse 5'-AGGCCGAGGCCTCGT-3' (SEQ ID NO:29). The PCR products were digested with DpnI and transformed into DH5α cells. Positive colonies were selected, and the presence of a C-to-A mutation was verified by sequencing.

### Immunohistochemistry (IHC)

The dewaxed and rehydrated slides were heated for antigen retrieval in sodium citrate buffer (pH 6. Endogenous peroxidase activity was inhibited by treatment with 3% hydrogen peroxide. Sections were subsequently incubated with AGPAT4 (1:1000, LSBio, NBP2-30948), followed by anti-rabbit HRP (Dako, K400311-2), and the reaction was developed using the DAB+ Substrate-Chromogen System (Dako). The slides were counterstained with Mayer's haematoxylin. Staining for AGPAT4 was quantified using the immunohistochemistry H-score as follows: H-score =ΣPi × (i + 1), where i is the intensity score (range 0-4), and Pi is the percentage of stained tumor cells at each intensity (range 0%-100%).

### Multiplex IHC

Dewaxed and rehydrated slides were subjected to antigen retrieval by heating in AR6 buffer (AKOYA Biosciences). To inhibit endogenous peroxidase activity, the Opal Antibody Diluent/Block (AKOYA Biosciences) was used. The sections were then sequentially incubated with the primary antibody, followed by Opal Polymer HRP Ms + Rb (AKOYA Biosciences) and Opal 570 (AKOYA Biosciences). For the second marker, the slides were heated with AR6 buffer, and another primary antibody was added, followed by Opal Polymer HRP Ms + Rb and Opal 520. Similarly, for the third marker, slides were heated with AR6 buffer, and another primary antibody was applied, followed by Opal Polymer HRP Ms+ Rb and Opal 690. The following primary antibodies were used: AGPAT4 (1:1000, LSBio, NBP2-30948), CD133 (1:100, Abcam, ab19898), AFP (1:200, Dako, A0008), and p-mTOR (1:1000, Cell Signaling Technology, 2971). Slides were counterstained with DAPI (AKOYA Biosciences), mounted using a Fluorescence Mounting Medium (DAKO), and visualized using the Vectra Polaris system (PerkinElmer).

### Immunofluorescence

Cells were fixed with 4% paraformaldehyde, permeabilized with 0.01% Triton X (Sigma-Aldrich), blocked with 5% bovine serum albumin and incubated with AGPAT4 (1:500, Novus Biologicals, NBP2-30948), followed by Alexa Fluor 488-conjugated secondary antibody (1:1000, Invitrogen, A11008). The cells were counterstained with anti-fade DAPI (Invitrogen) and visualized using a fluorescence confocal microscope (Carl Zeiss LSM 700).

### Annexin V-PI apoptosis assay

Cells were then treated with sorafenib or CL26 for 48 h. Following treatment, cells were harvested and stained with propidium iodide (PI) (BioLegend) and FITC-conjugated Annexin V (BioLegend). Samples were analysed on a BD LSRFortessa (BD Biosciences) with data analysed using FlowJo (BD Biosciences).

### Flow cytometry

Cells were stained with PE-conjugated CD133 (clone AC133) (1:100, Miltenyi Biotec, 130-080-801) or an equivalent amount of the corresponding isotype control (Thermo Fisher Scientific, 12-4714-82). Cells were analysed on a BD LSRFortessa (BD Biosciences), and data were processed using FlowJo (BD Biosciences).

### Luciferase reporter assay

For promoter activity, the full-length AGPAT4 promoter and truncations with one or two binding sites removed were amplified from the HCC cell line MHCC97L using the following primers: full-length (FL) F (5'-CCGGAGCTCAGGATAGACTCTAAATGGCACT-3' (SEQ ID NO:30)), T1 F (5'-CCGGAGCTCTCCCCCATCGCAATCAAA-3' (SEQ ID NO:31)), T2 F (5'-CCGGAGCTCCTGTAATAGTGGACATTTGAAAGG -3' (SEQ ID NO:32)), and R (5'- CCGCTCGAGAAGAGCTGTAAGTCAGCCA-3' (SEQ ID NO:33)). The amplified sequences were subsequently cloned into the pGL3-Basic luciferase reporter vector (Promega) using restriction enzymes SacI and XhoI. Luciferase reporter constructs were transiently transfected into HCC cells using Lipofectamine 3000 (Thermo Fisher Scientific). Luciferase activity was determined using the Dual-Glo Luciferase Assay System (Promega) and normalized to the co-transfected pRL-CMV Renilla luciferase control. Luciferase reporter activity was represented by the ratio of firefly to Renilla luminescence.

### Chromatin immunoprecipitation (ChIP) assay

ChIP was performed with the Magna ChIP G - Chromatin Immunoprecipitation Kit (Millipore) according to the manufacturer's instructions. Briefly, cells were crosslinked with 1% formaldehyde. The DNA was sonicated and immunoprecipitated with anti-SOX9 (Sigma-Aldrich, AB5535) or rabbit IgG control (Bethyl Laboratories, P120-101). Immunoprecipitated and eluted DNA was purified with columns and amplified by qPCR using the following primers: site 1 - F (5'-TCTCCCCGTCTCTCATCACA -3' (SEQ ID NO:34)) and R (5'-AAATAGCCCAAGCACATTGCAG-3' (SEQ ID NO:35)) and site 2 - F (5'-ATTCTGCAATGTGCTTGGGC-3' (SEQ ID NO:36)) and R (5'-AAGGGTGGGCTGTTGAAAGT -3' (SEQ ID NO:37)).

### Cell motility and invasion assays

Migration assays were conducted in 24well Millicell hanging inserts (Millipore), and invasion assays were conducted in 24well Millicell hanging inserts coated with Matrigel (Corning). Cells resuspended in serum-free DMEM were added to the top chamber, and medium supplemented with 10% FBS was added to the bottom chamber as a chemoattractant. After 48 h of incubation at 37 °C, cells that migrated or invaded through the membrane (migration) or Matrigel (invasion) were fixed and stained with crystal violet (Sigma-Aldrich). The number of cells was counted in three random fields under a 20x objective lens and imaged using the SPOT imaging software (Nikon).

### Recombinant protein preparation

The open reading frame (ORF) encoding the mRNA of AGPAT4 was cloned into pGEX-4T1 using restriction enzymes EcoRI and XhoI. The GST-AGPAT4 construct was expressed in BL21 Escherichia coli. Bacteria were pelleted by centrifugation at 5000 g for 10 min and lysed by pipetting up and down until homogenization in B-PER Reagent with 15 min incubation at room temperature. After centrifugation at 16,000g for 30 min, the supernatant containing the recombinant protein was collected and purified by incubation with glutathione-agarose beads for 1 h at 4 °C. The protein was then eluted from the beads using elution buffer (50 mM Tris, 150 mM NaCl, and 10 mM glutathione, pH 8.0) and subjected to dialysis to remove excess glutathione. Protein purity was examined by SDS-PAGE and silver staining.

### In vitro binding of CL26 with AGPAT4 by gel-based ABPP experiments

Purified human AGPAT4 protein (0.1 µg) was incubated with library compounds at 25 µM (unless indicated) for 1 h at room temperature, followed by incubation with IA-rhodamine (1 µM) for 1 h. Sampling buffer was added, and the samples were denatured by boiling at 90°C. The samples were resolved by SDS-PAGE and fluorescence signals were captured using ChemiDoc MP (Bio-Rad) before silver staining to assess protein loading. The band fluorescence intensity was quantified using the ImageJ software.

### Competitive gel-based ABPP in live cells

An in-gel fluorescence assay was performed to investigate the protein targets of CL26. Huh7 cells overexpressing AGPAT4, or cysteine mutants were pre-treated with CL26 at the indicated concentrations for 2 h, followed by CL26-alkyne (1 µM) treatment for another 2 h. Cells were harvested in RIPA buffer supplemented with protease and phosphatase inhibitors (Thermo Fisher Scientific #A32961). The proteins were precipitated by incubation with 6X volume of pre-chilled acetone at -20°C overnight. Proteins were pelleted by centrifugation at 5000 g for 10 min and resuspended in PBS. The protein content was measured using the BCA assay and normalized to 2 mg/mL. Cell lysates (50 µL) were labelled with azide-fluor 545 by click reaction (final reaction mixture: azide-fluor 545 (25 µM), copper(II) sulfate (1 mM), TBTA (100 µM), and freshly prepared TCEP (1 mM)) at room temperature with constant agitation. After incubation for 1 h, the reaction was quenched with the sampling buffer and boiled at 90°C for 5 min. Samples were subjected to SDS-PAGE, in-gel fluorescence was captured using ChemiDoc MP (Bio-Rad), and Coomassie Blue staining was performed. Similarly, immunoblotting was used to investigate the protein targets of CL26. Huh7 cells stably transfected with FLAG-AGPAT4 were pre-treated with either the solvent control or CL26 at the indicated concentrations for 2 h, followed by CL26-alkyne (10 µM) treatment for another 2 h. Cells were harvested, and lysates were prepared as described above unless specified. Anti-FLAG M2 affinity beads were added in FLAG-tag pull-down experiments. After 4 h of incubation at 4°C, the beads were washed with TBS-T and resuspended in TBS-T. Sampling buffer was added and the samples were boiled for protein elution. The samples were subjected to SDS-PAGE and imaged for fluorescence signals before western blot analysis. In the probe pull-down experiment, 1 mL of lysate was labelled with desthiobiotin (DTB)-azide by a click reaction (final reaction mixture: copper(II) sulfate (1 mM), TBTA (100 µM), DTB-PEG-azide (100 µM), and freshly prepared TCEP (1 mM)). Excess reagent was removed by overnight acetone precipitation. Proteins were pelleted by centrifugation and washed with 1 mL pre-chilled methanol. The protein was resuspended in 1 mL of 1.2% SDS/PBS and transferred to streptavidin agarose beads in PBS (final concentration: 0.2% SDS/PBS). After head-to-head rotation at 4°C overnight, the beads were washed thrice with PBS and thrice with water. Sampling buffer was added, and the samples were boiled for protein elution. The samples were subjected to SDS-PAGE, followed by western blot analysis or silver staining.

### In silico docking of CL26-AGPAT4 interactions

Schrödinger Maestro 13.4 was used for covalent docking. The structure of AGPAT4 (AF-Q9NRZ5V1) was retrieved for docking analysis. The protein structure was prepared using the protein preparation wizard tool by adding missing side chains and loops, removing water molecules, and optimizing hydrogen orientations at pH 7.0 using PROPKA. The protein content was minimized using the OPLS4 force field. The CL26 ligand structure was prepared using the LigPrep module, subjected to Epik at pH 7.0 (±2.0), and then minimized by the OPLS4 force field. Covalent docking was performed using CovDoc algorithm. Cys228 residue on AGPAT4 was chosen, with box size enabled docking of ligands of ≤20 Å length. Nucleophilic substitution was selected as reaction type. No constraints were imposed.

### Chemical synthesis and characterization

CL26. Chloroacetyl chloride (82.8 µL, 1.5 equiv.) was added to a stirring solution of 5-(2-benzothiazolyl)-2-furanmethanamine (200 mg, 1 equiv.) in anhydrous dichloromethane (4 mL), followed by the addition of triethylamine (300.8 µL, 2.5 equiv.) dropwise. The chemical structure for CL26 is shown below 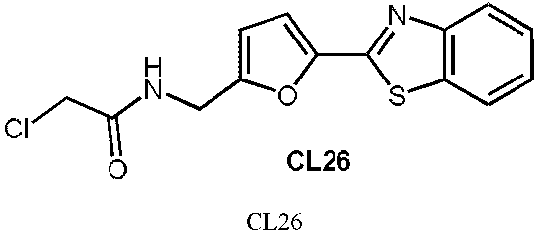 The mixture was allowed to react for 2 h at room temperature. After the reaction, any organic volatile solvent was evaporated under reduced pressure. The crude product was purified by column chromatography on silica gel using dichloromethane/methanol (92:8, v/v) as eluent, yielding CL26 as a yellow-brown powder (237.4 mg, 88%). 1H NMR (600 MHz, Chloroform-d) δ 8.08 (dt, J = 8.2, 0.9 Hz, 1H), 7.90 (ddd, J = 8.0, 1.2, 0.6 Hz, 1H), 7.51 (ddd, J = 8.3, 7.2, 1.2 Hz, 1H), 7.40 (ddd, J = 8.2, 7.2, 1.1 Hz, 1H), 7.15 (d, J = 3.5 Hz, 1H), 7.04 (s, 1H), 6.50 (dt, J = 3.5, 0.8 Hz, 1H), 4.62 (d, J = 5.8 Hz, 2H), 4.11 (s, 2H). 13C NMR (151 MHz, Chloroform-d) δ 165.91, 157.11, 153.68, 153.13, 148.39, 134.17, 126.60, 125.38, 123.19, 121.58, 112.76, 110.90, 42.52, 36.75, 31.60, 22.67, 14.13. 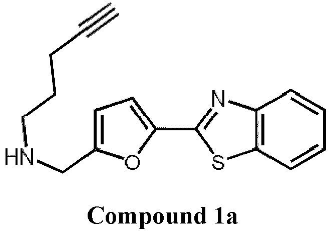 **Compound 1a**. 5-(2-Benzothiazolyl)-2-furanmethanamine (100 mg, 1 equiv.) in a mixture of acetonitrile (4.5 mL) and acetic acid (0.5 mL) was added to a stirring solution of pent-4-ynal (40.9 µL, 1 equiv.) in anhydrous acetonitrile (2 mL). Subsequently, sodium cyanoborohydride (43.7 mg, 1.6 equiv.) was added portion-wise. The mixture was allowed to react overnight at room temperature. Sodium hydroxide solution (1 M) was added to quench the reaction. The crude product was extracted and washed with brine twice. Any organic volatile solvent was removed under reduced pressure. The crude product was purified by column chromatography on silica gel using dichloromethane/methanol (95:5, v/v) as eluent, yielding 1a as a yellowish oil (17 mg, 13%). 1H NMR (600 MHz, Chloroform-d) δ 8.05 (dt, J = 8.1, 0.9 Hz, 1H), 7.89 (dt, J = 8.0, 1.0 Hz, 1H), 7.53 - 7.48 (m, 1H), 7.38 (ddd, J = 8.2, 7.2, 1.2 Hz, 1H), 7.17 - 7.06 (m, 1H), 6.49 (d, J = 3.5 Hz, 1H), 4.16 (d, J = 14.6 Hz, 1H), 4.00 (d, J = 14.5 Hz, 1H), 3.84 (t, J = 7.3 Hz, 1H), 2.55 - 2.40 (m, 2H), 2.09 - 1.93 (m, 3H), 1.83 - 1.62 (m, 2H). 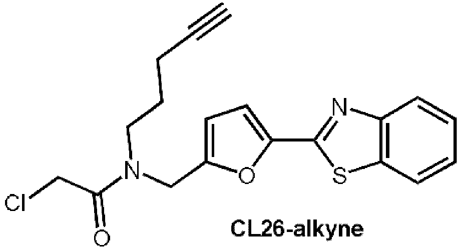 **CL26-alkyne.** Chloroacetyl chloride (9.1 µL, 2 equiv.) was added to a stirring solution of 1a (17 mg, 1 equiv.) in anhydrous dimethylformamide (1 mL), followed by the addition of triethylamine (20 µL, 2.5 equiv.) dropwise on ice. The mixture was allowed to react overnight. After reaction, the crude product was extracted with ethyl acetate and washed with brine twice. It was further dried over anhydrous magnesium sulphate and any organic volatile solvent was evaporated under reduced pressure. The crude product was purified by column chromatography on silica gel using dichloromethane/methanol (92:5, v/v) as eluent, yielding CL26-alkyne as a yellow-brown powder (7.4mg, 34.2%). 1H NMR (600 MHz, Chloroform-d) δ 8.05 (d, J = 8.2 Hz, 1H), 7.90 (d, J = 7.9 Hz, 1H), 7.54 - 7.48 (m, 1H), 7.40 (q, J = 7.3 Hz, 1H), 7.18 (d, J = 3.6 Hz, 1H), 6.62 (dt, J = 3.5, 0.8 Hz, 1H), 5.48 - 5.31 (m, 1H), 5.02 - 4.72 (m, 2H), 4.31 (s, 2H), 4.12 (q, J = 7.2 Hz, 1H), 2.36 (s, 2H), 2.15- 2.06 (m, 2H), 2.04 (s, 1H). 13C NMR (151 MHz, Chloroform-d) δ 166.93, 153.70, 150.33, 149.61, 134.25, 126.73, 123.29, 121.68, 112.31, 112.00, 80.81, 70.95, 46.58, 44.15, 40.99, 31.60, 22.66, 14.13.

### Results

### Identification of AGPAT4 as a potential metabolic driver of stemness in patients with HCC

To identify novel metabolic targets related to stemness, integrative analysis of publicly available datasets involving HCC clinical and stemness-related data was performed. First, all metabolism-related genes were isolated using KEGG pathway analysis. Of a total of 1341 metabolism-related genes, 333 were found to be frequently and significantly overexpressed in HCC compared to non-tumor liver in the TCGA Liver Hepatocellular Carcinoma (LIHC) dataset. It was further examined whether any of these preferentially overexpressed metabolic genes in HCC are related to the cancer stemness signature (GSE5975 and CSCdb) (9, 10) and worse overall survival. Thus, 62 genes linked metabolism, stemness and worse survival were identified **(****FIG. 1A****).** Pathway enrichment analysis on these 62 genes identified glycerophospholipid metabolism as significantly enriched (p=0.005; fold enrichment 6.99), with AGPAT4 ranking as the top hit. In the TCGA-LIHC dataset, AGPAT4 was found to be frequently overexpressed in HCC compared to normal liver, and high AGPAT4 was tightly correlated with worse overall survival and advanced tumor stage (T4 vs. T1/2/3) **(****FIG. 1B****).** Further analysis of genomic AGPAT4 expression in another publicly available dataset of liver tumors of Asian origin (GSE25097) (11) revealed a stepwise increase in AGPAT4 expression from healthy normal livers to cirrhotic livers and HCC tumors **(****FIG. 1C****).** In a separate GSE40367 dataset (12) that compared metastatic-free HCCs and HCCs with extrahepatic metastases, AGPAT4 expression was found to be significantly correlated with the presence of extrahepatic metastases **(****FIG. 1C****).** Immunohistochemical analysis using a tissue microarray comprising 53 paired primary HCC and matched non-tumor liver tissues revealed that proteomic AGPAT4 was frequently overexpressed in HCC, with >75% of the cases scored as high expression, whereas <10% of the non-tumor liver cases scored in the same category **(****FIG. 1D****).** Of the 53 paired cases examined, 44 (83%) exhibited higher AGPAT4 expression in HCC than their adjacent non-tumor counterparts **(****FIG. 1E****).** High proteomic AGPAT4 levels were also significantly correlated with worse overall survival **(****FIG. 1F****).** Data obtained from The Human Protein Atlas database also showed that AGPAT4 was expressed at extremely low levels in the liver **(****FIG. 9A****).** Of note, of all the other AGPAT family members that are classified as lysophosphatidic acid acyltransferases (LPAATs) (e.g. AGPAT1 to 5), only AGPAT1 was found up-regulated in HCC as compared to non-tumor liver in the TCGA-LIHC dataset **(****FIG. 9B****),** but none was found to be associated with a less differentiated tumor stage (G1 vs. G4;**FIG. 9C****).** AGPAT4 was not found to be elevated in non-alcoholic steatohepatitis (NASH)-related human HCC as compared to its non-tumor counterparts **(****FIG. 9D****),** suggesting that it may not be involved in HCC driven by this aetiology. TCGA analysis also showed HNSC (head and neck squamous cell carcinoma), LIHC (liver hepatocellular carcinoma) and PAAD (pancreatic adenocarcinoma) to have higher AGPAT4 expression in their tumors versus their non-tumor counterparts. The analysis also shows top 10 tumors that have high AGPAT4 expression (e.g. not necessarily higher AGPAT4 in tumor vs. non-tumor), and they are colorectal cancer, head and neck cancer, stomach cancer, pancreatic cancer, urothelial cancer, testis cancer, cervical cancer, endometrial cancer, ovarian cancer and skin cancer.

### Liver-specific knockdown of Agpat4 elicits anti-tumor effects in an immunocompetent HCC model.

To investigate the causative relationship between AGPAT4 overexpression and its functional role in HCC, endogenous knockdown of Agpat4 expression was performed in an immunocompetent mouse model. Using the hydrodynamic tail vein injection (HTVI) delivery approach, HCC tumors were induced in C57BL/6 mice using a combination of activated forms of myristylated AKT (myr-AKT) and N-RasV12 proto-oncogenes (NRAS+AKT) and sleeping beauty (SB) transposase. Note that the NRAS+AKT combination was chosen as activation of both the RAS/MAPK and PI3K/AKT/mTOR pathways is frequently detected in almost 50% of HCC patients (13), and a significant co-occurrence of high AGPAT4 expressing HCC and the presence of NRAS+AKT mutations/overexpression was noted. **(****FIG. 2A****).** A stepwise increase in Agpat4 mRNA expression was observed in the livers of the mice injected with the empty vector (EV) control alone to early (1-3 weeks after injection) and advanced HCC (4-5 weeks after injection), and subsequently to the endpoint, at which time the mice died naturally (6-8 weeks after injection) **(****FIGs. 2B-2C****).** Knockdown of endogenous Agpat4 expression delivered via tail vein injection of adeno-associated virus 8 (AAV8) viral particles significantly attenuated the liver-to-body weight ratio and extended survival compared to those of the shRNA scrambled non-targeting control (shNTC) **(****FIG. 2D-****2E).** Notably, liver-free body weight was not significantly different between the two groups, excluding data bias due to tumor burden-induced cachexia **(****FIG. 2F****).** Subsequent *ex vivo* limiting dilution assays using cells isolated from the liver of this HCC mouse model demonstrated that the frequency of tumor-initiating cells capable of forming spheres also decreased upon Agpat4 knockdown **(****FIG. 2G****).** Immunohistochemical analysis of the resected tumors confirmed a marked decrease in the expression of Agpat4 in the Agpat4-knockdown group **(****FIG. 2H****).** These findings support a key role for AGPAT4 in promoting HCC growth and self-renewal.

### AGPAT4 induces HCC tumor-lineage plasticity and metastasis

Next explored was the involvement of AGPAT4 as a possible oncofetal gene. Fetal mouse livers at different developmental stages were collected (14, 15) and the relative expression of Agpat4 was measured by qPCR. Agpat4 expression peaked on embryonic days 16-17 (E16-17) and then rapidly decreased during hepatocyte differentiation **(****FIG. 3A****),** which mirrored the expression of several previously reported liver progenitor markers, including Krt19, Krt7, Sox9, and Afp (14, 15). In a separate *in vitro* differentiation model (16), in which human embryonic stem cells were differentiated into hepatocytes, AGPAT4 was also found to be elevated in embryonic stem cells, endoderm, and liver progenitor states, with its expression dropping drastically upon hepatocyte differentiation **(****FIG. 3B****).**

To further confirm this in a human setting, TCGA-LIHC data was analyzed and it was found that AGPAT4 expression progressively increased from well to poorly differentiated HCC tumors (histological grade/differentiation stages 1 to 4) **(****FIG. 3C****),** with high AGPAT4 expression clustering well with hepatic progenitor markers (in contrast to its negative correlation with mature hepatocyte markers) **(****FIG. 3D****).** HCC patients with high AGPAT4 expression were also enriched in the HCC stem cell signature **(****FIG. 3E****);** while stable overexpression of AGPAT4 in Huh7 cells resulted in elevated levels of liver cancer stem/progenitor markers CD133 and AFP **(****FIGs. 3F-****3G;****FIGs. 10A-10B****).** Consistently, immunofluorescence analysis of NRAS+AKT HTVI HCC tumors with or without Agpat4 expression demonstrated decreased Cd133 and Afp levels upon Agpat4 suppression **(****FIG. 3H****).** AGPAT4 knockdown resulted in a 2-fold decrease in the ability of cells to self-renew, as demonstrated by the decreased tumor-initiating frequency *in vitro,* while overexpression yielded a similar enrichment trend **(****FIG. 3I****;** **FIGs. 10A-10B****).** Knockdown of AGPAT4 also diminished tumorigenicity *in vivo,* with a marked decrease in tumor incidence and tumor-initiating frequency, concomitant with increased tumor latency **(Table 1)** and tumor-free survival, as compared to HCC cells expressing the shRNA scrambled non-targeting control **(****FIG. 3J****;** **FIGs. 10C-10D****).** Furthermore, knockdown of AGPAT4 significantly reduced stem cell frequency *ex vivo* when compared to the shRNA scrambled non-targeting control **(****FIG. 3K****).** Collectively, these data suggest a link between AGPAT4 and oncofetal stemness characteristics.

As tumor plasticity is also closely linked with epithelial-mesenchymal transition and metastasis, the functional role of AGPAT4 in regulating metastatic events was explored. Knockdown of AGPAT4 significantly attenuated the ability to migrate and invade compared with control cells, while overexpression of AGPAT4 enhanced these aggressive effects **(****FIG. 3L****).** These findings were confirmed in an *in vivo* experimental metastasis model in which cells were orthotopically injected into the liver to observe metastasis to the lung. AGPAT4 suppression induced a potent decrease in the ability of MHCC97L cells to metastasize to the lungs (sh82: 5 of 7 tumors formed in the liver with 0 developing extrahepatic metastases in the lung; sh83: 7 of 7 tumors formed in the liver with 0 developing extrahepatic metastases in the lung). In contrast, MHCC97L control cells resulted in tumor growth in the liver in 7 of 7 mice injected, with 5 mice going on to develop lung metastasis **(****FIG. 3M****).** Mice were sacrificed after 8 weeks, and both the livers and lungs were removed for histological analyses. Haematoxylin and eosin (H&E) and immunohistochemical staining of the tumors confirmed the bioluminescence signals observed to represent tumor cells **(****FIG. 10E****)** and that AGPAT4-overexpressing tumors were more invasive in terms of the number of AGPAT4-expressing cells that could be detected across the tumor boundary **(****FIG. 10F****),** concomitant with the increased ability of the cells to metastasize to the lung, as evidenced by the increased number of tumor nodules present in the lung **(****FIG. 3N****).**

### SOX9 stem cell transcription factor drives AGPAT4 promoter activity and transcription

In order to investigate the potential upstream regulatory element responsible for AGPAT4 overexpression in HCC, the Gene Transcription Regulation Database (GTRD), a publicly available collection of ChIP-seq data and LASAGNA, a transcription factor binding site prediction algorithm, was used to predict gene transcription factors binding to the AGPAT4 promoter. The analysis revealed that the self-renewal-related transcription factor SOX9 contains two high-scoring predicted binding sites, including GATCAATGC at -1065 to -1057 and TGACAATGG at -862 to -854 **(****FIG. 11A****).** Correlation analysis in human HCC tumor samples showed that SOX9 and AGPAT4 expression was positively correlated **(****FIG. 11B****).** To test the ability of SOX9 to control AGPAT4 expression, AGPAT4 wild-type and mutants with either one or both predicted truncated binding sites were generated **(****FIG. 11C****),** and then performed a luciferase reporter assay. A significant reduction in luciferase activity was observed when either one or both of the predicted binding sites were truncated, suggesting that the two predicted SOX9 binding sites are critical for controlling AGPAT4 transcription **(****FIG. 11C****).** The binding of SOX9 to the AGPAT4 promoter by ChIP-qPCR was assessed, where the AGPAT4 promoter at predicted binding sites 1 and 2 showed 30.6- and 9.9-fold enrichment, respectively, in SOX9 binding compared to ChIP with nonspecific IgG **(****FIG. 11D****).** Consistently, stable knockdown of SOX9 by a lentiviral-based strategy resulted in a marked reduction in AGPAT4 transcription and protein expression **(****FIGs. 11E-11F****).**

### AGPAT4 activates the mTOR pathway through phosphatidic acid (PA) production axis

To decipher the downstream molecular mechanism by which AGPAT4 drives HCC, pathway analysis was conducted with HCC samples segregated into high and low AGPAT4, where enrichment of PI3K/AKT/mTOR signalling was apparent **(****FIG. 4A****).** Western blotting validated the downregulation of p-mTOR, p-S6K, and p-S6 upon AGPAT4 suppression, while the opposite observations were also noted upon AGPAT4 overexpression **(****FIG. 4B****).** To further validate the functional role of the mTOR signalling pathway in linking AGPAT4-mediated cancer stemness behaviours, rescue experiments were performed. The utilization of rapamycin (mTOR inhibitor) and LYS6K (S6 kinase inhibitor) in HCC cells overexpressing AGPAT4 demonstrated their ability to rescue the AGPAT4-induced increase in metastasis **(****FIG. 4C****),** and self-renewal **(****FIG. 4D****),** and p-mTOR/S6K signalling activation **(****Fig. 4B****).** Deregulated p-mTOR signalling was also confirmed by multiplex immunohistochemistry in mouse tissues harvested from NRAS+AKT HTVI HCC tumors and orthotopic MHCC97L HCC tumors with or without AGPAT4 suppression **(****FIG. 4E****;** **FIGs. 12A-12B****).** Following SOX9 knockdown, attenuated p-mTOR, p-S6K, and p-S6 were also observed **(****FIG. 11F****),** confirming the SOX9-AGPAT4-p-mTOR signalling axis. As AGPAT family members are known to catalyse the conversion of lysophosphatidic acid (LPA) to phosphatidic acid (PA) under normal physiological conditions (17, 18), and PA is known as an upstream regulator of mTOR signalling (19), tests were conducted to determine whether the addition of exogenous PA in HCC cells could promote similar cancer and stemness properties. Manipulation of AGPAT4 expression in HCC cells was positively correlated with PA production **(****FIG. 4F****).** The addition of PA also markedly enhanced the self-renewal, migration, and invasion capacities of HCC cells, concomitant with the activation of p-mTOR, p-S6K, and p-S6 signalling **(****FIG. 4G-4I****).** Overall, the data suggest that AGPAT4-mediated changes in PA production would alter mTOR signalling in HCC to confer aggressive cancer features.

### Overexpression of AGPAT4 confers sorafenib resistance in HCC cells and its expression is associated with patient's response to sorafenib in the clinic

To test whether AGPAT4-mediated cancer cell plasticity is related to drug resistance of HCC cells, examination was performed for altered AGPAT4 expression levels in sorafenib-sensitive and sorafenib-resistant HCC patient-derived xenografts and HepG2 cells (20-22), as well as control and sorafenib-treated NRAS+AKT HTVI HCC mouse model. AGPAT4 expression was markedly enhanced in sorafenib-treated and sorafenib-resistant groups **(****FIG. 5A-5C****),** concomitant with activation of mTOR signalling **(****FIG. 5D****).** Notably, of all AGPAT LPAAT family members, only AGPAT4 was found to be enriched in sorafenib-resistant HCC cells compared to their sensitive counterparts **(****FIG. 13A****).** To further substantiate the role of AGPAT4 in mediating sorafenib resistance in HCC, AGPAT4 was knocked down in sorafenib-resistant HepG2 cells and it was demonstrated that this could reverse ability of the cells to migrate, invade, self-renew as well as resist sorafenib-induced cell death **(****FIG. 5E-5H****).** Manipulation of AGPAT4 expression by overexpression in Huh7 and knockdown in MHCC97L cells also resulted in a similar phenomenon, further suggesting that AGPAT4 expression is critical in conferring resistance to sorafenib **(****FIG. 14B****).** Rescue experiments with rapamycin or LYS6K in AGPAT4-overexpressing cells further confirmed the significance of p-mTOR and p-S6K in AGPAT4-mediated sorafenib resistance **(****FIGs. 13C-13D****).** The exogenous addition of PA also similarly conferred resistance of cells to the treatment (**FIG. 13E**).

Considering the functional importance of AGPAT4 in conferring resistance to sorafenib treatment in HCC, the significance of AGPAT4 was investigated in the context of sorafenib treatment after liver resection in HCC patients. An independent cohort of 79 patients with HCC (paraffin-embedded tissues of patients who received sorafenib treatment after hepatectomy) was analysed. HCC patients with low AGPAT4 levels showed a significantly better prognosis (overall and recurrence-free survival) after sorafenib treatment than patients with high AGPAT4 levels **(****FIG. 6A-6B****).** High AGPAT4 levels in these patients also correlated with vascular invasion, advanced pathological stage, enlarged tumor size, and non-responsiveness to sorafenib treatment **(****FIG. 6C****),** suggesting that AGPAT4 may serve as a marker for patient response/stratification.

### AAV8-mediated liver-directed shAgpat4 and sorafenib combination reduces tumor burden in a pre-clinical HCC mouse model

Next, AGPAT4 dependency was investigated in a proof-of-principle therapeutic experiment. As demonstrated above, AGPAT4 was elevated in NRAS+AKT-driven HCC **(****FIG. 2C****).** This model was used and HCC tumors were treated with either sorafenib alone or in combination with AAV8 viruses encoding Agpat4 knockdown, with the latter delivered intravenously into the mice **(****FIG. 6D****).** AAV8 viruses encoding the Agpat4 knockdown and sorafenib combination produced a maximal suppression effect, as evidenced by the prolonged survival time **(****FIG. 6E****),** reduced tumor-initiating cell frequency, as measured by subjecting the harvested tumor cells to a limited dilution spheroid formation assay *ex vivo* **(****FIG. 6F****)** and suppressed liver-to-body weight ratio **(****FIG. 6G****).** Immunofluorescence was performed to demonstrate successful knockdown of Agpat4 *in vivo.* Although sorafenib treatment alone led to an increase in Agpat4 and p-mTOR expression in the control group, their expression was notably suppressed in the Agpat4 repressed group **(****FIG. 6H****).** Combination treatment also resulted in a significant decrease in the expression of stemness genes, including Cd133 and Afp **(****FIG. 12A****).**

### Discovery of an isoform-specific covalent inhibitor targeting Cys228 on AGPAT4

In view of the importance of AGPAT4 in promoting oncogenic tumor lineage plasticity and sorafenib resistance in HCC, specific small-molecule inhibitors for AGPAT4 were developed, which have not been reported previously but have immense potential for treating HCC. An ideal therapeutic target should be abundant in tumor cells but have low or negligible expression levels in normal tissues. As the current data suggest, AGPAT4 is highly expressed in the endoderm, and liver progenitor, and HCC states, but has markedly lower expression in mature normal hepatocytes **(****FIG. 3B****),** thus rendering AGPAT4 to be an ideal target. Analysis of the primary protein sequence and tertiary structure of AGPAT4 and its related protein isoforms (AGPAT1, 2, 3 and 5) revealed a unique Cys228 on AGPAT4. Cys228 is positioned adjacent to the active HX₄D motif, which is important for the acyltransferase activity of AGPAT4, suggesting that targeting of Cys228 by covalent ligands could lead to potent inhibition of AGPAT4. Targeting cysteine residues on oncoproteins is a promising approach for the development of covalent drugs for targeted cancer therapy. This can be attributed to the p*K*ₐ of 8-9 for cysteine thiol, which exhibits significant changes in reactivity depending on the protein microenvironment (23), thus enabling specific targeting of a cysteine on the oncoprotein. Thus, cysteine-reactive covalent inhibitors for AGPAT4 were developed using activity-based protein profiling (ABPP), a well-established chemoproteomics platform for developing therapeutic covalent ligands (23-28).

The *in vitro* binding of cysteine-reactive compounds to AGPAT4 were investigated using a gel-based ABPP experiment. In this competitive binding experiment, binding events to AGPAT4 were identified by the diminished fluorescence signal from iodoacetamide-rhodamine (IA-rhodamine) compared to the solvent control. Among the few hits, CL26 demonstrated dose-dependent binding with the highest binding affinity toward AGPAT4, as indicated by an IC₅₀ of 766 nM **(****FIGs. 14A-14C****).**

After identifying the strong *in vitro* binding of CL26 to AGPAT4, the target engagement of CL26 with AGPAT4 in HCC was investigated. A molecular probe for CL26, CL26-alkyne, was synthesized by introducing an alkyne handle onto CL26. This allowed functionalization through copper-catalysed azide-alkyne cycloaddition (CuAAC) for analysis and identification of the binding protein(s) **(****FIG. 7A****).** By treating Huh7 cells stably expressing FLAG-AGPAT4 with CL26-alkyne, AGPAT4 was found to be a binding protein of CL26-alkyne, as supported by immunoblotting and in-gel fluorescence imaging experiments after anti-FLAG pulldown **(****FIG. 7B****).** Pre-treatment of Huh7 cells with CL26 resulted in a significant decrease in fluorescence intensity, suggesting that CL26 and CL26-alkyne share the same protein target in HCC cells; that is, CL26-alkyne can serve as a probe of CL26 for target identification, and hence AGPAT4 is the cellular target of CL26 in HCC. To investigate the specificity of CL26 toward AGPAT4 over other AGPAT LPAAT family members, Huh7 cells were treated with CL26-alkyne, and the cell lysates were reacted with DTB-PEG-azide by CuAAC. This allows the installation of a DTB moiety onto CL26-alkyne-bound proteins; and as a result, these bound proteins can be enriched by streptavidin pulldown and identified by immunoblotting. AGAPT4 was the only protein bound to CL26-alkyne among the five family members **(****FIG. 15****),** confirming the specificity of CL26 for AGPAT4 in HCC.

The impact of CL26 on the inhibition of acyltransferase activity of AGPAT4 *in vitro* was verified in Huh7 cells overexpressing AGPAT4, and in sorafenib-resistant HepG2 cells that have enriched AGPAT4 expression. This was accomplished using the PA conversion assay **(****FIG. 7C****).** To assess the synergistic effect of combining sorafenib and CL26 treatment, XTT proliferation and cell viability assays were conducted in HCC cells exhibiting varying levels of AGPAT4 expression. The synergistic effect was observed solely in cells with high AGPAT4-expressing MHCC97L and MHCC97H cells, as indicated by the excess over bliss scores **(****FIG. 7D****).** Similarly, the synergistic effect of combination treatment was exclusively observed in sorafenib-resistant HepG2 cells **(****FIG. 7E****).** Furthermore, the impact of the combination treatment on apoptosis, self-renewal, migration, invasion, and the mTOR/S6K pathway was confirmed using the Annexin V apoptosis assay **(****FIG. 7F** **and** **FIG. 16A****),** *in vitro* limiting dilution spheroid formation **(****FIG. 7G** **and** **FIG. 16B****),** and migration and invasion assays **(****FIGs. 17A-****17B)** and western blot analysis **(****FIG. 7H** **and** **FIGs. 18A****-18B),** respectively.

The binding site of CL26 on AGPAT4 was examined using a genetic mutation experiment, with focus on cysteine residues that were in close proximity to the acyltransferase domain and excluded cysteine residues in the transmembrane region to which the ligand is least accessible- Cys104, Cys175, Cys228, and Cys270. The effect of these cysteine-to-alanine (C-to-A) mutations was tested in Huh7 cells with similar levels of overexpression in the AGPAT4 wild-type (WT) or mutants (**FIG. 7I**). The C228A mutant showed maximal attenuation of acyltransferase activity **(****FIG. 7J****)** and downstream mTOR/S6K pathways **(****FIG. 18C****).** In addition, the C228A mutant rescued the synergistic effect observed across proliferation, self-renewal, and sensitivity to sorafenib **(****FIGs. 7K-7L****;** **FIGs. 18D-18F****).** C228A mutant also did not show difference in LPA to PA conversion when treated with CL26 as compared to the DMSO control **(****FIG. 18G****).** The Huh7 C-to-A mutation at these cysteine sites was also incubated with the CL26-alkyne probe. Subsequent CuAAC reaction to install a fluorophore onto the CL26-alkyne-bound proteins and parallel immunoblotting by anti-AGPAT4 enabled visualization of the extent of CL26-alkyne binding onto AGPAT4 with these mutations. Notably, only the AGPAT4 C228A mutation resulted in the complete abolishment of the fluorescence signal from the AGPAT4 protein **(****FIG. 7M****),** indicating that no CL26-alkyne was bound to the C228A mutant protein. Therefore, Cys228 is the primary binding site for CL26 on AGPAT4. Consistently, covalent docking also suggested that Cys228 harboured the highest docking score for CL26, further substantiating our data **(****FIG. 19****).** In addition, the docking model indicated that CL26 bound covalently to Cys228 and non-covalently to Trp106 and Trp136 through π-π interactions **(****FIG. 20****).** As CL26 is positioned in close proximity to the HX₄D catalytic motif (aa 96-101) and the substrate-binding site of AGPAT4, it is likely to interfere with the lengthy LPA interaction. Therefore, it is believed that CL26 binding could induce steric hindrance in substrate binding and hence impede the acyltransferase activity of AGPAT4.

### CL26 exhibits specificity for AGPAT4 in HCC

ABPP experiments were performed to investigate off-target binding from CL26. Huh7 cells expressing empty vehicle (EV) or FLAG-AGPAT4 were incubated with CL26-alkyne, followed by a CuAAC reaction to install fluorophores onto CL26-alkyne-bound proteins. Owing to the very low expression level of AGPAT4 in Huh7 cells, there was only weak in-gel fluorescence from EV cells, suggesting minimal off-target binding of CL26 in Huh7 cells. In contrast, a much stronger fluorescent band was observed in FLAG-AGPAT4 expressing cells, highlighting the specific targeting of AGPAT4 by CL26 **(****FIG. 7N****).** In addition, by reacting with DTB-PEG-azide instead of fluorophore-azide through CuAAC, CL26-alkyne-bound proteins, including the protein target and any off-targets of CL26, were enriched by streptavidin pulldown and subsequently visualized by silver staining after SDS-PAGE. Only a few protein bands were found in the stained gel, with the major protein band identified as AGPAT4 in a parallel immunoblotting experiment **(****FIG. 21****),** illustrating the high specificity of CL26 for AGPAT4, with minimal off-target binding.

### Selective AGPAT4 targeting by CL26 sensitizes HCC tumors to eradication by sorafenib treatment with no evidence of toxicity

To assess the therapeutic potential of combining CL26 treatment with sorafenib, two patient-derived xenograft (PDX) mouse models (HCC PDX model #1 and sorafenib-resistant HCC PDX model #2) (29) were used in which PDXs were orthotopically engrafted into the mouse liver **(****FIG. 8A****).** The combination treatment, administered orally for sorafenib and intraperitoneally for CL26, exhibited a significant effect in reducing tumor weight **(****FIGs. 8B-8C****),** number of tumor nodules **(****FIG. 22A****)** and tumor size **(****FIG. 22B****).** Subsequent *ex vivo* limiting dilution analysis demonstrated a decreased self-renewal ability due to the combination treatment **(****FIG. 8D****).** Notably, combination treatment also yielded the highest survival rate in the mouse model **(****FIG. 8E****).** The inhibitory effect of CL26 on acyltransferase activity in sorafenib-resistant PDX was confirmed using a PA conversion assay **(****FIG. 22C****),** accompanied by a reduction in p-mTOR levels, as indicated by multiplex immunohistochemistry analysis **(****FIG. 22D****).** Importantly, following treatment with CL26, mice did not exhibit significant differences in body weight compared with the control group **(****FIG. 22E****).** Furthermore, minimal toxicity associated with CL26 treatment was observed, with no significant differences in blood chemistry [e.g. levels of aspartate aminotransferase (AST) and alanine aminotransferase (ALT)], major organ weight and histology from those in control mice **(****FIGs. 8F-8H****).**

### Discussion

Relative to the huge amount of development and differentiation that occur during organogenesis (embryonic differentiation), cells are typically restricted to the extent to which they can differentiate. This restriction allows cells to remain organized and functional within their respective tissues. However, in cancer, cells undergo molecular and phenotypic changes that allow them to adopt different identities along a phenotypic spectrum referred to as cellular plasticity. Different types of phenotypic plasticity can be unlocked during cancer development, including (1) dedifferentiation, (2) blocked differentiation, and (3) transdifferentiation. Phenotypic plasticity describes the ability of cancer cells to undergo dynamic, non-genetic cell state changes that amplify cancer heterogeneity to promote therapy evasion. Human tumors contain less differentiated cells that are resistant to therapy and are associated with the development of relapse and metastasis. There is ample evidence linking plasticity to tumor stemness, therapy resistance, and tumor recurrence. Notably, a study utilizing machine learning found stemness features associated with oncogenic dedifferentiation to inform new drug targets for anti-cancer therapies (30). Numerous studies have shown that pluripotent transcription factors and developmental signalling pathways of embryonic stem cells are invariably hijacked by cancer stem cells, termed 'oncofetal drivers' in many cancers. Oncofetal drivers or proteins, which are predominantly expressed during embryonic development but are silenced in fully differentiated cells, present potential therapeutic windows for tumors that express these proteins. In this study, the metabolic acyltransferase protein AGPAT4 was identified as an oncofetal protein that is abundant in embryonic stem cells and HCC tumor cells but is low or absent in most normal tissues.

In the present study, it was demonstrated that AGPAT4 is a functional regulator of tumor lineage plasticity, which correlates with enhanced metastasis and resistance to sorafenib. Heightened plasticity was induced, at least in part, as a result of increased phosphatidic acid (PA) (a product of AGPAT4 through conversion of LPA to PA), which then acts on its downstream mTOR/S6K/SK signalling pathway to drive sorafenib resistance and tumor recurrence. Using a chemical biology approach, the latter part of this study identified an isoform-specific covalent inhibitor CL26 that could target AGPAT4 at its unique cysteine residue at position 228 and further demonstrated that it could sensitize AGPAT4+ HCC tumors with cancer cell plasticity to sorafenib therapy. Preclinical *in vitro* and *in vivo* patient-derived xenograft data demonstrated that CL26 effectively inhibited the acyltransferase activity of AGPAT4 in both settings. This suggests that CL26 and sorafenib combination should be tested in clinical trials for the treatment of AGPAT4+ HCC tumors and/or late-stage sorafenib-refractory HCC patients. Additionally, toxicological analysis revealed minimal side effects of CL26 on other major organs in mice, indicating its promising safety profile. It should be noted that while an elevated AGPAT4 expression in sorafenib non-responders than responders on the CARE (Computational Analysis of Resistance) platform (http://care.dfci.harvard.edu/) was observed, this association was not observed in lenvatinib responding and non-responding HCC patients **(****FIG. 23****).**

AGPAT belongs to a large family of enzymes, with some isoforms previously reported to be involved in fatty acid remodelling of phospholipids and tumor growth. Of the many AGPAT isoforms, only AGPATs 1 to 5 are classified as lysophosphatidic acid acyltransferases (LPAATs) that are responsible for the conversion of lysophosphatidic acid (LPA) to phosphatidic acid (PA). PA, apart from its role as an intermediate in TAG synthesis, is also a precursor of glycerophospholipids and an intracellular signalling molecule with impact on a wide range of cellular processes (17). In contrast, AGPATs 6 to 11 are classified as lysophospholipid acyltransferases (LPLATs), based on their substrate specificities (31). A recent study discovered that high expression of AGPAT5 was related to increased stemness in patients with HCC from the TCGA-LIHC cohort. This was demonstrated by a higher stemness score (mRNAsi), indicating a greater level of stemness (32). Increased levels of AGPAT2 have been linked to poor prognosis for ovarian cancer patients, and AGPAT2 inhibitors have demonstrated anti-tumor effects in xenograft mouse models (33, 34). Murine Agpat4 mRNA expression is high in the brain, and moderate to low in skeletal muscle, gut, kidney, spleen and lung (31). AGPAT4 knockout mice are available but they are reported to be viable, fertile and grossly similar to their wild-type littermates. A more recent study found that the Agpat4/LPA axis in colorectal cancer cells regulates antitumor responses through p38/p65 signalling in macrophages (35). However, the association between AGPAT4, cancer cell plasticity, and sorafenib resistance has not been explored. Furthermore, there is currently no selective inhibitor that specifically targets AGPAT4, highlighting a gap in the availability of targeted inhibitors for this particular member of the AGPAT family.

As mentioned above, AGPAT LPAATs are responsible for the conversion of LPA to PA. While one study reported the regulation of stemness-related Hippo pathway by PA-mediated lipid-protein interaction in cancer (36), the role of LPA to PA conversion in HCC stemness and drug resistance remains elusive. PA has been shown to activate the mTOR signalling pathway by regulating mTORC1 (37). Notably, recent studies focusing on acute myeloid leukaemia and liver cholangiocarcinoma have identified the AKT/mTOR pathway as an escape mechanism from sorafenib treatment, indicating its involvement in drug resistance (38, 39). However, the specific connection between the AGPAT4/PA/mTOR axis and sorafenib in HCC has not yet been reported, and the findings of this study fill this knowledge gap.

Covalent ligand screening through an activity-based protein profiling (ABPP) experiment, in which a cysteine-reactive covalent ligand, CL26, with strong *in vitro* binding (IC₅₀=766 nM) onto AGPAT4 was successfully identified. The target engagement of CL26 with AGPAT4 in HCC cells was confirmed by a molecular probe, CL26-alkyne, which enabled CuAAC click chemistry on CL26-bound proteins for subsequent enrichment and identification by in-gel fluorescence, silver staining, and immunoblotting. Importantly, CL26 showed promising inhibitory effects on the conversion of LPA to PA, which, as explained above, is a key enzymatic reaction of AGPAT4 that drives mTOR signalling and aggressive cancer features in AGPAT4+ HCC tumors and sorafenib-resistant HCC tumors that have enriched AGPAT4 expression. This allowed CL26 to work synergistically with sorafenib to treat sorafenib-resistant HCC cells and two patient-derived HCC xenograft mouse models *in vivo.* In addition to the significant reduction in tumor weight, tumor size, and number of nodules, CL26 treatment resulted in decreased self-renewal and mTOR signalling in tumor cells, indicating the excellent efficacy of CL26 in targeting tumor lineage plasticity in HCC *in vivo.* Notably, no observable toxicity was observed in the CL26-treated mice, as indicated by the lack of significant differences in blood biochemistry, major organ weight, and histology from those in the control mice. The minimal toxicity observed after CL26 treatment can be explained by the much higher expression of AGPAT4 in endoderm and liver progenitor cells and HCC but not in mature normal hepatocytes. With good selectivity toward AGPAT4, this enabled specific targeting of highly plastic HCC cells by CL26. More in-depth studies on the on-target toxicity, if any, from CL26 in other organs and tissues are ongoing.

It is noteworthy that CL26 only targets AGPAT4 and no other related AGPAT isoforms. This is feasible because of the unique Cys228 on AGPAT4, which cannot be found in the protein sequence analysis of AGPAT3 and AGPAT5, whereas the sequence similarities of AGAPT4 with AGPAT1 (13%) and AGPAT2 (12%) were low. This makes Cys228 a perfect binding site for AGPAT4 to achieve isoform-specific targeting by cysteine-reactive covalent ligands. Through genetic mutation experiments and using CL26-alkyne as the molecular probe, it was confirmed that CL26 binds primarily to Cys228 on AGPAT4 in HCC cells. Functional assays on PA conversion and apoptosis in HCC cells expressing the AGPAT4 C228A mutant protein also support the idea that the biological activity of CL26 originates from its covalent binding to Cys228 on AGPAT4.

Cys228 in AGPAT4 is spatially close to the active site of acyltransferase. In the enzymatic reaction, this active site must accommodate two bulky substrates, LPA and acyl-CoA. Targeting Cys228 by a covalent ligand should induce steric hindrance on substrate binding, thus explaining the strong inhibitory effects of AGPAT4 activity by CL26. Also found was a significant decrease in AGPAT4 activity in the C228A mutant protein compared to the WT protein, suggesting that modification of Cys228 could mediate remarkable changes in protein activity. This further supports Cys228 on AGPAT4 as an ideal binding site for covalent ligands to achieve isoform-specific and functional binding for AGPAT4. The molecular probe, CL26-alkyne, which can bind specifically to Cys228 on AGPAT4, is a useful tool for the research and development of AGPAT4 covalent inhibitors for cancer therapy.

### References

- 1.: Cheng et al. Lancet Oncol 2009; 10:25-34.
- 2.: Llovet et al. N Engl J Med 2008; 359:378-90.
- 3.: Llovet et al. Nat Rev Clin Oncol 2022; 19:151-72.
- 4.: Boumahdi et al. Nat Rev Drug Discov 2020; 19:39-56.
- 5.: Dagogo-Jack et al. Nat Rev Clin Oncol 2018; 15:81-94.
- 6.: Lee et al. Nat Rev Gastroenterol Hepatol 2022; 19:26-44.
- 7.: Battle et al. Nat Med 2017; 23:1124-34.
- 8.: Qin et al. Proc Natl Acad Sci USA 2018; 115:E6390-1.
- 9.: Yamashita et al. Gastroenterology 2009; 136:1012-24.
- 10.: Shen et al. Database (Oxford) 2016; 2016.
- 11.: Ivanovska et al. PLoS One 2011; 6:e24582.
- 12.: Ye et al. Cancer Cell 2016; 30:444-58.
- 13.: Llovet et al. Nat Rev Clin Oncol 2015; 12:408-24.
- 14.: Wang et al. Nat Commun 2021; 12:1518.
- 15.: Cheng et al. Nat Commun 2021; 12:7142.
- 16.: Liu et al. Proc Natl Acad Sci USA 2020; 117:6103-13.
- 17.: Karagiota et al. Cancers 2022; 14:228.
- 18.: Zhukovsky et al. Front Cell Dev Biol 2019; 7:147
- 19.: Frias et al. J Biol Chem 2020; 295:263-74.
- 20.: Ma et al. J Hepatol 2017; 67:979-90.
- 21.: Tong et al. J Hepatol 2018; 69:826-39.
- 22.: Leung et al. Cancer Res 2021; 81:3229-40.
- 23.: Weerapana et al. Nature 2010; 468:790-5.
- 24.: Backus et al. Nature 2016; 534:570-4.
- 25.: Chung et al. Nat Chem Biol 2019; 15:776-85.
- 26.: Vinogradova et al. Cell 2020; 182:1009-26.
- 27.: Henning et al. Nat Chem Biol 2022; 18:412-21.
- 28.: Heal et al. Chem Soc Rev 2011; 40:246-57.
- 29.: Mok et al. Cancer Res 2022; 82:3102-15.
- 30.: Malta et al. Cell 2018; 173:338-54.
- 31.: Yamashita et al. Biology 2014; 3:801-30.
- 32.: Wen et al. Front Immunol 2023; 14:1026669.
- 33.: Pagel et al. Clin Cancer Res 2005; 11:4857-66.
- 34.: Springett et al. Cancer Res 2005; 65:9415-25.
- 35.: Zhang et al. Signal Transduct Target Ther 2020; 5:24.
- 36.: Han et al. Mol Cell 2018; 72:328-40.
- 37.: Yoon et al. J Biol Chem 211; 286:29568-74.
- 38.: Lindblad et al. Oncogene 2016; 35:5119-31.
- 39.: Yokoi et al. Oncol Rep 2018; 39:843-50.
- 40.: Li et al. World J Gastroenterol 2001; 7:630-6.
- 41.: et al. Am J Pathol 2014; 184:912-23.
- 42.: Ho et al. Hepatology 2012; 55:833-45.
- 43.: Sukumaran et al. J Mol Endocrinol 2009; 42:469-78.
- 44.: Uhlén et al. Science 2015; 347:1260419.
- 45.: Pinyol et al. J Hepatol 2021; 75:865-78.

It is understood that the disclosed method and compositions are not limited to the particular methodology, protocols, and reagents described as these can vary. It is also to be understood that the terminology used herein is for the purpose of describing particular embodiments only and is not intended to limit the scope of the present invention which will be limited only by the appended claims.

Those skilled in the art will recognize, or be able to ascertain, using no more than routine experimentation, many equivalents to the specific embodiments of the method and compositions described herein. Such equivalents are intended to be encompassed by the following claims.

### CLAUSES

1. A compound having the structure: 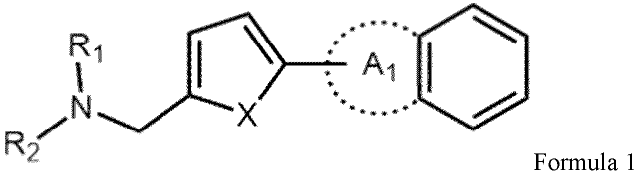 wherein:
   (i) A₁ is a C₅-C₆ aromatic ring or a C₄-C₅ heteroaromatic ring;
   (ii) R₁ is a hydrogen, a deuterium, a substituted or unsubstituted alkyl, or a substituted or unsubstituted aryl;
   (iii) R₂ is a cysteine-reactive group (such as haloacetamide, acrylamide, maleimide, vinyl sulfone and epoxide), which allows chemical reaction with cysteines on AGPAT4;
   (iv) X is O, S, NR₃, PR₃, CR₃R₄ or SiR₃R₄ where R₃ and R₄ are independently a hydrogen, a deuterium, a substituted or unsubstituted alkyl, or a substituted or unsubstituted aryl.
2. The compound of clause 1, having a structure of: 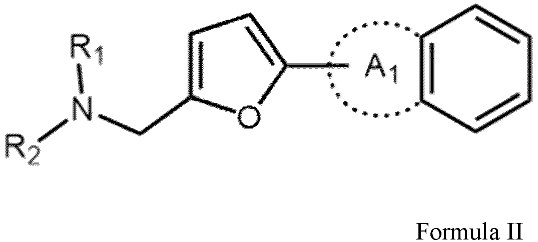 wherein:
   (i) A₁ is a C₅-C₆ aromatic ring or a C₄-C₅ heteroaromatic ring;
   (ii) R₁ is a hydrogen, a deuterium, a substituted or unsubstituted alkyl, or a substituted or unsubstituted aryl;
   (iii) R₂ is a cysteine-reactive group (such as haloacetamide, acrylamide, maleimide, vinyl sulfone and epoxide), which allows chemical reaction with cysteines on AGPAT4;
3. The compound of clause 1 or 2, having a structure of: 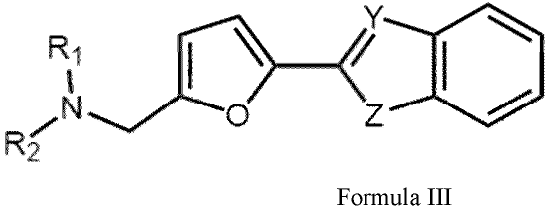 wherein:
   (i) Y is N, P, CR₃ or SiR₃ where R₃ is a hydrogen, a deuterium, a substituted or unsubstituted alkyl, or a substituted or unsubstituted aryl;
   (ii) Z is O, S, NR₄, PR₄, CR₄R₅ or SiR₄R₅ where R₄ and R₅ are independently a hydrogen, a deuterium, a substituted or unsubstituted alkyl, or a substituted or unsubstituted aryl;
   (ii) R₁ is a hydrogen, a deuterium, a substituted or unsubstituted alkyl, or a substituted or unsubstituted aryl;
   (iii) R₂ is a cysteine-reactive group (such as haloacetamide, acrylamide, maleimide, vinyl sulfone and epoxide), which allows chemical reaction with cysteines on AGPAT4;
4. The compound of any one of clauses 1- 3, having a structure of: 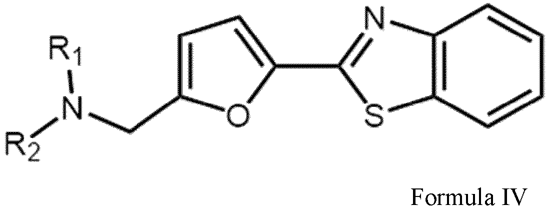 wherein:
   (i) R₁ is a hydrogen, a deuterium, a substituted or unsubstituted alkyl, or a substituted or unsubstituted aryl;
   (ii) R₂ is a cysteine-reactive group (such as haloacetamide, acrylamide, maleimide, vinyl sulfone and epoxide), which allows chemical reaction with cysteines on AGPAT4;
5. The compound of any one of clauses 1- 4, having a structure of: 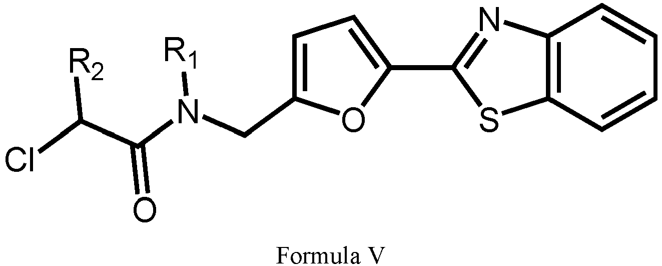 wherein:
   (i) R₁ is a hydrogen, a deuterium, a substituted or unsubstituted alkyl, or a substituted or unsubstituted aryl;
   (ii) R₂ is a hydrogen, a deuterium, a halogen, a substituted or unsubstituted alkyl, or a substituted or unsubstituted aryl;
6. The compound of any one of clauses 1-5, having a structure of: 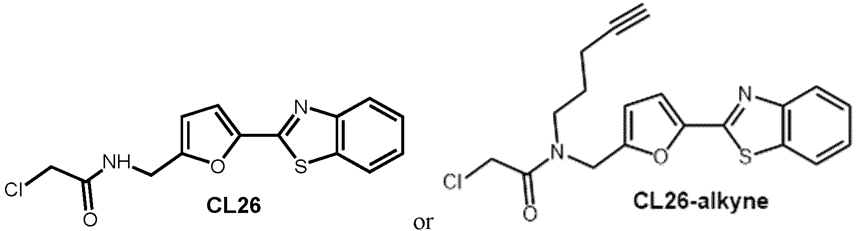
7. The compounds of any one of clauses 1-6, wherein the compounds covalently bind onto AGPAT4.
8. A composition for inhibiting or reducing the expression of 1-Acylglycerol-3-Phosphate O-Acyltransferase 4 (AGPAT4), comprising one or more compounds or molecules for inhibiting or reducing the expression of AGPAT4 in a pharmaceutically acceptable carrier.
9. The composition of clause 8, wherein the one or more compounds or molecules are selected from small molecule inhibitors, inhibitory nucleic acids, inhibitory peptides, inhibitory proteins, and derivatives thereof.
10. The composition of clause 8 or 9, wherein the small molecule inhibitor is the compounds from any one of the clauses 1-6, and optionally one or more pharmaceutically acceptable excipients.
11. The composition of clause 8 or 9, wherein the inhibitory nucleic acid is selected from the group consisting of antisense oligonucleotide (ASO), siRNA, miRNA, shRNA, and external guide sequence.
12. The composition of any of clauses 8, 9, or 11, wherein the inhibitory nucleic acid comprises the sequence 5' CGCACCAAAGGCTTTGCTATTACTTCAAGAGAGTAATAGCAAAGC CTTTGGTGTTTTTTG -3' (SEQ ID NO:2) or a variant thereof.
13. The composition of any of clauses 8, 9, 11, or 12 wherein the inhibitory nucleic acid is in an expression vector.
14. The composition of any of clauses 8, 9, 11-13, wherein the expression vector is selected from the group consisting of plasmid, minicircle DNA (mcDNA) and viral vector.
15. The composition of clause 8, wherein the vector is selected from the group consisting of bacteriophage, baculoviruses, tobacco mosaic virus, herpes virus, cytomegalo virus, retrovirus, vaccinia virus, adenovirus, and adeno-associated virus.
16. The composition of clause 8 or clause 10, wherein the small molecule inhibitor covalently binds to Cys228 on AGPAT4 .
17. The composition of any of clauses 8, 10, or 16, wherein when the small molecule inhibitor covalently binds to Cys228 on AGPAT4, the small molecule inhibitor engages in an π-π interaction with W106 and inhibits or reduces AGPAT4 activity.
18. A method of treating cancer comprising administering to a subject with cancer an effective amount of a composition comprising an AGPAT4 inhibitor, wherein administration of the AGPAT4 inhibitor reduces cancer cell proliferation and/or viability in the subject with cancer.
19. The method of clause 18, wherein the cancer is selected from liver cancer, breast cancer, head and neck squamous cell carcinoma, pancreatic adenocarcinoma, or colorectal cancer.
20. The method of clause 18 or clause 19, wherein the AGPAT4 inhibitor is selected from a small molecule inhibitor, an inhibitory nucleic acid, inhibitory peptide, or an inhibitory protein.
21. The method of any of clauses 18-20, wherein is the compounds from any one of the clauses 1-6, and optionally one or more pharmaceutically acceptable excipients.
22. The method of any of clauses 18-20, wherein the inhibitory nucleic acid is selected from the group consisting of antisense oligonucleotide (ASO), siRNA, miRNA, shRNA, and external guide sequence.
23. The method of any of clauses 18-22, wherein the composition is administered by oral administration, intramuscular administration, intravenous administration, intraperitoneal administration, or subcutaneous administration, or a combination thereof.
24. The method of clause 23, comprising subcutaneously administering the composition to the subject.
25. The method of any of clauses 18-24, wherein the composition alleviates one or more symptoms of cancer in the subject.
26. The method of any of clauses 18-25, wherein the cancer is characterized by increased expression and/or activity of 1-Acylglycerol-3-Phosphate O-Acyltransferase 4.
27. The method of any of clauses 18-26, wherein the dosage of compounds from any one of clauses 1-6 is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject.
28. The method of any of clauses 18-27, further comprising administering an effective amount of a kinase inhibitor, wherein administration of the combination of the composition and the kinase inhibitor reduces cancer cell proliferation, reduces cancer cell viability, or reduces both cancer cell viability and proliferation in a subject with cancer to a greater degree than administering to the subject the same amount of the AGPAT4 inhibitor alone or the same amount of the kinase inhibitor alone.
29. The method of any of clauses 18-28, wherein the reduction in cancer cell proliferation and/or viability in the subject with cancer is more than the additive reduction achieved by administering the AGPAT4 inhibitor alone or the kinase inhibitor alone.
30. The method of clause 29, wherein the kinase inhibitor is a receptor tyrosine kinase inhibitor.
31. The method of clause 29 or clause 30, wherein receptor tyrosine kinase inhibitor is an inhibitor of Fibroblast Growth Factor Receptor or Fms-related tyrosine kinase 4.
32. The method of any of clauses 29-30, wherein the kinase inhibitor is selected from the group consisting of sorafenib, lenvatinib, infigratinib, erdafitinib, SAR131675, crizotinib, ceritinib, alectinib, brigatinib, bosutinib, dasatinib, imatinib, nilotinib, vemurafenib, dabrafenib, ibrutinib, palbociclib, ribociclib, cabozantinib, gefitinib, erlotinib, lapatinib, vandetanib, afatinib, osimertinib, ruxolitinib, tofacitinib, trametinib, axitinib, toceranib, nintedanib, pazopanib, regorafenib, sunitinib, dacomitinib, and ponatinib.
33. The method of any of clauses 29-32, wherein the kinase inhibitor is sorafenib.
34. The method of any of clauses 29-33, wherein the dosage of sorafenib is between about 200-400 mg.
35. The method of any of clauses 18-34, wherein the cancer cells are hepatocellular carcinoma.
36. The method of any of clauses 18-35, wherein the AGPAT4 inhibitor is administered to the subject 1, 2, 3, 4, 5, 6, 8, 10, 12, 18, or 24 hours, 1, 2, 3, 4, 5, 6, or 7 days, 1, 2, 3, or 4 weeks, or any combination thereof prior to administration of the kinase inhibitor to the subject.
37. The method of any of clauses 18-36, wherein the kinase inhibitor is administered to the subject 1, 2, 3, 4, 5, 6, 8, 10, 12, 18, or 24 hours, 1, 2, 3, 4, 5, 6, or 7 days, 1, 2, 3, or 4 weeks, or any combination thereof prior to administration of the AGPAT4 inhibitor to the subject.
38. The method of any of clauses 18-37, further comprising surgery or radiation therapy.
39. The method of any of clauses 18-38, wherein the cancer to be treated is characterized by expression of genes involved in cancer stemness and/or PI3K/Akt/mTOR signaling pathway.
40. The compounds of any one of clauses 1-6 for use in a method to study AGPAT4 in a cell.
41. The composition of clause 41, wherein the compound has the structure of: 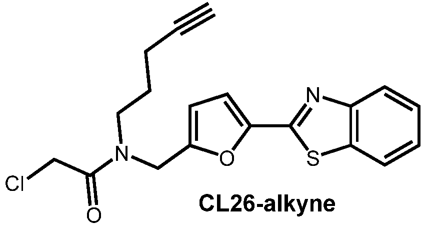
42. The composition of clause 40 or 41, wherein the compound is used for studying AGPAT4 binding with small molecule compounds.

## Claims

1. A compound having the structure: 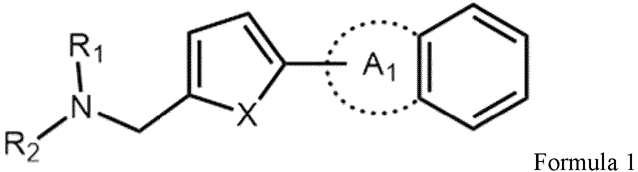 wherein:
(i) A₁ is a C₅-C₆ aromatic ring or a C₄-C₅ heteroaromatic ring;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4;
(iv) X is O, S, NR₃, PR₃, CR₃R₄ or SiR₃R₄ where R₃ and R₄ are independently a hydrogen, a deuterium, a substituted or unsubstituted alkyl, or a substituted or unsubstituted aryl.

2. The compound of claim 1, having a structure of: 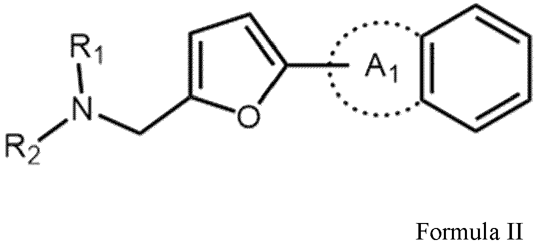 wherein:
(i) A₁ is a C₅-C₆ aromatic ring or a C₄-C₅ heteroaromatic ring;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4; and/or
having a structure of: 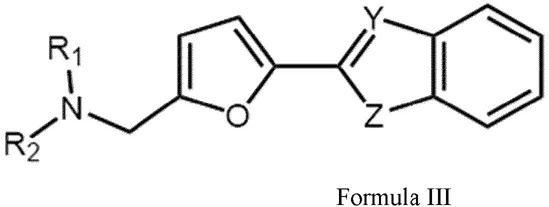 wherein:
(i) Y is N, P, CR₃ or SiR₃ where R₃ is a hydrogen, a deuterium, a substituted or unsubstituted alkyl, or a substituted or unsubstituted aryl;
(ii) Z is O, S, NR₄, PR₄, CR₄R₅ or SiR₄R₅ where R₄ and R₅ are independently a hydrogen, a deuterium, a substituted or unsubstituted alkyl, or a substituted or unsubstituted aryl;
(ii) R₁ is a hydrogen, a deuterium, a substituted or unsubstituted alkyl, or a substituted or unsubstituted aryl;
(iii) R₂ is a cysteine-reactive group (such as haloacetamide, acrylamide, maleimide, vinyl sulfone and epoxide), which allows chemical reaction with cysteines on AGPAT4; and/or
having a structure of: 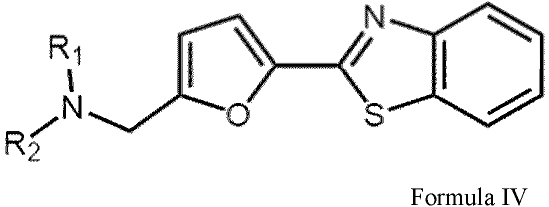 wherein:
(i) R₁ is a hydrogen, a deuterium, a substituted or unsubstituted alkyl, or a substituted or unsubstituted aryl;
(ii) R₂ is a cysteine-reactive group (such as haloacetamide, acrylamide, maleimide, vinyl sulfone and epoxide), which allows chemical reaction with cysteines on AGPAT4; and/or
having a structure of: 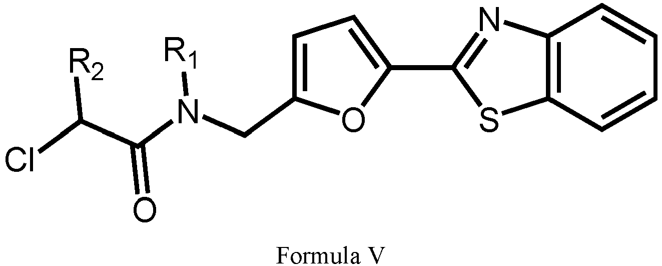 wherein:
(i) R₁ is a hydrogen, a deuterium, a substituted or unsubstituted alkyl, or a substituted or unsubstituted aryl;
(ii) R₂ is a hydrogen, a deuterium, a halogen, a substituted or unsubstituted alkyl, or a substituted or unsubstituted aryl; and/or
having a structure of:

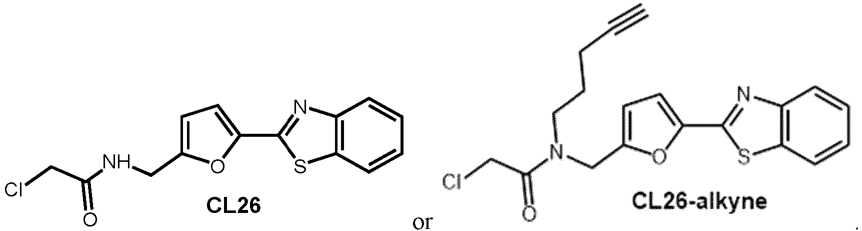

3. The compounds of claims 1 or 2, wherein the compounds covalently bind onto AGPAT4.

4. A composition for inhibiting or reducing the expression of 1-Acylglycerol-3-Phosphate O-Acyltransferase 4 (AGPAT4), comprising one or more compounds or molecules for inhibiting or reducing the expression of AGPAT4 in a pharmaceutically acceptable carrier.

5. The composition of claim 4, wherein the one or more compounds or molecules are selected from small molecule inhibitors, inhibitory nucleic acids, inhibitory peptides, inhibitory proteins, and derivatives thereof.

6. The composition of claims 4 or 5, wherein the small molecule inhibitor is the compounds from 1 or 2, and optionally one or more pharmaceutically acceptable excipients.

7. The composition of claims 4 or 5, wherein the inhibitory nucleic acid is selected from the group consisting of antisense oligonucleotide (ASO), siRNA, miRNA, shRNA, and external guide sequence.

8. The composition of any of claims 4, 5 or 7, wherein the inhibitory nucleic acid comprises the sequence 5' CGCACCAAAGGCTTTGCTATTACTTCAAGAGAGTAATAGCAAAGC CTTTGGTGTTTTTTG -3' (SEQ ID NO:2) or a variant thereof, and/or wherein the inhibitory nucleic acid is in an expression vector, and/or wherein the expression vector is selected from the group consisting of plasmid, minicircle DNA (mcDNA) and viral vector.

9. The composition of claim 4, wherein the vector is selected from the group consisting of bacteriophage, baculoviruses, tobacco mosaic virus, herpes virus, cytomegalo virus, retrovirus, vaccinia virus, adenovirus, and adeno-associated virus.

10. The composition of claim 4 or claim 6, wherein the small molecule inhibitor covalently binds to Cys228 on AGPAT4, and/or
wherein when the small molecule inhibitor covalently binds to Cys228 on AGPAT4, the small molecule inhibitor engages in an π-π interaction with W106 and inhibits or reduces AGPAT4 activity.

11. A composition comprising an AGPAT4 inhibitor for use in a method of treating cancer comprising administering to a subject with cancer an effective amount of the composition comprising an AGPAT4 inhibitor, wherein administration of the AGPAT4 inhibitor reduces cancer cell proliferation and/or viability in the subject with cancer, optionally
wherein the cancer is selected from liver cancer, breast cancer, head and neck squamous cell carcinoma, pancreatic adenocarcinoma, or colorectal cancer, and/or
wherein the AGPAT4 inhibitor is selected from a small molecule inhibitor, an inhibitory nucleic acid, inhibitory peptide, or an inhibitory protein, and/or
wherein is the compounds from claims 1 or 2, and optionally one or more pharmaceutically acceptable excipients, or
wherein the inhibitory nucleic acid is selected from the group consisting of antisense oligonucleotide (ASO), siRNA, miRNA, shRNA, and external guide sequence.

12. The composition for use according to claim 11, wherein the composition is administered by oral administration, intramuscular administration, intravenous administration, intraperitoneal administration, or subcutaneous administration, or a combination thereof, optionally
comprising subcutaneously administering the composition to the subject.

13. The composition for use according to claims 11 or 12, wherein the composition alleviates one or more symptoms of cancer in the subject, and/or
wherein the cancer is **characterized by** increased expression and/or activity of 1-Acylglycerol-3-Phosphate O-Acyltransferase 4, and/or
wherein the dosage of compounds from claims 1 or 2 is from about 0.1 µg to about 1000 µg, from about 0.1 µg to about 500 µg, from about 0.1 µg to about 100 µg, from about 0.5 µg to about 50 µg, from about 1 µg to about 1000 µg, from about 1 µg to about 500 µg, from about 1 µg to about 100 µg, from about 1 µg to about 50 µg, from about 1 µg to about 25 µg, from about 1 µg to about 10 µg, from about 0.1 µg to about 50 µg, from about 5 µg to about 50 µg, or from about 0.1 µg to about 20 µg per g of the subject, and/or
further comprising administering
an effective amount of a kinase inhibitor, wherein administration of the combination of the composition and the kinase inhibitor reduces cancer cell proliferation, reduces cancer cell viability, or reduces both cancer cell viability and proliferation in a subject with cancer to a greater degree than administering to the subject the same amount of the AGPAT4 inhibitor alone or the same amount of the kinase inhibitor alone.

14. The composition for use according to any of claims 11-13, wherein the reduction in cancer cell proliferation and/or viability in the subject with cancer is more than the additive reduction achieved by administering the AGPAT4 inhibitor alone or the kinase inhibitor alone, optionally
wherein the kinase inhibitor is a receptor tyrosine kinase inhibitor, and/or wherein receptor tyrosine kinase inhibitor is an inhibitor of Fibroblast Growth Factor Receptor or Fms-related tyrosine kinase 4, or
wherein the kinase inhibitor is selected from the group consisting of sorafenib, lenvatinib, infigratinib, erdafitinib, SAR131675, crizotinib, ceritinib, alectinib, brigatinib, bosutinib, dasatinib, imatinib, nilotinib, vemurafenib, dabrafenib, ibrutinib, palbociclib, ribociclib, cabozantinib, gefitinib, erlotinib, lapatinib, vandetanib, afatinib, osimertinib, ruxolitinib, tofacitinib, trametinib, axitinib, toceranib, nintedanib, pazopanib, regorafenib, sunitinib, dacomitinib, and ponatinib, and/or
wherein the kinase inhibitor is sorafenib, and/or
wherein the dosage of sorafenib is between about 200-400 mg and/or
wherein the cancer cells are hepatocellular carcinoma, and/or
wherein the AGPAT4 inhibitor is administered to the subject 1, 2, 3, 4, 5, 6, 8, 10, 12, 18, or
24 hours, 1, 2, 3, 4, 5, 6, or 7 days, 1, 2, 3, or 4 weeks, or any combination thereof prior to administration of the kinase inhibitor to the subject, and/or
wherein the kinase inhibitor is administered to the subject 1, 2, 3, 4, 5, 6, 8, 10, 12, 18, or 24 hours, 1, 2, 3, 4, 5, 6, or 7 days, 1, 2, 3, or 4 weeks, or any combination thereof prior to administration of the AGPAT4 inhibitor to the subject, and/or
further comprising surgery or radiation therapy, and/or
wherein the cancer to be treated is **characterized by** expression of genes involved in cancer stemness and/or PI3K/Akt/mTOR signaling pathway.

15. The compounds of claims 1 or 2 for use in a method to study AGPAT4 in a cell, optionally
wherein the compound has the structure of: 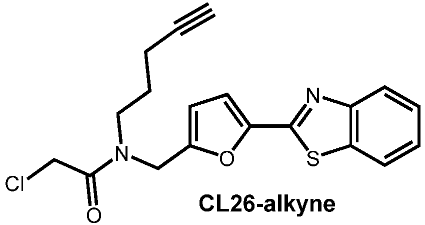 and/or wherein the compound is used for studying AGPAT4 binding with small molecule compounds.
